# EUROPEAN PATENT SPECIFICATION

(11) **EP 4 458 986 B1**
(45) Date of publication and mention of the grant of the patent: **04.11.2026**
(21) Application number: 22914834.1
(22) Date of filing: 27.12.2022
(51) Int. Cl.: C12Q 1/686, C12N 15/11, C12Q 1/6853

(54) **MULTIPLEX PCR SYSTEM**
MULTIPLEX-PCR-SYSTEM
SYSTÈME DE PCR MULTIPLEX

(30) Priority: 27.12.2021 CN 202111620423
(43) Date of publication of application: 06.11.2024
(73) Proprietor: Maccura Biotechnology Co., Ltd., Chengdu Sichuan 611731 (CN)
(72) Inventor: ZHAO, Yuhang, Chengdu, Sichuan 611731 (CN); HU, Wan er, Chengdu, Sichuan 611731 (CN); ZHAN, Haomiao, Chengdu, Sichuan 611731 (CN)
(74) Representative: Elzaburu S.L.P.
(86) International application number: PCT/CN2022/142537
(87) International publication number: WO 2023/125582

(56) References cited:
- WO-A2-2011/100057
- CN-A- 109 112 201
- CN-A- 111 733 293
- CN-A- 113 528 709
- US-A1- 2007 178 476
- US-A1- 2018 363 040
- US-A1- 2021 381 067
- LIUSHIQI BORJIGIN ET AL.: "A novel real time PCR assay for bovine leukemia virus detection using mixed probes and degenerate primers targeting novel BLV strains", JOURNAL OF VIROLOGICAL METHODS, vol. 297, 16 August 2021 (2021-08-16), XP086820403, DOI: 10.1016/j.jviromet.2021.114264

## Description

### Field of the Invention

The present invention belongs to the field of molecular biology, in particular to the field of nucleic acid amplification and detection.

### Background of the Invention

Polymerase chain reaction (PCR) is a molecular biological technology that performs DNA replication via enzyme without using living organisms. The PCR is usually used in medical and biological research laboratories to undertake a variety of tasks, such as diagnosis of infectious diseases, gene cloning, phenotypic identification of laboratory animals, research on transcriptomes, detection of genetic diseases, identification of gene fingerprints, paternity test, etc. Since the PCR has an incomparable replication ability and accuracy, it is considered by the molecular biologists as a preferred method for nucleic acid detection. In the late 1990s, a real-time fluorescence quantitative PCR (qPCR) technology and related products launched by ABI in the USA further developed PCR to be a highly sensitive, highly specific and accurately quantitative nucleic acid sequence analysis technology.

At present, a primer probe design method that is widely used in qPCR platforms is mainly a TaqMan hydrolysis probe method, a working principle of which is mainly characterized by using an oligonucleotide probe that can specifically bind with a template and is labeled with a fluorescence group (donor) and a quencher group (receptor) at two ends thereof, and by designing a specific PCR primer upstream and downstream of the probe respectively. Before the start of PCR reaction, due to a principle of fluorescence resonance energy transfer (FRET), a fluorescence signal emitted by the fluorescence group at one end of the TaqMan probe is absorbed by the quencher group at the other end, such that the fluorescence signal cannot be detected by an instrument; but after the start of PCR amplification, the TaqMan probe specifically binds with the template, and when a DNA polymerase (Taqase) extends to a site at which the probe binds to the template, the TaqMan probe will be cleaved due to 5'-3' exonuclease activity of the DNA polymerase (Taqase), such that the fluorescence group labeled on the probe will be far away from the quencher group and a FRET structure cannot be formed any more, and thus a signal emitted by the fluorescence group can be detected by the instrument. However, if a PCR detection for multiplex targets is intented to be achieved in a same reaction system, it is required to arrange multiple TaqMan hydrolysis probes labeled with fluorescence groups of different wavelengths, and at present, only a detection of 4-6-plex different targets can be achieved at most due to limitation of a number of fluorescent channels in a detection instrument.

In order to achieve an ultra-multiplex PCR detection, in the prior art, the PCR detection technology is usually combined with other methods, for example, the PCR is combined with a hybrid chip to perform detection of multiplex targets with different amplification product lengths. For example, a patent document CN107090519A discloses a detection kit for common respiratory pathogens combining multiplex RT-PCR with a gene chip, in which, a principle of nucleic acid molecule hybridization is utilized, single-stranded probes targeting individual targets are arranged and fixed on the gene chip in a specific order to form a probe array, and then multiplex PCR products to be detected are allowed to be hybridized with it, such that target amplification products are hybridized with the probes on the chip and emit fluorescence signals. Although 20 types of respiratory pathogens can be simultaneously detected by this method, the gene chip is complex in preparation process, and is expensive, thereby being not conducive to clinical promotion. Moreover, the method is cumbersome in procedures, requires multiple steps of uncovering and cleaning, and may easily cause contamination and result in a false positive result.

For another example, a patent document CN103074450B discloses a kit for simultaneous detection of thirty types of pathogenic bacteria causing diarrhea and a detection method thereof. The method combines the PCR amplification and capillary electrophoresis. In the method, PCR amplification products are taken out and subjected to capillary electrophoresis analysis; and the obtained spectrum is compared with a standard spectrum to determine the types of pathogenic bacteria causing diarrhea. In addition to the use of a PCR amplification detection equipment, this method further requires the use of a capillary electrophoresis equipment for product analysis, such that a detection cost is greatly increased, thereby being not conducive to clinical promotion and application either.

WO2011/100057A2 generally provides methods and compositions for detecting the presence or amount of one or more target nucleic acids in a sample. Methods of this invention include linking universal nucleic acids segments into a single molecule in a linking reaction dependent on a target nucleic acid of interest. A variety of universal segment linking strategies are provided, including preamplification by polymerase chain reaction, ligation-based strategies, reverse transcription and linear polymerase extension. Linking the universal segments into a single molecule generates a tagged target nucleic acid which is detected in a manner that uses an intramolecular interaction between a segment of a first universal primer and a segment of the tagged target nucleic acid. In certain embodiments, the intramolecular interaction includes the formation of a hairpin having a stem between a universal segment at one end of the tagged target nucleic acid and a second universal segment at the opposite end of the tagged target nucleic acid. Therefore, there has been an urgent clinical need for a method for multiplex nucleic acid molecule detection, which is fast, simple, and low-cost for pathogenic microorganism detection, non-invasive birth defect screening, single nucleotide polymorphism analysis, methylation analysis, gene mutation analysis, or gene typing, etc.

### Summary of the Invention

The problem to be solved by the present invention is to detect and differentiate multiple different targets in a single-tube PCR reaction by using single-tube ultra-multiplex PCR. The method can avoid false positive signals generated by a primer dimer when multiple specific primers exist. The present invention further provides a primer and probe for the single-tube super-multiplex PCR, a reaction system, and a multiple-target detection kit containing the primer, the probe and the reaction system.

In a first aspect, the present invention provides a probe primer composition for PCR detection, comprising a probe and a first primer set, wherein the first primer set comprises a first primer and a second primer that are a forward primer and a reverse primer or a reverse primer and a forward primer, respectively, wherein
the probe comprises a first binding region A' and a second binding region H,
the first primer comprises a sequence region A that is partially or completely complementary with the first binding region A' of the probe, and the second primer comprises a sequence region h that is partially or completely identical to the second binding region H of the probe,
none of the first binding region A' , the second binding region H, the sequence region A and the sequence region h are complementary or identical to a target sequence or any fragment thereof, and
the probe has a detectable label thereon.

In some embodiments, the first primer is a forward primer, and the second primer is a reverse primer; or the first primer is a reverse primer, and the second primer is a forward primer.

In some embodiments, one single-stranded amplification product of the double-stranded amplification product of the first primer and the second primer comprises the sequence region A and a sequence h' that is partially or completely complementary with the second binding region H of the probe.

In some embodiments, the probe has a length of 20-100 bases.

In some embodiments, the first binding region A' of the probe has a length of 5-65 bases.

In some embodiments, the first binding region A' of the probe has a length of 5-35 bases.

Preferably, the first binding region A' has a Tm value of 30°C-80°C, and preferably 40°C -80°C.

Preferably, the first binding region A' has a GC content of 30%-80%.

Preferably, the first binding region A' is designed at a 5' or 3' end of the probe, and is completely or partially complementary with the sequence region A of the first primer.

Preferably, a blocking region is provided at the 3' end of the probe.

The blocking region may prevent the probe from extending at the 3' end.

The blocking region is a portion that is configured to prevent extension of the nucleic acid strand through DNA polymerase, thereby blocking strand extension during, for example, a PCR process. The blocking region may function as follows: 3'OH is labeled by 3'-Spacer C3, 3'-Phosphat, 3'-ddC, 3'-InvertedEnd, etc., such that the 3' OH is blocked, so as to prevent its extension reaction. The blocking region may also be a polymerase enzyme blocking group, which is a group that has a property function of blocking further enlongation of a polymer. The blocking group may be any chemical group that can connect to a nucleotide, which allows a 5' end of a labeled nucleotide to connect to a 3' end of another nucleotide in a DNA strand but does not allow a nucleotide to connect to a 3 'hydroxyl group of the labeled nucleotide. Properly, the absence of OH group at 3' position will prevent further extension by means of polymerase activity. In some embodiments, the blocking group is selected from acetyl, CH3, glycyl, lencyl, and alanyl groups. In some other embodiments, the blocking group may be in a form of a dipeptide or tripeptide.

In some embodiments, the second binding region H has a length of 5-65 bases.

Preferably, the second binding region H has a Tm value of 30°C-80°C.

Preferably, he second binding region H ha a GC content of 20%-80%.

In some embodiments, the detectable label comprises a first detectable label and a second detectable label, wherein the first detectable label is a fluorescence reporter, and the second detectable label is a quencher or other labeling groups that can produce a signal change with the first detectable label through fluorescence resonance energy transfer.

In some embodiments, the second detectable label is spaced apart from the first detectable label by 3-250 angstroms; preferably, the second detectable label is spaced apart from the first detectable label by 3-201 angstroms; and more preferably, the second detectable label is spaced apart from the first detectable label by 3-140 angstroms.

Preferably, the second detectable label is at a position spaced apart from the first detectable label by 5 to 25 bases.

In some embodiments, both of the first detectable label and the second detectable label are located on the first binding region A', or both of the first detectable label and the second detectable label are located on the second binding region H.

In some embodiments, at least one of the first detectable label and the second detectable label is located on the first binding region A', or at least one of the first detectable label and the second detectable label is located on the second binding region H.

In some embodiments, a spacer region between the first detectable label and the second detectable label is at least partially located on the first binding region A', or the spacer region between the first detectable label and the second detectable label is at least partially located on the second binding region H.

In some embodiments, the first primer further comprises a first target sequence binding region, and the second primer further comprises a second target sequence binding region.

Preferably, the first primer sequentially comprises the sequence region A and the first target sequence binding region from 5' end to 3' end.

Preferably, the first target sequence binding region is a target specific sequence that specifically binds with a target sequence to be amplified.

In some embodiments, an annealing temperature between the probe and the first primer is TmA, an annealing temperature between the probe and a complementary sequence of the second primer is TmH, an annealing temperature between the first primer and the target sequence is Tmb1, and an annealing temperature between the second primer and the target sequence is Tmb2. In some specific embodiments, TmA<Tmb1 and TmA<Tmb2, and/or TmH<Tmb1 and TmH<Tmb2.

In some embodiments, the first binding region A' is complementary with the sequence region A of the first primer.

Preferably, the sequence region A of the first primer is a freely designed sequence that does not bind with the target sequence or any fragment thereof in a paired manner, and is partially or completely complementary with the first binding region A' of the probe. More preferably, the sequence region A of the first primer is partially or completely complementary with the first binding region A' of the probe.

Preferably, there are 0-20 basess spaced between the sequence region A and the target sequence binding region of the first primer.

Preferably, the first primer has a length of 20-80 bases.

Preferably, the first target sequence binding region of the first primer has a Tm value of 40°C-80°C.

Preferably, the first target sequence binding region of the first primer has a GC content of 20%-80%.

Preferably, the sequence region A of the first primer has a length of 5-65 bases.

Preferably, the sequence region A of the first primer has a Tm value of 40°C-80°C.

Preferably, the sequence region A of the first primer has a GC content of 30%-80%.

In some embodiments, the second binding region H and the sequence region h of the second primer have a partially or completely identical sequence.

In some embodiments, the second primer sequentially comprises the sequence region h and the second target sequence binding region from 5' end to 3' end.

Preferably, the second target sequence binding region is a target specific sequence, and specifically binds with a target sequence to be amplified.

Preferably, the second primer further comprises an extension blocking region, which is a sequence that is not identical to or complementary with a base sequence of any part of the probe.

In some embodiments, the second primer sequentially comprises the extension blocking region, the sequence region h, and the second target sequence binding region from 5' end to 3' end.

Preferably, the sequence region h of the second primer is a freely designed sequence that does not bind with the target sequence in a paired manner, and has a sequence partially or completely identical to that of the second binding region H of the probe.

Preferably, there are 0-20 bases spaced between the sequence region h and the second target sequence binding region.

Preferably, the second primer has a length of 20-80 bases.

Preferably, the extension blocking region of the second primer has a length of 1-20 bp.

Preferably, the second target sequence binding region of the second primer has a Tm value of 40°C-80°C.

Preferably, the second target sequence binding region of the second primer has a GC content of 20%-80%.

Preferably, the sequence region h of the second primer has a length of 5-65 bases.

Preferably, the sequence region h of the second primer has a Tm value of 30°C-80°C.

Preferably, the sequence region h of the second primer has a GC content of 20%-80%. In some embodiments, when there is no target to be amplified, the first binding region A' of the probe completely or partially binds with the sequence region A of the first primer in a complementary manner, and the other part of the probe is in a single-stranded state. At this time, the single-stranded flexibile probe is formed as a curly shape due to molecular flexibility, at this time, a distance between the first detectable label and the second detectable label is relatively close, and the efficiency of fluorescence resonance energy transfer is relatively high.

In some embodiments, during PCR amplification, the first primer and the second primer specifically bind with the target sequence respectively and extend to produce a double-stranded pre-amplification product. One of first single-stranded preamplification products of the double-stranded pre-amplification product comprises the sequence region A, the target sequence, and a complementary sequence h' of the sequence region h; and the second binding region H of the probe is complementarily paired with the complementary sequence h' of the sequence region h of the first single-stranded pre-amplication product, and the first binding region A' of the probe is complementarily paired with the sequence region A of the first single-stranded pre-amplication product. The probe is changed from a single-stranded state to a double-stranded state, and a distance between the first detectable label and the second detectable label is increased, thereby generating a signal that may be detected by an instrument.

In some embodiments, both of the first detectable label and the second detectable label of the probe are located on the first binding region A'; or both of the first detectable label and the second detectable label of the probe are located on the second binding region H; or a spacer region between the first detectable label and the second detectable label is at least partially located on the first binding region A'; or a spacer region between the first detectable label and the second detectable label is at least partially located on the second binding region H. During PCR amplification, the first primer and the second primer specifically bind with the target sequence and extend to produce a double-stranded pre-amplification product. One of first single-stranded preamplification products of the double-stranded pre-amplification product comprises the sequence region A, the target sequence, and a complementary sequence h' of the sequence region h; and the second binding region H of the probe is complementarily paired with the complementary sequence h' of the sequence region h of the first single-stranded pre-amplication product, and the first binding region A' of the probe is complementarily paired with the sequence region A of the first single-stranded pre-amplication product. The first single-stranded pre-amplication product forms an omega (Ω) hybridization form or an open-loop hybridization form with the probe, and the distance between the first detectable label and the second detectable label is increased, thereby generating a signal that may be detected by the instrument.

Preferably, the first single-stranded pre-amplication product further comprises a complementary sequence of an extension blocking region at 3' end thereof, which is not complementary with any part of the probe.

In some embodiments, a target sequence amplification is identified by means of a fluorescence channel, or a target sequence amplification is identified by means of a melting temperature curve, or a target sequence amplification is identified by means of a fluorescence channel + a melting temperature curve.

Preferably, a target sequence amplification is identified by means of the melting temperature curve. Specifically, after PCR amplification, a melting curve analysis is performed, during a process of temperature change, the double-stranded product formed by the probe and the first single-stranded amplication product will melt at a certain temperature, at this time, the probe with the first detectable label and the second detectable label will be changed from a double strand to a single strand, such that the distance between the detectable labels is changed, thereby generating a signal that may be detected by the instrument.

In some embodiments, the probe primer composition for n target sequences comprises n first primer sets and m probes, wherein 1 ≤ n ≤ 20, and 1 ≤ m ≤ n;

Base sequences of different probes are different, and the detectable labels on the probes are different.

Single-stranded amplification products obtained by amplifying different target sequences with all of the first primer sets for the same probe are different from each other in an annealing temperature with the probe.

Preferably, single-stranded amplification products obtained by amplifying different target sequences with all of the first primer sets for the same probe are different from each other by a △Tm of 6-10°C in an annealing temperature with the probe

Preferably, the target sequence binding regions included in all of the first primers are different from each other, and the target sequence binding regions included in all of the second primers are different from each other.

Preferably, the probe primer composition for n target sequences comprises n first primer sets and 1 or several probes, wherein 1 ≤ n ≤ 20.

In some embodiments, n probes are designed for n target sequences, and then an identification of amplifications of n target sequences is carried out by using a fluorescence channel. In such embodiments, the n probes are flexible probes.

The flexible probe forms a curly shape due to molecular flexibility in single-stranded state, but after it is extended to be double-stranded state by hybridization or annealing, it is fixed to form a relatively rigid double-helix structure due to hydrogenbond interaction. The flexible probe may be selected from a group consisting of: an oligonucleotide that forms a curly shape due to molecular flexibility in single-stranded state, an oligonucleotide that can form a hairpin structure in single-stranded state, an oligonucleotide that can form a stem-loop structure in single-stranded state, an oligonucleotide that can form a pseudoknot structure in single-stranded state, and an oligonucleotide that can form a triplex structure in single-stranded state.

In some embodiments, only 1 or several commonly used probes are designed for n target sequences, and then amplifications of n target sequences are identified by using melting curves. In such embodiments, a sequence and/or length of the sequence region h of the second primer is adjusted, and/or a sequence and/or length of the sequence region A of the first primer is adjusted to obtain the sequence region A and the complementary sequence of the sequence region h of the first single-stranded preamplification product which are partially or completely complementary with the second binding region H of the probe, and partially or completely complementary with the first binding region A', such that a melting temperature of the double-stranded product formed by the probe and the first single-stranded amplification product is adjusted.

Preferably, a length or sequence of the sequence region A and/or the sequence region h is adjusted, or positions at which the sequence region A and/or the complementary sequence of the sequence region h are complementary with the first binding region A' and/or the second binding region H of the probe are adjusted, the melting temperature of the double-stranded product formed by the probe and the first single-stranded amplification product may be increased or decreased.

Such embodiments can achieve super-multiplex PCR in a single tube and effectively avoid the formation of primer dimers.

In some embodiments, there is provided a probe primer composition for detecting respiratory syncytial virus (RSV), comprising a probe, a forward primer, and a reverse primer selected from the following group:
a probe with a sequence shown in SEQ ID NO: 1;
a forward primer with a sequence shown in SEQ ID NO: 3; and
a reverse primer with a sequence shown in SEQ ID NO: 2.

In some embodiments, the reaction conditions are as follows: optionally, peforming reverse transcription at 35-65°C for 5-60 minutes; peforming pre-denaturation at 90°C-96°C for 5-15 minutes; performing denaturation at 90°C-95°C for 10-60 seconds, and performing annealing and extension at 50°C-75°C for 30-90 seconds, for 35-50 cycles; optionally, performing denaturation at 90°C-95°C for 10-600 seconds, and performing incubation at a constant temperature of 30°C-55°C for 10-600 seconds; and performing melting curve analysis at 35-95°C with a heating rate of 0.05°C/s, and collecting fluorescence signals.

When target sequence to be detected is RNA, a reverse transcription step may be used. When the target sequence to be detected is DNA, a reverse transcription step may not be used.

More preferably, the reaction conditions are as follows: performing reverse transcription at 55°C for 15 minutes; performing pre-denaturation at 95°C for 2 minutes; performing denaturation at 94°C for 10 seconds, and performing annealing and extension at 56°C for 30 seconds, for a total of 45 cycles; performing denaturation at 94°C for 150 seconds, and performing incubation at a constant temperature of 40°C for 300 seconds; and performing melting curve analysis at 40-95°C with a heating rate of 0.05°C/S, and collecting fluorescence signals.

Preferably, concentrations of the primers and probe in a reaction system are as follows respectively: the forward primer has a concentration of 30 nM-1000 nM, the reverse primer has a concentration of 30 nM-500 nM, and the probe has a concentration of 150 nM-1200 nM.

In some embodiments, there is provided a probe primer composition for detecting respiratory syncytial virus (RSV) and influenza A virus (IAV), comprising a probe, forward primers, and reverse primers selected from the following group:
a probe with a sequence shown in SEQ ID NO: 4;
a forward primer with a sequence shown in SEQ ID NO: 5;
a reverse primer with a sequence shown in SEQ ID NO: 6;
a forward primer with a sequence shown in SEQ ID NO: 7; and
a reverse primer with a sequence shown in SEQ ID NO: 8.

Preferably, the reaction conditions are as follows: performing reverse transcription at 35-65°C for 5-60 minutes, and performing pre-denaturation at 90-96°C for 5-15 minutes; performing denaturation at 90°C -95°C for 10-60 seconds, and performing annealing and extension at 50°C-75°C for 30-90 seconds, for 35-50 cycles; performing denaturation at 90°C-95°C for 10-600 seconds, and performing incubation at a constant temperature of 30°C-55°C for 10-600 seconds; and performing melting curve analysis at 35-95°C with a heating rate of 0.05°C/s, and collecting fluorescence signals.

More preferably, the reaction conditions are as follows: performing reverse transcription at 55°C for 15 minutes; performing pre-denaturation at 95°C for 2 minutes; performing denaturation at 94°C for 10 seconds, and performing annealing and extension at 56°C for 30 seconds, for a total of 45 cycles; performing denaturation at 94°C for 150 seconds, and performing incubation at a constant temperature of 40°C for 300 seconds; and performing melting curve analysis at 40-95°C with a heating rate of 0.05°C/S, and collecting fluorescence signals.

Preferably, concentrations of the primers and probe in a reaction system of the present invention are as follows respectively: the forward primer (F) has a concentration of 30 nM-1000 nM, the reverse primer (R) has a concentration of 30 nM-500 nM, and the probe (P) has a concentration of 150 nM-1200 nM.

In some embodiments, the first primer further comprises an amplification product capturing region B, and the probe primer composition further comprises a second primer set, wherein the second primer set comprises a third primer and the second primer that are a forward primer and a reverse primer or a reverse primer and a forward primer, respectively, the third primer comprises a third target sequence binding region and an amplification product capturing region B, and the second primer further comprises a second target sequence binding region.

Preferably, the third primer sequentially comprises the amplification product capturing region B and the third target sequence binding region from 5' end to 3' end; the first primer sequentially comprises the sequence region A and the amplification product capturing region B from 5' end to 3' end, and the second primer sequentially comprises the sequence region h and the second target sequence binding region from 5' end to 3' end; and the amplification product capturing region B does not bind with the target sequence or any fragment thereof in a paired manner.

The second primer further comprises an extension blocking region, which is a sequence that is not identical to or complementary with a base sequence of any part of the probe.

More preferably, the second primer sequentially comprises the extension blocking region, the sequence region h, and the second target sequence binding region from 5' end to 3' end.

Preferably, the target sequence binding region is a target specific sequence, and specifically binds with a target sequence to be amplified.

In some embodiments, an annealing temperature between the probe and the first primer is TmA, an annealing temperature between the probe and a complementary sequence of the second primer is TmH, an annealing temperature between the third primer and the target sequence is Tmb1, and an annealing temperature between the second primer and the target sequence is Tmb2. In some specific embodiments, TmA<Tmb1 and TmA<Tmb2, and/or TmH<Tmb 1 and TmH<Tmb2.

Preferably, the third primer has a length of 20-80 bases.

Preferably, the third target sequence binding region of the third primer has a Tm value of 40°C-80°C.

Preferably, the third target sequence binding region of the third primer has a GC content of 20%-80%.

Preferably, the amplification product capturing region B has a length of 5-35 bases.

Preferably, the amplification product capturing region B has a Tm value of 40°C-80°C.

Preferably, the amplification product capturing region B has a GC content of 20%-80%.

Preferably, the first primer has a length of 20-80 bases, wherein the sequence region A has a length of 5-65 bases.

Preferably, the sequence region A of the first primer has a Tm value of 40°C -80°C.

Preferably, the sequence region A of the first primer has a GC content of 30%-80%.

Preferably, there may be 0-20 bases spaced between the amplification product capturing region B and the target sequence binding region.

In some embodiments, when there is no target to be amplified, the first binding region A' of the probe complementarily binds with the sequence region A of the first primer, and the other part of the probe is in a single-stranded state, the single-stranded flexibile probe is formed as a curly shape due to molecular flexibility, at this time, a distance between the first detectable label and the second detectable label is relatively close, and the efficiency of fluorescence resonance energy transfer is relatively high.

In some embodiments, during PCR amplification, the third primer and the second primer respectively specifically bind with the target sequence and extend to produce a first double-stranded amplification product. One of first single-stranded amplification products of the first double-stranded amplification product comprises the extension blocking region, the sequence region h, the target sequence, and a complementary sequence of the amplification product capturing region B; then, the amplification product capturing region B of the first primer is complementary with the complementary sequence of the amplification product capturing region at 3' end of the first single-stranded amplification product and extends to obtain a second double-stranded amplification product. One of second single-stranded amplification products of the second double-stranded amplification product comprises the sequence region A, the amplification product capturing region B, the target sequence, a complementary sequence of the sequence region h, and a complementary sequence of the extension blocking region.

In such embodiments, the second binding region H of the probe is complementarily paired with the complementary sequence of the sequence region h of the second single-stranded amplication product, and the first binding region A' of the probe is complementarily paired with the sequence region A of the second single-stranded amplication product. At this time, the probe is changed from a single-stranded state to a double-stranded state, and a distance between the first detectable label and the second detectable label is increased, thereby generating a signal that may be detected by an instrument.

In some embodiments, both of the first detectable label and the second detectable label of the probe are located on the first binding region A'; or both of the first detectable label and the second detectable label of the probe are located on the second binding region H; or a spacer region between the first detectable label and the second detectable label is at least partially located on the first binding region A'; or a spacer region between the first detectable label and the second detectable label is at least partially located on the second binding region H. The second binding region H of the probe is complementarily paired with the complementary sequence of the sequence region h of the second single-stranded amplication product, and the first binding region A' of the probe is complementarily paired with the sequence region A of the second single-stranded amplication product, the second single-stranded pre-amplication product forms an omega (Ω) hybridization form or an open-loop hybridization form with the probe, and the distance between the first detectable label and the second detectable label is increased, thereby generating a signal that may be detected by the instrument.

Preferably, the complementary sequence of the extension blocking region of the first single-stranded amplification product is not complementary with the probe and any fragment thereof.

In some embodiments, a target sequence amplification is identified by means of a fluorescence channel, or a target sequence amplification is identified by means of a melting temperature curve, or a target sequence amplification is identified by means of a fluorescence channel + a melting temperature curve.

Preferably, a target sequence amplification is identified by means of the melting temperature curve. Specifically, after PCR amplification, a melting curve analysis is performed, during a process of temperature change, the double-stranded product formed by the probe and the second single-stranded amplication product will melt at a certain temperature, at this time, the probe with the first detectable label and the second detectable label will be changed from a double strand to a single strand, the distance between the first detectable label and the second detectable label is decreased and the efficiency of fluorescence resonance energy transfer is increased, thereby generating a signal that may be detected by the instrument.

In some embodiments, the probe primer composition comprises n second primer sets, m probes and o first primers for n target sequences, wherein 1 ≤ n ≤ 20, 1 ≤ m ≤ n, and 1 ≤ o ≤ n; and preferably, wherein 1 ≤ m ≤ o.

Single-stranded amplification products obtained by amplification with all of the first primer sets for the same probe are different from each other in an annealing temperature with the probe.

Preferably, single-stranded amplification products obtained by amplification with all of the first primer sets for the same probe are different from each other by a △Tm of 6-10°C in an annealing temperature with the probe

Preferably, the target sequence binding regions included in all of the third primers are different from each other, and the target sequence binding regions included in all of the second primers are different from each other.

Preferably, the probe primer composition for n target sequences comprises n second primer sets, 1 or several probes, and 1 or several first primers, wherein 1 ≤ n ≤ 20.

In some embodiments, n probes and n first primers are designed for n target sequences, and then an identification of amplifications of n target sequences is carried out by using a fluorescence channel. In such embodiments, the n probes are flexible probes.

The flexible probe forms a curly shape due to molecular flexibility in single-stranded state, but after it is extended to be double-stranded state by hybridization or annealing, it is fixed to form a relatively rigid double-helix structure due to hydrogenbond interaction. The flexible probe may be selected from a group consisting of: an oligonucleotide that forms a curly shape due to molecular flexibility in single-stranded state, an oligonucleotide that can form a hairpin structure in single-stranded state, an oligonucleotide that can form a stem-loop structure in single-stranded state, an oligonucleotide that can form a pseudoknot structure in single-stranded state, and an oligonucleotide that can form a triplex structure in single-stranded state.

In some embodiments, only one probe and one first primer are designed for n target sequences, and then amplifications of n target sequences are identified by using melting curves. In such embodiments, a sequence and/or length of the sequence region h of the second primer is adjusted to obtain the sequence region A and the complementary sequence of the sequence region h of the second single-stranded pre-amplification product which are partially or completely complementary with the first binding region A' and the second binding region H of the probe, such that a melting temperature of the double-stranded product formed by the probe and the seond single-stranded amplification product is adjusted.

Preferably, a length or sequence of the sequence region A and/or the sequence region h is adjusted, or positions at which the sequence region A and/or the complementary sequence of the sequence region h are complementary with the first binding region A' and/or the second binding region H of the probe are adjusted, the melting temperature of the double-stranded product formed by the probe and the second single-stranded amplification product may be increased or decreased.

Such embodiments can achieve super-multiplex PCR in a single tube and effectively avoid the formation of primer dimers.

In some embodiments, there is provided a probe primer composition for detecting respiratory syncytial virus, comprising:
a probe with a sequence shown in SEQ ID NO: 9;
a third primer with a sequence shown in SEQ ID NO: 17;
a first primer with a sequence shown in SEQ ID NO: 18; and
a second primer with a sequence shown in SEQ ID NO: 16.

Preferably, the reaction conditions are as follows: performing reverse transcription at 35-65°C for 5-60 minutes, and performing pre-denaturation at 90-96°C for 5-15 minutes; performing denaturation at 90°C-95°C for 10-60 seconds, and performing annealing and extension at 50°C-75°C for 30-90 seconds, for 35-50 cycles; performing denaturation at 90°C-95°C for 10-600 seconds, and performing incubation at a constant temperature of 30°C-55°C for 10-600 seconds, peforming melting curve analysis at 35-95°C with a heating rate of 0.05°C/s, and collecting fluorescence signals.

More preferably, the reaction conditions are as follows: performing reverse transcription at 55°C for 15 minutes, and performing pre-denaturation at 95°C for 2 minutes; performing denaturation at 94°C for 10 seconds, and performing annealing and extension at 56°C for 30 seconds, for a total of 45 cycles; performing denaturation at 94°C for 150 seconds, and performing incubation at a constant temperature of 40°C for 300 seconds; and performing melting curve analysis at 40-95°C with a heating rate of 0.05°C/S, and collecting fluorescence signals.

Preferably, concentrations of the primers and probe in a reaction system of the present invention are as follows respectively: the third primer (F) has a concentration of 30 nM-1000 nM, the second primer (R) has a concentration of 30 nM-500 nM, the first primer (F0) has a concentration of 30 nM-1000 nM, and the probe (P) has a concentration of 150 nM-1200 nM.

In some embodiments, the second primer further comprises an amplification product capturing region C, and the probe primer composition further comprises a second primer set, the second primer set comprises the first primer and a fourth primer that are a forward primer and a reverse primer or a reverse primer and a forward primer, respectively, wherein the fourth primer set comprises a fourth target sequence binding region and an amplification product capturing region C, and the first primer further comprises a first target sequence binding region.

In some embodiments, the first primer sequentially comprises the sequence region A and the first target sequence binding region from 5' end to 3' end; the second primer sequentially comprises the sequence region h and the amplification product capturing region C from 5' end to 3' end; and the fourth primer sequentially comprises the amplification product capturing region C and the fourth target sequence binding region from 5' end to 3' end,

The amplification product capturing region C does not bind with a target sequence or any fragment thereof in a paired manner.

Preferably, the second primer further comprises an extension blocking region, which is a sequence that is not identical to or complementary with a base sequence of any part of the probe.

More preferably, the second primer sequentially comprises the extension blocking region, the sequence region h, and the amplification product capturing region C from 5' end to 3' end.

In some embodiments, an annealing temperature between the probe and the first primer is TmA, an annealing temperature between the probe and a complementary sequence of the second primer is TmH, an annealing temperature between the first primer and the target sequence is Tmb1, and an annealing temperature between the fourth primer and the target sequence is Tmb2. In some specific embodiments, TmA<Tmb1 and TmA<Tmb2, and/or TmH<Tmb1 and TmH<Tmb2.

Preferably, the fourth primer has a length of 20-80 bases.

Preferably, the fourth target sequence binding region of the fourth primer has a Tm value of 40°C-80°C.

Preferably, the fourth target sequence binding region of the fourth primer has a GC content of 20%-80%.

Preferably, the amplification product capturing region C is has a length of 5-35 bases.

Preferably, the amplification product capture region C has a Tm value of 40°C-80°C.

Preferably, the amplification product capturing region C has a GC content of 20%-80%.

Preferably, the first primer has a length of 20-80 bases, with sequence region A having a length of 5-65 bases.

Preferably, the sequence region A of the first primer has a Tm value of 40°C-80°C. Preferably, the sequence region A of the first primer has a GC content of 30%-80%.

Preferably, there may be a 0-20 bases spaced between the amplification product capture region C and the target sequence binding region.

In some embodiments, when there is no target to be amplified, the first binding region A' of the probe complementarily binds with the sequence region A of the first primer, and the other part of the probe is in a single-stranded state, the single-stranded flexibile probe is formed as a curly shape due to molecular flexibility, at this time, a distance between the first detectable label and the second detectable label is relatively close, and the efficiency of fluorescence resonance energy transfer is relatively high.

In some embodiments, during PCR amplification, the first primer and the fourth primer of the second primer set are used to specifically bind with a target sequence and extend to produce a first double-stranded pre-amplification product, wherein one single-stranded amplification product of the first double-stranded pre-amplification product comprises a complementary sequence of the sequence region A, the target sequence, and the amplification product capture region C;
the first primer set is used and the single-stranded amplification product is taken as a template to extend to obtain a second double-stranded amplification product, wherein one single-stranded amplification product of the second double-stranded amplification product comprises the sequence region A, the target sequence, a complementary sequence of the amplification product capturing region C, and a complementary sequence of the sequence region h, and
a signal change that can be detected by an instrument is formed, by specifical binding of the A and h' of the single-stranded amplification product with the probe, and whether the target sequence is present is determined based on the signal change.

Preferably, the one single-stranded amplification product of the first double-stranded pre-amplification product and/or the one single-stranded amplification product of the second double-stranded amplification product further comprises a complementary sequence of the extension blocking region, and the complementary sequence of the extension blocking region is not complementary with any part of the probe.

In such embodiments, the second binding region H of the probe is complementarily paired with the complementary sequence of the sequence region h of the second single-stranded amplication product, and the first binding region A' of the probe is complementarily paired with the sequence region A of the second single-stranded amplication product. At this time, the probe is changed from a single-stranded state to a double-stranded state, and a distance between the first detectable label and the second detectable label is increased, thereby generating a signal that may be detected by an instrument.

In some embodiments, both of the first detectable label and the second detectable label of the probe are located on the first binding region A'; or both of the first detectable label and the second detectable label of the probe are located on the second binding region H; or a spacer region between the first detectable label and the second detectable label is at least partially located on the first binding region A'; or a spacer region between the first detectable label and the second detectable label is at least partially located on the second binding region H. The second binding region H of the probe is complementarily paired with the complementary sequence of the sequence region h of the second single-stranded amplication product, and the first binding region A' of the probe is complementarily paired with the sequence region A of the second single-stranded amplication product, the second single-stranded amplication product forms an omega (Ω) hybridization form or an open-loop hybridization form with the probe, and the distance between the first detectable label and the second detectable label is increased, thereby generating a signal that may be detected by the instrument.

Preferably, the complementary sequence of the extension blocking region of the second single-stranded amplification product is not complementary with the probe and any fragment thereof.

In some embodiments, a target sequence amplification is identified by means of a fluorescence channel, or a target sequence amplification is identified by means of a melting temperature curve, or a target sequence amplification is identified by means of a fluorescence channel + a melting temperature curve.

Preferably, a target sequence amplification is identified by means of the melting temperature curve. Specifically, after PCR amplification, a melting curve analysis is performed, during a process of temperature change, the double-stranded product formed by the probe and the second single-stranded amplication product will melt at a certain temperature, at this time, the probe with the first detectable label and the second detectable label will be changed from a double strand to a single strand, the distance between the first detectable label and the second detectable label is decreased and the efficiency of fluorescence resonance energy transfer is increased, thereby generating a signal that may be detected by the instrument.

In some embodiments, the probe primer composition for n target sequences comprises n second primer sets, m probes and o second primers, wherein 1 ≤ n ≤ 20, 1 ≤ m ≤ n, and 1 ≤ o ≤ n; and preferably, wherein 1 ≤ m ≤ o.

Single-stranded amplification products obtained by amplification with all of the first primer sets for the same probe are different from each other in an annealing temperature with the probe.

Preferably, single-stranded amplification products obtained by amplification with all of the first primer sets for the same probe are different from each other by a △Tm of 6-10°C in an annealing temperature with the probe

Preferably, the target sequence binding regions included in all of the first primers are different from each other, and the target sequence binding regions included in all of the fourth primers are different from each other.

Preferably, the probe primer composition comprises n second primer sets, 1 or several probes, and 1 or several second primers for n target sequences, wherein 1 ≤ n ≤ 20.

In some embodiments, n probes and n second primers are designed for n target sequences, and then an identification of amplifications of n target sequences is carried out by using a fluorescence channel. In such embodiments, the n probes are flexible probes.

The flexible probe forms a curly shape due to molecular flexibility in single-stranded state, but after it is extended to be double-stranded state by hybridization or annealing, it is fixed to form a relatively rigid double-helix structure due to hydrogenbond interaction. The flexible probe may be selected from a group consisting of: an oligonucleotide that forms a curly shape due to molecular flexibility in single-stranded state, an oligonucleotide that can form a hairpin structure in single-stranded state, an oligonucleotide that can form a stem-loop structure in single-stranded state, an oligonucleotide that can form a pseudoknot structure in single-stranded state, and an oligonucleotide that can form a triplex structure in single-stranded state.

In some embodiments, only one probe and one second primer are designed for n target sequences, and then amplifications of n target sequences are identified by using melting curves. In such embodiments, a sequence and/or length of the sequence region A of the first primer is adjusted to obtain the sequence region A and the complementary sequence of the sequence region h of the second single-stranded pre-amplification product which are partially or completely complementary with the first binding region A' and the second binding region H of the probe, such that a melting temperature of the double-stranded product formed by the probe and the first single-stranded amplification product is adjusted.

Preferably, a length or sequence of the sequence region A and/or the sequence region h is adjusted, or positions at which the sequence region A and/or the complementary sequence of the sequence region h are complementary with the first binding region A' and/or the second binding region H of the probe are adjusted, the melting temperature of the double-stranded product formed by the probe and the second single-stranded amplification product may be increased or decreased.

Such embodiments can achieve super-multiplex PCR in a single tube and effectively avoid the formation of primer dimers.

In some embodiments, the first primer further comprises an amplification product capturing region B, the second primer further comprises an amplification product capturing region C, and the probe primer composition further comprises a second primer set, the second primer set comprises a third primer and a fourth primer that are a forward primer and a reverse primer or a reverse primer and a forward primer, respectively, the third primer set comprises a third target sequence binding region and an amplification product capturing region B, and the fourth primer set comprises a fourth target sequence binding region and an amplification product capturing region C.

In some embodiments, the first primer sequentially comprises the sequence region A and the amplification product capturing region B from 5' end to 3' end; the second primer sequentially comprises the sequence region h and the amplification product capturing region C from 5' end to 3' end; the third primer sequentially comprises the amplification product capturing region B and the third target sequence binding region from 5' end to 3' end; and the fourth primer sequentially comprises the amplification product capturing region C and the fourth target sequence binding region from 5' end to 3' end.

Neither the amplification product capturing region B nor the amplification product capturing region C is paired with the target sequence or any fragment thereof.

Preferably, the second primer further comprises an extension blocking region, which is a base sequence that is not identical to or complementary with the probe or any fragmet thereof.

More preferably, the second primer sequentially comprises the extension blocking region, the sequence region h, and the amplification product capturing region C from 5' end to 3' end.

Preferably, the third target sequence binding region of the third primer is a target specific sequence, and specifically binds with the target sequence to be amplified.

An annealing temperature between the probe and the first primer is TmA, an annealing temperature between the probe and a complementary sequence of the second primer is TmH, an annealing temperature between the third primer and the target sequence is Tmb1, and an annealing temperature between the fourth primer and the target sequence is Tmb2. In some specific embodiments, TmA<Tmb1 and TmA<Tmb2, and/or TmH<Tmb1 and TmH<Tmb2
Preferably, the third primer comprises the amplification product capturing region B and the third target sequence binding region.

Preferably, the third primer has a length of 20-80 bases.

Preferably, the third target sequence binding region of the third primer has a Tm value of 40°C-80°C.

Preferably, the third target sequence binding region of the third primer has a GC content of 20%-80%.

Preferably, the amplification product capturing region B has a length of 5-35 bases.

Preferably, the amplification product capturing region B has a Tm value of 40°C-80°C.

Preferably, the amplification product capturing region B has a GC content of 20%-80%.

Preferably, the first primer has a length of 20-80 bases, with sequence region A having a length of 5-35 bases.

Preferably, the sequence region A of the first primer has a Tm value of 40°C-80°C.

Preferably, the sequence region A of the first primer has a GC content of 30%-80%.

Preferably, there may be a 0-20 bases spaced between the amplification product capturing region B and the target sequence binding region.

Preferably, the second primer further comprises the extension blocking region, which is a freely designed sequence that is not identical to a base sequence of any part of the probe.

Preferably, the fourth target sequence binding region of the fourth primer is a target specific sequence, and specifically binds with the target sequence to be amplified.

Preferably, the fourth primer comprises an amplification product capturing region C and a fourth target sequence binding region.

Preferably, the fourth primer has a length of 20-80 bases.

Preferably, the fourth target sequence binding region of the fourth primer has a Tm value of 40°C-80°C.

Preferably, the fourth target sequence binding region of the fourth primer has a GC content of 20%-80%.

Preferably, the amplification product capturing region C has a length of 5-35 bases.

Preferably, the amplification product capture region C has a Tm value of 40°C-80°C.

Preferably, the amplification product capturing region C has a GC content of 20%-80%.

Preferably, the second primer comprises the extension blocking region, the sequence region h, and the amplification product capturing region C.

Preferably, the second primer has a length of 20-80 bases, with sequence region h having a length of 5-65 bases.

Preferably, the sequence region h of the second primer has a Tm value of 30-80°C.

Preferably, the sequence region h of the second primer has a GC content of 20-80%.

Preferably, the extension blocking region of the second primer has a length of 1-20 bp.

Preferably, there are 0-20 bases spaced between the sequence region h and the amplification product capture region C.

In some embodiments, when there is no target to be amplified, the first binding region A' of the probe complementarily binds with the sequence region A of the first primer, and the other part of the probe is in a single-stranded state, the single-stranded flexibile probe is formed as a curly shape due to molecular flexibility, at this time, a distance between the first detectable label and the second detectable label is relatively close, and the efficiency of fluorescence resonance energy transfer is relatively high.

In some embodiments, during a first PCR amplification, the third primer and the fourth primer specifically bind with a target sequence respectively and extend to produce a first double-stranded amplification product, wherein one of single-stranded amplification products of the first double-stranded amplification product comprises the amplification product capturing region B, the target sequence, and a complementary sequence of the amplification product capturing region C; then, the first double-stranded amplification product is taken as a template, and the first primer and the second primer are used to perform a second PCR to obtain a second double-stranded amplification product, wherein one of second single-stranded amplification products of the second double-stranded amplification product comprises the sequence region A, the amplification product capturing region B, the target sequence, a complementary sequence of the amplification product capturing region C, a complementary sequence of the sequence region h, and a complementary sequence of the extension blocking region.

In such embodiments, the second binding region H of the probe is complementarily paired with the complementary sequence of the sequence region h of the second single-stranded amplication product, and the first binding region A' of the probe is complementarily paired with the sequence region A of the second single-stranded amplication product at 5' end thereof. At this time, the probe is changed from a single-stranded state to a double-stranded state, and a distance between the first detectable label and the second detectable label is increased, thereby generating a signal that may be detected by an instrument.

In some embodiments, both of the first detectable label and the second detectable label of the probe are located on the first binding region A'; or both of the first detectable label and the second detectable label of the probe are located on the second binding region H; or a spacer region between the first detectable label and the second detectable label is at least partially located on the first binding region A'; or a spacer region between the first detectable label and the second detectable label is at least partially located on the second binding region H. The second binding region H of the probe is complementarily paired with the complementary sequence of the sequence region h of the second single-stranded amplication product, and the first binding region A' of the probe is complementarily paired with the sequence region A of the second single-stranded amplication product, the second single-stranded amplication product forms an omega (Ω) hybridization form or an open-loop hybridization form with the probe, and the distance between the first detectable label and the second detectable label is increased, thereby generating a signal that may be detected by the instrument.

Preferably, the complementary sequence of the extension blocking region of the second single-stranded pre-amplification product at 3' end thereof is not complementary with the probe and any fragment thereof.

In some embodiments, a target sequence amplification is identified by means of a fluorescence channel, or a target sequence amplification is identified by means of a melting temperature curve, or a target sequence amplification is identified by means of a fluorescence channel + a melting temperature curve.

Preferably, a target sequence amplification is identified by means of the melting temperature curve. Specifically, after the second PCR amplification, a melting curve analysis is performed, during a process of temperature change, the double-stranded product formed by the probe and the second single-stranded amplication product will melt at a certain temperature, at this time, the probe with the first detectable label and the second detectable label will be changed from a double strand to a single strand, such that the distance between the first detectable label and the second detectable label is decreased and the efficiency of fluorescence resonance energy transfer is increased, thereby generating a signal that may be detected by the instrument.

In some embodiments, the probe primer composition comprises n second primer sets for n target sequences, m probes and p first primer sets, wherein 1 ≤ n ≤ 20, 1 ≤ m ≤ n, and 1 ≤ p ≤ n.

Single-stranded amplification products obtained by amplification with all of the first primer sets for the same probe are different from each other in an annealing temperature with the probe.

Preferably, single-stranded amplification products obtained by amplification with all of the first primer sets for the same probe are different from each other by a △Tm of 6-10°C in an annealing temperature with the probe

Preferably, the target sequence binding regions included in all of the third primers are different from each other, and the target sequence binding regions included in all of the fourth primers are different from each other.

Preferably, the probe primer composition for n target sequences comprises n second primer sets, 1 or several probes, and 1 or several first primer sets, wherein 1 ≤ n ≤ 20.

In some embodiments, n probes, n first primers and n second primers are designed for n target sequences, and then an identification of amplifications of n target sequences is carried out by using a fluorescence channel. In such embodiments, the n probes are flexible probes.

The flexible probe forms a curly shape due to molecular flexibility in single-stranded state, but after it is extended to be double-stranded state by hybridization or annealing, it is fixed to form a relatively rigid double-helix structure due to hydrogenbond interaction. The flexible probe may be selected from a group consisting of: an oligonucleotide that forms a curly shape due to molecular flexibility in single-stranded state, an oligonucleotide that can form a hairpin structure in single-stranded state, an oligonucleotide that can form a stem-loop structure in single-stranded state, an oligonucleotide that can form a pseudoknot structure in single-stranded state, and an oligonucleotide that can form a triplex structure in single-stranded state.

In some embodiments, only one probe, one first primer and one second primer are designed for n target sequences, and then amplifications of n target sequences are identified by using melting curves. In such embodiments, a sequence and/or length of the sequence region A of the first primer and/or the sequence region h of the second primer are adjusted to obtain the sequence region A and the complementary sequence of the sequence region h of the second single-stranded pre-amplification product which are partially or completely complementary with the first binding region A' and the second binding region H of the probe, such that a melting temperature of the double-stranded product formed by the probe and the second single-stranded amplification product is adjusted.

Preferably, a length or sequence of the sequence region A and/or the sequence region h is adjusted, or positions at which the sequence region A and/or the complementary sequence of the sequence region h are complementary with the first binding region A' and/or the second binding region H of the probe are adjusted, the melting temperature of the double-stranded product formed by the probe and the second single-stranded amplification product may be increased or decreased.

Such embodiments can achieve super-multiplex PCR in a single tube and effectively avoid the formation of primer dimers.

In some embodiments, there is provided a probe primer composition for detecting respiratory syncytial virus, comprising:
a probe with a sequence shown in SEQ ID NO: 9;
a third primer with a sequence shown in SEQ ID NO: 17;
a first primer with a sequence shown in SEQ ID NO: 18;
a fourth primer with a sequence shown in SEQ ID NO: 19; and
a second primer with a sequence shown in SEQ ID NO: 20.

Preferably, the reaction conditions are as follows: performing reverse transcription at 35-65°C for 5-60 minutes, and performing pre-denaturation at 90-96°C for 5-15 minutes; performing denaturation at 90°C-95°C for 10-60 seconds, and performing annealing and extension at 50°C-75°C for 30-90 seconds, for 35-50 cycles; performing denaturation at 90°C-95°C for 10-600 seconds, and performing incubation at a constant temperature of 30°C-55°C for 10-600 seconds, peforming melting curve analysis at 35-95°C with a heating rate of 0.05°C/s, and collecting fluorescence signals.

More preferably, the reaction conditions are as follows: performing reverse transcription at 55°C for 15 minutes; performing pre-denaturation at 95°C for 2 minutes; performing denaturation at 94°C for 10 seconds, and performing annealing and extension at 56°C for 30 seconds, for a total of 45 cycles; performing denaturation at 94°C for 150 seconds, and performing incubation at a constant temperature of 40°C for 300 seconds; and performing melting curve analysis at 40-95°C with a heating rate of 0.05°C/S, and collecting fluorescence signals.

Preferably, concentrations of the primers and probe in a reaction system of the present invention are as follows respectively: the third primer (F) has a concentration of 30 nM-1000 nM, the fourth primer (R) has a concentration of 30 nM-500 nM, the first primer (F0) has a concentration of 30 nM-1000 nM, the second primer (F) has a concentration of 30 nM-1000 nM, and the probe (P) has a concentration of 150 nM-1200 nM.

In a second aspect, the present invention provides a reaction system for PCR detection, comprising the probe primer composition of the first aspect.

In a third aspect, the present invention provides a kit for PCR detection, comprising the probe primer composition of the first aspect or the reaction system of the second aspect.

In a fourth aspect, the present invention provides a method for PCR detection, comprising steps of performing PCR by using the probe primer composition of the first aspect, the reaction system of the second aspect, or the kit of the third aspect.

In some embodiments, the first primer set is used to specifically bind with a target sequence and extend to produce a double-stranded pre-amplification product, wherein one single-stranded amplification product of the double-stranded pre-amplification product comprises the sequence region A, the target sequence, and a complementary sequence h' of the sequence region h; and
a signal change that can be detected by an instrument is formed, by specifical binding of the A and h' of the single-stranded amplification product with the probe, and whether the target sequence is present is determined based on the signal change.

Preferably, the single-stranded amplification product further comprises a complementary sequence of the extension blocking region at 3' end thereof, which is not complementary with any part of the probe.

In some embodiments, the third primer and the second primer of the second primer set are used to specifically bind with a target sequence and extend to produce a first double-stranded pre-amplification product, wherein one single-stranded amplification product of the first double-stranded pre-amplification product comprises a complementary sequence B' of the amplification product capturing region B, the target sequence, and the sequence region h;
the first primer set is used and the first double-stranded pre-amplification product is taken as a template, to extend to obtain a second double-stranded amplification product, wherein one single-stranded amplification product of the second double-stranded amplification product comprises the sequence region A, the amplification product capturing region B, the target sequence, and a complementary sequence h' of the sequence region h; and
a signal change that can be detected by an instrument is formed, by specifical binding of the A and h' of the single-stranded amplification product with the probe, and whether the target sequence is present is determined based on the signal change.

Preferably, the one single-stranded amplification product of the first double-stranded pre-amplification product, and/or the one single-stranded amplification product of the second double-stranded amplification product further comprises a complementary sequence of the extension blocking region, and the complementary sequence of the extension blocking region is not complementary with any part of the probe.

In some embodiments, the first primer and the fourth primer of the second primer set are used to specifically bind with a target sequence and extend to produce a first double-stranded pre-amplification product, wherein one single-stranded amplification product of the first double-stranded pre-amplification product comprises a complementary sequence of the sequence region A, the target sequence, and the amplification product capturing region C;
the first primer set is used and the first double-stranded pre-amplification product is taken as a template to extend to obtain a second double-stranded amplification product, wherein one single-stranded amplification product of the second double-stranded amplification product comprises the sequence region A, the target sequence, a complementary sequence of the amplification product capturing region C, and a complementary sequence h' of the sequence region h, and
a signal change that can be detected by an instrument is formed, by specifical binding of the A and h' of the single-stranded amplification product with the probe, and whether the target sequence is present is determined based on the signal change.

Preferably, the one single-stranded amplification product of the second double-stranded amplification product further comprises a complementary sequence of the extension blocking region, and the complementary sequence of the extension blocking region is not complementary with any part of the probe.

In some embodiments, the third primer and the fourth primer of the second primer set are used to specifically bind with a target sequence and extend to produce a first double-stranded pre-amplification product, wherein one single-stranded amplification product of the first double-stranded pre-amplification product comprises a complementary sequence of the sequence B, the target sequence, and the amplification product capturing region C;
the first primer set is used and the first double-stranded pre-amplification product is taken as a template to extend to obtain a second double-stranded amplification product, wherein one single-stranded amplification product of the second double-stranded amplification product comprises the sequence region A, the amplification product capturing region B, the target sequence, a complementary sequence of the amplification product capturing region C, and a complementary sequence h' of the sequence region h; and
a signal change that can be detected by an instrument is formed, by specifical binding of the A and h' of the single-stranded amplification product with the probe, and whether the target sequence is present is determined based on the signal change.

Preferably, the one single-stranded amplification product of the second double-stranded amplification product further comprises a complementary sequence of the extension blocking region, and the complementary sequence of the extension blocking region is not complementary with any part of the probe.

In a fifth aspect, the present invention provides a use of the probe primer composition of the first aspect, the reaction system of the second aspect, or the kit of the third aspect in PCR detection.

In particular, the present invention provides a use of the probe primer composition of the first aspect, the reaction system of the second aspect, or the kit of the third aspect in single-tube super-multiplex PCR detection.

In a sixth aspect, the present invention provides a use of the probe primer composition of the first aspect, the reaction system of the second aspect, the kit of the third aspect, or the method of the fourth aspect in pathogenic microorganism detection, non-invasive birth defect screening, single nucleotide polymorphism analysis, methylation analysis, gene mutation analysis, or gene typing.

### Brief Description of the Drawings

Fig. 1 shows a primer probe design principle and method in a preferred embodiment 1 of the present invention.
Fig. 2 shows a primer probe design principle and method in a preferred embodiment 2 of the present invention.
Fig. 3 shows a primer probe design principle and method in a preferred embodiment 3 of the present invention.
Fig. 4 shows a melting curve representing RSV (62.25°C) positive in a CY5 channel according to a singleplex PCR detection reaction of Example 1.
Fig. 5 is a view of an effect for detecting multiplex upper respiratory tract pathogens on a SLAN real-time fluorescence quantitative PCR instrument.
Fig. 6 is a view of an effect for detecting multiplex upper respiratory tract pathogens on a SLAN real-time fluorescence quantitative PCR instrument.
Fig. 7 shows melting curves representing RSV (62.38°C) positive and a primer dimer (without melting peak) when a probe P5 is used in a CY5 channel according to a singleplex PCR detection reaction of Comparative Example.
Fig. 8 shows melting curves representing RSV positive (68.48°C, a melting peak with a high Tm value) and a primer dimer (68.52°C, a melting peak with a low Tm value) when a probe P4 is used in an FAM channel according to a singleplex PCR detection reaction of Comparative Example.
Fig. 9 shows a melting curve representing RSV (56.62°C) positive in an FAM channel according to a singleplex PCR detection reaction of Example 3.
Fig. 10 shows a melting curve representing RSV (56.36°C) positive in an FAM channel according to a singleplex PCR detection reaction of Example 4.
Fig. 11 illustrates a reaction principle of Example 5.
Fig. 12 shows that a melting peak with a Tm value of 56.76°C is formed in a PCR reaction well with addition of non small cell lung cancer EGFR exon 20 H773_V774insH mutation positive sample in Example 5.
Fig. 13 illustrates a reaction principle of Example 6.
Fig. 14 shows that a melting peak with a Tm value of 53.67°C is formed by reverse complementation of a probe anchoring region (A) of a forward primer (F9) and a primer anchoring region (A') of a probe (P7) in a PCR reaction well in a CY5 channel in Example 6.
Fig. 15 shows that a melting peak with a Tm value of 52.43°C and a positive melting peak with a Tm value of 62.41°C are formed by reverse complementation of a probe anchoring region (A) of a forward primer (F9) and a primer anchoring region (A') of a probe (P7) in a PCR reaction well with addition of non small cell lung cancer EGFR exon 20 H773_V774insH mutation positive sample in a CY5 channel.
Fig. 16 shows multiplex melting peaks of non-small cell lung cancer EGFR exon 20 T790M mutation, exon 21 L858R mutation, and exon 21 L861Q mutation in Example 7.

### Detailed Description of the Embodiments

Herein, the terms "target sequence", "target nucleic acid", "target nucleic acid sequence", or "target" may be used interchangeablly and mean a portion of the nucleic acid sequence to be amplified, detected, or amplified and detected, which may be annealed with a primer under annealing or amplification conditions.

Herein, the term "upstream primer", also known as forward primer, is an oligonucleotide that extends continuously along a negative strand; and the term "downstream primer" used in the present invention, also known as reverse primer, is an oligonucleotide that is continuously extended along a positive strand, wherein, the positive strand, i.e. a sense strand, also known as a positive-sense strand or a coding strand, is generally located at an upper end of a double-stranded DNA, with a direction of 5'-3' from left to right, a base sequence thereof is substantially the same as a gene mRNA, and a primer binding with this strand is a reverse primer; and the negative strand, i.e. a nonsense strand, also known as a non-coding chain, is complementary with the positive strand, and a primer binding with this strand is a positive primer. It should be understood that, when the designations of the sense strand and the antisense strand are interchanged, the nominations of corresponding forward and reverse primer may also be interchanged accordingly.

Herein, the term "probe" refers to a labeled oligonucleotide for detecting whether a target is present.

Herein, the term "base-complementary pairing" refers to a phenomenon where A and T, A and U, and G and C, etc., in a corresponding relationship, are connected to each other via a hydrogen bond. Correspondingly, "mismatched bases" refer to all other pairing situations except for those specified in "base-complementary pairing", such as a mismatching of A and C, A and G, T and G, or T and C.

### Embodiment 1:

In this embodiment, the probe primer composition comprises a probe, a forward primer, and a reverse primer. The forward primer (F) sequentially comprises a probe anchoring region (A) and a target sequence binding region from 5' end to 3' end, wherein the target sequence binding region is a target specific sequence; and the probe anchoring region (A) is a freely designed sequence that does not bind with any target sequence in a paired manner and has a complementary with that of a primer anchoring region (A') of the probe (P); and there are 0-20 bases spaced between the probe anchoring region (A) and the target sequence binding region.

The probe (P) is a freely designed sequence that does not bind with any target sequence in a paired manner, and comprises the primer anchoring region (A') and a probe signal detection region (H); and the primer anchoring region (A') is complementary with the probe anchoring region (A) of the forward primer (F).

In some specific embodiments, a first detectable label is labeled at a 1^{st}-95^{th} base position away from the primer anchoring region (A'), and a second detectable label is labeled at the base position spaced from the first detectable label by 5-25 bases.

In a specific embodiment, both of the first detectable label and the second detectable label are located in the primer anchoring region (A') or in the probe signal detection region (H), wherein the first detectable label may be a fluorescence reporter, and the second detectable label may be a quencher or other labeling groups that can produce a signal change with the first detectable label via fluorescence resonance energy transfer (FRET).

The reverse primer (R) sequentially comprises an extension blocking region (M), a primer signal detection region (h), and a target sequence binding region from 5' end to 3' end; wherein the extension blocking region (M) is a freely designed sequence that is not identical to or complementary with a base segment of any portion of the probe (P); the target sequence binding region is a target specific sequence; the primer signal detection region (h) is a freely designed sequence that does not bind with any target sequence in a paired manner, and has a sequence partially or completely identical to that of the second binding region (H) of probe (P); an there may be 0-20 bases spaced between the primer signal detection region (h) and the target sequence binding region.

During PCR amplification, the forward primer (F) and reverse primer (R) specifically bind with a target sequence respectively and extend to produce a double-stranded amplification product, wherein one single-stranded amplification product (S1) sequentially comprises the probe anchoring region (A), the target sequence, a complementary sequence (h') of the primer signal detection region, and a complementary sequence (M') of the extension blocking region from 5' end to 3' end.

After the double-stranded amplification product is produced, the probe signal detection region (H) of the probe (P) is complementarily paired with the complementary sequence (h') of the primer signal detection region of the one of single-stranded amplification products (S1), the primer anchoring region (A') of the probe (P) is complementarily paired with the probe anchoring region (A) at 5' end of the single-stranded amplification product (S1), and the complementary sequence (M') of the extension blocking region at 3' end of the single-stranded amplification product (S1) is not complementary with any part of the probe (P); and at this time, the probe (P) is changed from a single-stranded state to a double-stranded state, and a distance between the first detectable label and the second detectable label is changed, thereby generating a signal that may be detected by an instrument.

After the PCR amplification is completed, a melting curve analysis is performed, wherein, during a process of temperature change, the double-stranded product formed by the probe (P) and the single-stranded amplification product (S1) will melt at a certain temperature, at this time, the probe (P) with the first detectable label and the second detectable label will be changed from the double-stranded state to the single-stranded state, so as to cause a change in the distance between the detectable labels, thereby generating a signal that may be detected by the instrument.

Due to the fact that a melting temperature of the double-stranded product formed by the probe (P) and the single-stranded amplification product (S1) is detected during melting curve analysis, it is possible to adjust the melting temperature of the double-stranded product formed by the probe (P) and the single-stranded amplification product (S1) by adjusting a sequence and length of the primer signal detection region (h) of the reverse primer (R) or adjusting a sequence and length of the probe anchoring region A of the forward primer (F) to obtain the single-stranded amplification product (S1) that is partially or completely complementary with the primer anchoring region (A') of the probe (P) and is partially or completely complementary with the probe signal detection region (H) of the probe (P). For example, it is possible to increase or decrease the melting temperature of the double-stranded product formed by the probe (P) and the single-stranded amplification product (S1) by adjusting a length or sequence of the primer signal detection region (h), or adjusting a position at which the complementary sequence (h') of the primer signal detection is complementary with the probe signal detection region (H) of the probe (P).

When a multiplex PCR detection is performed, there may be multigroups of forward primers (F) and reverse primers (R) targeting different target sequences, and different target sequences may be identified by means of fluorescence channels or melting temperatures; and when different target sequences are identified by means of fluorescence channels, different probes (P) may be used for different target sequences, and when different target sequences are identified by means of melting temperatures, a same probe (P) may be shared by different target sequences.

In some specific embodiments, the forward primer (F) has a length of 20-80 bases, wherein the target sequence binding region has a Tm value of 40°C-80°C and a GC content of 20%-80%; and the probe anchoring region (A) has a length of 5-65 bases, a Tm value of 40°C-80°C, and a GC content of 30%-80%.

In some specific embodiments, the reverse primer (R) has a length of 20-80 bases, wherein the extension blocking region (M) has a length of 1-20 bp; the target sequence binding region has a Tm value of 40°C-80°C, and a GC content of 20%-80%; and the primer signal detection region (h) has a length of 5-65 bases, a Tm value of 30°C-80°C, and a GC content of 20%-80%.

In some specific embodiments, the probe (P) has a length of 20-100 bases, wherein the primer anchoring region (A') has a length of 5-65 bases, a Tm value of 40°C-80°C, and a GC content of 30%-80%; more preferably, the primer anchoring region (A') is designed at 3' end of the probe (P); and the probe signal detection region (H) has a length of 5-65 bases, a Tm value of 30°C-80°C, and a GC content of 20%-80%.

An annealing temperature of the probe and forward primer is TmA, an annealing temperature of the probe and the complementary sequence of the reverse primer is TmH, an annealing temperature of the forward primer and the target sequence is Tmb1, and an annealing temperature of the reverse primer and the target sequence is Tmb2. In some specific embodiments, the TmA<Tmb1 and TmA<Tmb2, and/or TmH<Tmb1 and TmH<Tmb2.

When there is no target to be detected, the primer anchoring region (A') of the probe (P) is complementary with the probe anchoring region (A) of the forward primer (F), and the other part of the probe (P) is in a single-stranded state, and the single-stranded flexible probe has a curly shape due to molecular flexibility; and at this time, a distance between the first detectable label and the second detectable label is relatively close, and the efficiency of fluorescence resonance energy transfer is relatively high. When a target to be detected is present, the forward primer (F) and the reverse primer (R) specifically bind with a target sequence respectively and extend to produce a double-stranded amplification product, wherein one single-stranded amplification product (S1) of the amplification product sequentially comprises the probe anchoring region (A), the target sequence, a complementary sequence (h') of the primer signal detection region, and a complementary sequence (M') of the extension blocking region from 5' end to 3' end; the probe signal detection region (H) on the probe (P) is complementarily paired with the complementary sequence (h') of the primer signal detection region of the single-stranded amplification product (S1), the complementary sequence (M') of the extension blocking region at 3' end of the single-stranded amplification product (S1) is not complementary with any part of the probe (P), and the primer anchoring region (A') of the probe (P) is complementarily paired with the probe anchoring region (A) at 5' end of the single-stranded amplification product (S1); and at this time, the distance between the first detectable label and the second detectable label is increased due to a change from the single strand to the double strand, and the efficiency of fluorescence resonance energy transfer is decreased, such that a fluorescence signal change is produced and may be detected by an instrument. After the completion of PCR, when the melting curve analysis is performed, the double-stranded product formed by the probe (P) and the single-stranded amplification product (S1) will melt at a certain temperature, such that the probe (P) with the first detectable label and the second detectable label becomes a single-stranded state, at this time, the molecular flexibility is restored, the distance between the first detectable label and the second detectable label is decreased, and the efficiency of fluorescence resonance energy transfer is increased, such that a fluorescence signal change is produced again, which may be used for melting curve analysis.

### Embodiment 2:

In this embodiment, the probe primer composition comprises a probe, a first forward primer, a second forward primer, and a reverse primer. The first forward primer (F) sequentially comprises an amplification product capturing region (B) and a target sequence binding region from 5' end to 3' end, wherein the target sequence binding region is a target specific sequence; the amplification product capturing region (B) is a freely designed sequence that does not bind with any target sequence in a paired manner; and there are 0-20 bases spaced between the amplification product capturing region (B) and the target sequence binding region. The second forward primer (F0) comprises a probe anchoring region (A) and an amplification product capturing region (B) from 5' end to 3' end.

The probe (P) is a freely designed sequence that does not bind with any target sequence in a paired manner, and comprises the primer anchoring region (A') and a probe signal detection region (H); and the primer anchoring region (A') is complementary with the probe anchoring region (A) of the second forward primer (F0).

In some specific embodiments, a first detectable label is labeled at a 1^{st}-95^{th} base position away from the primer anchoring region (A'), and a second detectable label is labeled at the base position spaced from the first detectable label by 5-25 bases. Both of the first detectable label and the second detectable label are located on a same side of the primer anchoring region (A'), wherein the first detectable label may be a fluorescence reporter, and the second detectable label may be a quencher or other labeling groups that can produce a signal change with the first detectable label via fluorescence resonance energy transfer (FRET).

In some specific embodiments, the reverse primer (R) sequentially comprises an extension blocking region (M), a primer signal detection region (h), and a target sequence binding region from 5' end to 3' end; wherein the extension blocking region (M) is a freely designed sequence that is not identical to or complementary with a base segment of any portion of the probe (P); the target sequence binding region is a target specific sequence; the primer signal detection region (h) is a freely designed sequence that does not bind with any target sequence in a paired manner, and has a sequence partially or completely identical to that of the second binding region (H) of probe (P); and there are 0-20 bases spaced between the primer signal detection region (h) and the target sequence binding region.

During PCR amplification, the first forward primer (F) and reverse primer (R) specifically bind with a target sequence respectively and extend to produce a first double-stranded amplification product, wherein one single-stranded amplification product (S1) sequentially comprises the extension blocking region (M), the primer signal detection region (h), the target sequence, and a complementary sequence (B') of the amplification product capturing region from 5' end to 3' end; and the amplification product capturing region (B) of the second forward primer (F0) may be complementary with the complementary sequence (B') of the amplification product capturing region at 3' end of the first single-stranded amplification product (S1) and extend to obtain a second double-stranded amplification product, wherein one second single-stranded amplification product (D1) sequentially comprises the probe anchoring region (A), the amplification product capturing region (B), the target sequence, a complementary sequence (h') of the primer signal detection region (h), and a complementary sequence (M') of the extension blocking region from 5' end to 3' end.

After the second double-stranded amplification product is produced, the probe signal detection region (H) of the probe (P) is complementarily paired with the complementary sequence (h') of the primer signal detection region of the one second single-stranded amplification products (D1), the primer anchoring region (A') of the probe is complementarily paired with the probe anchoring region (A) at 5' end of the second single-stranded amplification product (D1), and the complementary sequence (M') of the extension blocking region at 3' end of the single-stranded amplification product (S1) is not complementary with any part of the probe (P); and at this time, the probe (P) is changed from a single-stranded state to a double-stranded state, and a distance between the first detectable label and the second detectable label is changed, thereby generating a signal that may be detected by an instrument.

After the PCR amplification is completed, a melting curve analysis is performed, wherein, during a process of temperature change, the double-stranded product formed by the probe (P) and the single-stranded amplification product (D1) will melt at a certain temperature, at this time, the probe (P) with the first detectable label and the second detectable label will be changed from the double-stranded state to the single-stranded state, so as to cause a change in the distance between the detectable labels, thereby generating a signal that may be detected by the instrument.

Due to the fact that a melting temperature of the double-stranded product formed by the probe (P) and the single-stranded amplification product (S1) is detected during melting curve analysis, it is possible to adjust the melting temperature of the double-stranded product formed by the probe (P) and the second single-stranded amplification product (D1) by adjusting a sequence and length of the primer signal detection region (h) of the reverse primer (R) or adjusting a sequence and length of the probe anchoring region A of the second forward primer (F0) to obtain the second single-stranded amplification product (D1) that is partially or completely complementary with the primer anchoring region (A') of the probe (P) and is partially or completely complementary with the probe signal detection region (H) of the probe (P). For example, it is possible to increase or decrease the melting temperature of the double-stranded product formed by the probe (P) and the second single-stranded amplification product (D1) by adjusting a length or sequence of the primer signal detection region (h), or adjusting a position at which the complementary sequence (h') of the primer signal detection is complementary with the probe signal detection region (H) of the probe (P).

When a multiplex PCR detection is performed, there may be multigroups of first forward primers (F) and reverse primers (R) targeting different target sequences, and different target sequences may be identified by means of fluorescence channels or melting temperatures; and when different target sequences are identified by means of fluorescence channels, different probes (P) and different second forward primers (F0) may be used for different target sequences, and when different target sequences are identified by means of melting temperatures, a same probe (P) may be shared by different target sequences.

In some specific embodiments, the first forward primer (F) has a length of 20-80 bases, wherein the target sequence binding region has a Tm value of 40°C-80°C and a GC content of 20%-80%; and the amplification product capturing region (B) has a length of 5-35 bases, a Tm value of 40°C-80°C, and a GC content of 20%-80%.

In some specific embodiments, the second forward primer (F0) has a length of 20-80 bases, wherein the probe anchoring region (A) has a length of 5-65 bases, a Tm value of 40°C-80°C, and a GC content of 30%-80%; and the amplification product capturing region (B) has a length of 5-35 bases, a Tm value of 40°C-80°C, and a GC content of 20%-80%.

In some specific embodiments, the reverse primer (R) has a length of 20-80 bases, wherein the target sequence binding region has a Tm value of 40°C-80°C, and a GC content of 20%-80%; and the primer signal detection region (h) has a length of 5-65 bases, a Tm value of 30°C-80°C, and a GC content of 20%-80%; and the extension blocking region (M) has a length of 1-20 bp.

In some specific embodiments, the probe (P) has a length of 20-100 bases, wherein the primer anchoring region (A') has a length of 5-65 bases, a Tm value of 40°C-80°C, and a GC content of 30%-80%; more preferably, the primer anchoring region (A') is designed at 5' end of the probe (P); and the probe signal detection region (H) has a length of 5-65 bases, a Tm value of 30°C-80°C, and a GC content of 20%-80%.

An annealing temperature of the probe and second forward primer is TmA, an annealing temperature of the probe and the complementary sequence of the reverse primer is TmH, an annealing temperature of the first forward primer and the target sequence is Tmb1, and an annealing temperature of the reverse primer and the target sequence is Tmb2. In some specific embodiments, the TmA<Tmb1 and TmA<Tmb2, and/or TmH<Tmb1 and TmH<Tmb2.

When there is no target to be detected, the primer anchoring region (A') of the probe (P) is complementary with the probe anchoring region (A) of the second forward primer (F0), and the other part of the probe (P) is in a single-stranded state, and a curly shape is formed due to molecular flexibility; and at this time, a distance between the first detectable label and the second detectable label is relatively close, and the efficiency of fluorescence resonance energy transfer is relatively high. When a target to be detected is present, the first forward primer (F) and the reverse primer (R) specifically bind with a target sequence respectively and extend to produce a first double-stranded amplification product, wherein one single-stranded amplification product (S1) sequentially comprises the extension blocking region (M), the primer signal detection region (h), the target sequence, and a complementary sequence (B') of the amplification product capturing region from 5' end to 3' end; and at this time, the amplification product capturing region (B) of the second forward primer (F0) may be complementary with the complementary sequence (B') of the amplification product capturing region at 3' end of the first single-stranded amplification product (S1) and extend to obtain a second double-stranded amplification product, wherein one second single-stranded amplification product (D1) sequentially comprises the probe anchoring region (A), the amplification product capturing region (B), the target sequence, a complementary sequence (h') of the primer signal detection region (h), and a complementary sequence (M') of the extension blocking region from 5' end to 3' end; the probe signal detection region (H) of the probe (P) is complementarily paired with the complementary sequence (h') of the primer signal detection region of the second single-stranded amplification products (D1), the primer anchoring region (A') of the probe (P) is complementarily paired with the probe anchoring region (A) at 5' end of the second single-stranded amplification product (D1), and the complementary sequence (M') of the extension blocking region at 3' end of the single-stranded amplification product (D1) is not complementary with any part of the probe (P); and at this time, the probe (P) is changed from the single-stranded state to a double-stranded state, and the distance between the first detectable label and the second detectable label is changed, thereby generating a signal that may be detected by an instrument.

After the completion of PCR, when the melting curve analysis is performed, the double-stranded product formed by the probe (P) and the second single-stranded amplification product (D1) will melt at a certain temperature, such that the probe (P) with the first detectable label and the second detectable label becomes a single-stranded state, at this time, the distance between the first detectable label and the second detectable label is decreased, and the efficiency of fluorescence resonance energy transfer is increased, such that a fluorescence signal change is produced again, which may be used for melting curve analysis.

### Embodiment 3:

In this embodiment, the probe primer composition comprises a probe, a first forward primer, a second forward primer, a first reverse primer, and a second reverse primer. The first forward primer (F) sequentially comprises a first amplification product capturing region (B) (a forward amplification product capturing region) and a target sequence binding region from 5' end to 3' end, wherein the target sequence binding region is a target specific sequence; the first amplification product capturing region (B) is a freely designed sequence that does not bind with any target sequence in a paired manner; and there are 0-20 bases spaced between the first amplification product capturing region (B) and the target sequence binding region.

The second forward primer (F0) comprises a probe anchoring region (A) and a first amplification product capturing region (B) from 5' end to 3' end.

The probe (P) is a freely designed sequence that does not bind with any target sequence in a paired manner, and comprises the primer anchoring region (A') and a probe signal detection region (H); and the primer anchoring region (A') is complementary with the probe anchoring region (A) of the second forward primer (F0).

In some specific embodiments, a first detectable label is labeled at a 1^{st}-95^{th} base position away from the primer anchoring region (A'), and a second detectable label is labeled at the base position spaced from the first detectable label by 5-25 bases. Both of the first detectable label and the second detectable label are located on the probe signal detection region (H), wherein the first detectable label may be a fluorescence reporter, and the second detectable label may be a quencher or other labeling groups that can produce a signal change with the first detectable label via fluorescence resonance energy transfer (FRET).

The first reverse primer (R) sequentially comprises a second amplification product capturing region (C) (a reverse amplification product capturing region), and a target sequence binding region from 5' end to 3' end, wherein the target sequence binding region is a target specific sequence; the second amplification product capturing region (C) is a freely designed sequence that does not bind with any target sequence in a paired manner; and there are 0-20 bases spaced between the second amplification product capturing region (C) and the target sequence binding region.

The second reverse primer (R0) sequentially comprises an extension blocking region (M), a primer signal detection region (h), and a second amplification product capturing region (C) from 5' end to 3' end.

During first PCR amplification, the first forward primer (F) and the first reverse primer (R) specifically bind with a target sequence respectively and extend to produce a first double-stranded amplification product, wherein one single-stranded amplification product (S1) sequentially comprises the first amplification product capturing region (B), the target sequence, and a complementary sequence (C') of the second amplification product capturing region (C) from 5' end to 3' end, and wherein another single-stranded amplification product (S1') sequentially comprises the second amplification product capturing region (C), the target sequence, and a complementary sequence (B') of the first amplification product capturing region from 5' end to 3' end.

During second PCR amplification, the first double-stranded amplification product is taken as a template, and the first amplification product capturing region (B) of the second forward primer (F0) may be complementary with the complementary sequence (B') of the first amplification product capturing region of the another first single-stranded amplification product (S1') and extend; and the secopnd amplification product capturing region (C) of the second reverse primer (R0) may be complementary with the complementary sequence (C') of the second amplification product capturing region of the one first single-stranded amplification product (S1) and extend to obtain a second double-stranded amplification product, wherein one second single-stranded amplification product (D1) sequentially comprises the probe anchoring region (A), the first amplification product capturing region (B), the target sequence, the complementary sequence (C') of the second amplification product capturing region, a complementary sequence (h') of the primer signal detection region (h), and a complementary sequence (M') of the extension blocking region from 5' end to 3' end.

After the second double-stranded amplification product is produced, the probe signal detection region (H) of the probe (P) is complementarily paired with the complementary sequence (h') of the primer signal detection region of the one second single-stranded amplification products (D1), the primer anchoring region (A') of the probe is complementarily paired with the probe anchoring region (A) at 5' end of the second single-stranded amplification product (D1), and the complementary sequence (M') of the extension blocking region at 3' end of the single-stranded amplification product (S1) is not complementary with any part of the probe (P); and at this time, the probe (P) is changed from a single-stranded state to a double-stranded state, and a distance between the first detectable label and the second detectable label is changed, thereby generating a signal that may be detected by an instrument.

Due to the fact that a melting temperature of the double-stranded product formed by the probe (P) and the second single-stranded amplification product (D1) is detected during melting curve analysis, it is possible to adjust the melting temperature of the double-stranded product formed by the probe (P) and the second single-stranded amplification product (D1) by adjusting a sequence and length of the primer signal detection region (h) of the second reverse primer (R0) or adjusting a sequence and length of the probe anchoring region (A) of the second forward primer (F0) to obtain the second single-stranded amplification product (D1) that is partially complementary or completely complementary with the primer anchoring region (A') of the probe (P) and is partially or completely complementary with the probe signal detection region (H) of the probe (P). For example, it is possible to increase or decrease the melting temperature of the double-stranded product formed by the probe (P) and the second single-stranded amplification product (D1) by adjusting a length or sequence of the primer signal detection region (h), or adjusting a position at which the complementary sequence (h') of the primer signal detection region is complementary with the probe signal detection region (H) of the probe (P).

When a multiplex PCR detection is performed, there may be multigroups of first forward primers (F) and first reverse primers (R) targeting different target sequences, and different target sequences may be identified by means of fluorescence channels or melting temperatures; and when different target sequences are identified by means of fluorescence channels, different probes (P) as well as different second forward primers (F0) and second reverse primers (R0) may be used for different target sequences, and when different target sequences are identified by means of melting temperatures, a same probe (P), or a same second forward primer (F0) and a same second reverse primer (R0) may be shared by different target sequences.

In some specific embodiments, the first forward primer (F) has a length of 20-80 bases, wherein the target sequence binding region has a Tm value of 40°C-80°C and a GC content of 20%-80%; and the amplification product capturing region (B) has a length of 5-35 bases, a Tm value of 40°C-80°C, and a GC content of 20%-80%.

In some specific embodiments, the second forward primer (F0) has a length of 20-80 bases, wherein the probe anchoring region (A) has a length of 5-65 bases, a Tm value of 40°C-80°C, and a GC content of 30%-80%; and the amplification product capturing region (B) has a length of 5-35 bases, a Tm value of 40°C-80°C, and a GC content of 20%-80%.

In some specific embodiments, the first reverse primer (R) has a length of 20-80 bases, wherein the target sequence binding region has a Tm value of 40°C-80°C, and a GC content of 20%-80%; and the primer signal detection region (h) has a length of 5-65 bases, a Tm value of 30°C-80°C, and a GC content of 20%-80%.

In some specific embodiments, the second reverse primer has a length of 20-80 bases, wherein the primer signal detection region has a length of 5-65 bases.

In some specific embodiments, the primer signal detection region of the second reverse primer has a Tm value of 30-80°C.

In some specific embodiments, the primer signal detection region of the second reverse primer has a GC content of 20-80%.

In some specific embodiments, the extension blocking region of the second reverse primer has a length of 1-20 bp.

In some specific embodiments, the probe (P) has a length of 20-100 bases, wherein the primer anchoring region (A') has a length of 5-65 bases, a Tm value of 40°C-80°C, and a GC content of 30%-80%; more preferably, the primer anchoring region (A') is designed at 5' end of the probe (P); and the probe signal detection region (H) has a length of 5-65 bases, a Tm value of 30°C-80°C, and a GC content of 20%-80%.

An annealing temperature of the probe and second forward primer is TmA, an annealing temperature of the probe and the complementary sequence of the second reverse primer is TmH, an annealing temperature of the first forward primer and the target sequence is Tmb1, and an annealing temperature of the first reverse primer and the target sequence is Tmb2. In some specific embodiments, the TmA<Tmb1 and TmA<Tmb2, and/or TmH<Tmb1 and TmH<Tmb2.

When there is no target to be detected, the primer anchoring region (A') of the probe (P) is complementary with the probe anchoring region (A) of the second forward primer (F0), and the other part of the probe (P) is in a single-stranded state, and a curly shape is formed due to molecular flexibility; and at this time, a distance between the first detectable label and the second detectable label is relatively close, and the efficiency of fluorescence resonance energy transfer is relatively high. When a target to be detected is present, the first forward primer (F) and the first reverse primer (R) specifically bind with a target sequence respectively and extend to produce a first double-stranded amplification product, and the second forward primer (F0) and the second reverse primer (R0) further amplify the first double-stranded amplification product to obtain a second double-stranded amplification product, wherein one second single-stranded amplification product (D1) sequentially comprises the probe anchoring region (A), the first amplification product capturing region (B), the target sequence, a complementary sequence (C') of the second amplification product capturing region, a complementary sequence (h') of the primer signal detection region, and a complementary sequence (M') of the extension blocking region from 5' end to 3' end; the probe signal detection region (H) of the probe (P) is complementarily paired with the complementary sequence (h') of the primer signal detection region of the second single-stranded amplification products (D1), the primer anchoring region (A') of the probe (P) is complementarily paired with the probe anchoring region (A) at 5' end of the one second single-stranded amplification product (D1), and the complementary sequence (M') of the extension blocking region at 3' end of the second single-stranded amplification product (D1) is not complementary with any part of the probe (P); and at this time, the probe (P) is changed from a single-stranded state to a double-stranded state, and the distance between the first detectable label and the second detectable label is changed, thereby generating a signal that may be detected by an instrument. After the completion of PCR, when the melting curve analysis is performed, the double-stranded product formed by the probe (P) and the second single-stranded amplification product (D1) will melt at a certain temperature, such that the probe (P) with the first detectable label and the second detectable label becomes a single-stranded state, at this time, the distance between the first detectable label and the second detectable label is decreased, and the efficiency of fluorescence resonance energy transfer is increased, such that a fluorescence signal change is produced again, which may be used for melting curve analysis.

Compared with the prior art, the advantages of the technical solution described in the present invention lie in:
Multiplex detection: when the method described in the present invention is used, both of a fluorescence channel analysis and melting temperature analysis in a single tube reaction may be simultaneously performed in two dimensions, that is, different targets may be detected by using a same fluorescence channel and by means of melting temperature characteristics; or, by using different fluorescent channels, the detection of target species may be achieved by multiplying the number of the fluorescent channels by the melting temperature characteristic, for example, when two melting temperature detection points are set in one fluorescence channel, if there are two fluorescence channels, 2×2 target sequences may be detected, thereby achieving a multiplex detection of more target sequences;
Low fluorescence background: for each fluorescence channel, only one probe is used, and then a differentiation may be achieved by the different melting temperatures of the amplification products, thereby greatly reducing the fluorescence background in the PCR reaction and improving reaction sensitivity;
Adjustable melting temperature: in the method described in the present invention, two ends of the probe are simultaneously combined with two ends of the amplification product and then the probe is stretched and light is emitted, and thus, by adjusting the number and sequence of bases in the probe anchoring region (A) and the complementary sequence (h') of the primer signal detection region on the amplification product via primer design, it is possible to obtain different melting temperatures, such that different targets that share a same probe have different melting temperatures; and for example, by selecting sequences at different positions of the probe as signal detection region sequences, it is possible to obtain double-stranded products complementary with different parts of the probe, so as to differentiate the melting temperatures of different target products.

Good inclusiveness: compared with a Taqman hydrolysis probe method which requires three parts that are complementarily paired with the template, in the primer probe design method of the present invention, oly two parts, namely the forward primer (F) and the reverse primer (R) are complementarily paired with the target sequence, such that when there are many high variation regions in the target sequence (such as viruses or bacterial genomes), the primer probe of the present invention has a better inclusiveness and a lower design difficulty;
Low cost: by means of the method of the present invention, a detection may be completed in a single tube reaction by only requiring a fluorescence quantitative PCR instrument, without the need for additional downstream instruments and without the need for chips or multiple reaction tubes for dividing reactions, thereby greatly reducing the cost of instruments and consumables, meanhwile, the number of fluorescence probes in the reagent is reduced, thereby greatly reducing the cost of the reagent, and allowing a large-scale promotion to be possible;
Single tube reaction: the method has low sample consumption, is particularly suitable for the detection of a rare sample, and can greatly increase a concentration of the sample added to the detection reaction, so as to improve detection sensitivity, and provide the possibility for a subsequent extraction-free one-tube detection;
Easy and simple operation: only a fluorescence quantitative PCR instrument is required for detection, without subsequent steps such as for capillary electrophoresis or nucleic acid hybridization, etc., meanwhile, the preparation is simple, and there is no need to manual operate after the instrument is enabled;
No easy to cause pollution: in the method described in the present invention, the instrument is completely closed after the sample addition is completed, without the need to open the cover for subsequent detection, thereby greatly reducing the possibility of polluting an experimental environment;
High sensitivity: the method described in the present invention has a very low requirement for the length of the target sequence, when the target type is a short fragment nucleic acid, such as a free nucleic acid, a higher sensitivity is achieved in detection for a shorter target sequence length; and
Wide applicable range: the present invention is suitable for nucleic acid detection of various sample types, including a serum sample, a plasma sample, a whole blood sample, a sputum sample, a swab sample, a lavage fluid sample, a fresh tissue sample, a formalin fixed paraffin embedded tissue (FFPE), and a target (such as bacteria, viruses, gene mutations), etc., such that clinical units, including primary hospitals, can quickly carry out a common target detection (such as adetection for pathogenic microorganism infection), which may be assisted with other detection and definite diagnostic means, thereby quickly providing diagnostic evidence and medication schemes for clinical practice, reducing mental and economic burden of patients, and achieving an objective of precision medicine.

The used reagents without specifying the manufacturer are all conventional products that may be commercially available.

### Example

### Example 1: Detection for respiratory syncytial virus (RSV) by using a probe primer system

**Table 1. Probe primer**

| Name of primer probe | Number | Nucleotide sequence (5'-3') | Label |
|---|---|---|---|
| P1 (probe) | SEQ ID NO:1 | CCCTGGGAGCTCATGGAGGTACTG GTCCTGTCGACGCACG | T16-CY5, T28-BHQ1 3'Spacer C3 |
| R1 (Reverse primer) | SEQ ID NO:2 | TTATTTCATGGAGGTACTGGTCCTGT CGAAATATTGCAGATCCWACACCTA ACAA | / |
| F1 (Forward primer) | SEQ ID NO:3 | GCTCCCAGGGTTTAACATTAAGCAA GAAGAGGAAAMGAAGAT | / |

The forward primer F1 (SEQ ID NO: 3) is a specific primer designed for a target sequence of respiratory syncytial virus, and the F1 has a total length of 42 bp, wherein a 1^{st} to 29^{th} bases at 3' end thereof serve as a target sequence binding region, and a 1^{st} to 10^{th} bases at 5' end thereof serve as a probe anchoring region (A), which is complementary with a sequence of a 1^{st} to 10^{th} bases (i.e. a primer anchoring region (A')) at 5' end of the probe P1 (SEQ ID NO: 1).

The reverse primer R1 (SEQ ID NO: 2) is a specific primer designed for the target sequence of respiratory syncytial virus, and the R1 has a total length of 55 bp, wherein a 1^{st} to 24^{th} bases at 3' end thereof serve as a target sequence binding region, a 1^{st} to 5^{th} bases at 5' end thereof serve as an extension blocking region (M), and a 6^{th} to 28^{th} bases at 5' end thereof serve as a primer signal detection region (h), which is identical to a sequence of a 8^{th} to 30^{th} bases at 3' end of the probe P1 (SEQ ID NO: 1)

A reaction system comprises the above primer probe, a template, a DNA polymerase, a reverse transcriptase, and a reaction buffer, and is used for target specific amplification and fluorescence signal detection. The reaction system is involved in the following steps:
(1) Firstly, in a cycle of PCR amplification reaction, the forward primer (F1) and the reverse primer (R1) may specifically amplify a target nucleic acid sequence. After amplification, a double-stranded pre-amplification product will be obtained, wherein one single-stranded amplification product (S1) comprises the probe anchoring region (A), the target sequence, a complementary sequence (h') of the primer signal detection region, and a complementary sequence (M') of the extension blocking region from 5' end to 3' end; and the probe signal detection region (H) of the probe (P) is complementarily paired with the complementary sequence (h') of the primer signal detection region of the single-stranded amplification product (S1), the primer anchoring region (A') of the probe (P) is paired with the probe anchoring region (A) at 5' end of the single-stranded amplification product (S1) in a reverse complementary manner, and the complementary sequence (M') of the extension blocking region at 3' end of the single-stranded pre-amplification product (S1) is not complementary with any part of the probe (P). At this time, the probe (P) is changed from a single-stranded state to a double-stranded state, and a distance between the first detectable label and the second detectable label is changed, thereby generating a signal that may be detected by the instrument.
(2) After the PCR amplification is completed, a melting curve analysis is performed, and the double-stranded product formed by the probe (P) and the single-stranded amplification product (S1) will melt at a certain temperature, at this time, the probe (P) with the first detectable label and the second detectable label will be changed to the single-stranded state, thereby generating a fluorescence signal change that may be detected by the instrument.

The reaction system for respiratory syncytial virus detection comprises the following components:

**Table 2. Reaction system**

| Reagents and components | | Concentration |
|---|---|---|
| A system for singleplex detection for respiratory syncytial virus | 2× PCR Reaction Buffer | 1× |
| | DNA Polymerase | 2U |
| | Reverse transcriptase | 5U |
| | Probe (P1) | 400 nM |
| | Forward primer (F1) | 500 nM |
| | Reverse primer (R1) | 100 nM |
| | Nucleic acid template | 0.1 ng-60 ng |
| | Ultra-pure water | Adding to 20 µL |

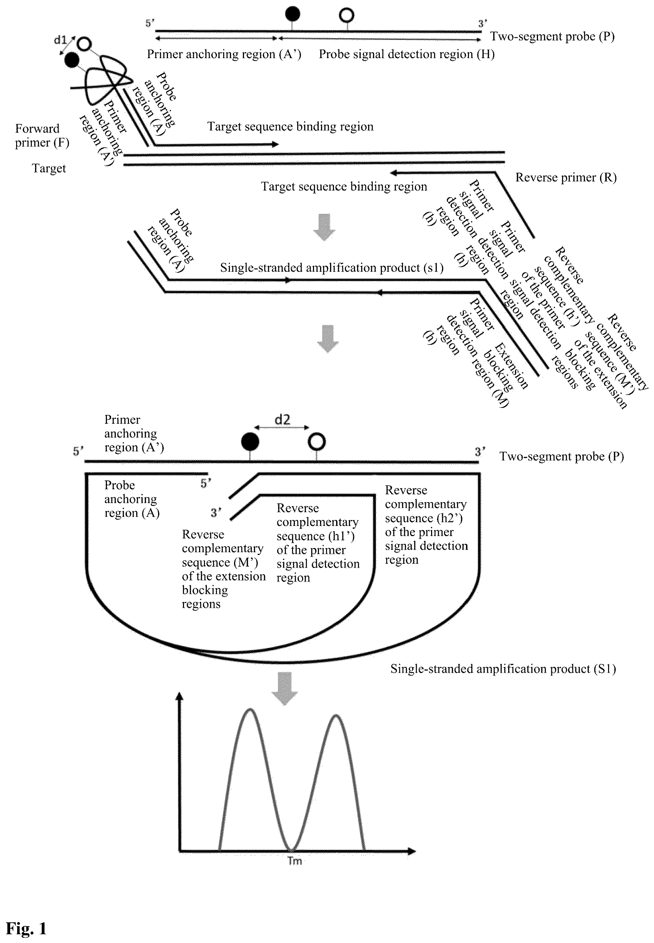

Among others, the nucleic acid template was devided from an in-vitro transcribed RNA sample. The reaction buffer and a primer probe premixed solution were mixed according to the reaction system described in Table 2, a nucleic acid was extracted from a sample to be detected and was added into the prepared reaction system, and then a PCR amplification and a melting curve analysis were performed. A pathogen type in the sample may be judged based on a characteristic Tm value of the target in a specific detection channel. A reaction principle was shown in Fig. 1.

The detection was performed by using an SLAN real-time fluorescence quantitative PCR instrument and reagent consumables from Shanghai Hongshi Medical Technology Co., Ltd. The 2xPCR reaction buffer comprises 3 mM MgCl₂, 30 mM Tris-HCl with a pH of 8.3, 0.5 mM dNTP, and 70 mM (NH₄)₂SO₄.

After a PCR tube was sealed by a cap, the sample was gently and uniformly mixed, and centrifuged for a short time and then left for 5 min at a room temperature. The PCR tube was put in a handheld centrifuge again, centrifuged for a short time, and then transferred to a tray of the fluorescence quantitative PCR instrument. The procedure used was as follows: performing reverse transcription at 55 °C for 15 minutes; performing pre-denaturation at 95°C for 2 minutes; performing denaturation at 94 °C for 10 seconds, and performing annealing and extension at 56 °C for 30 seconds, for a total of 45 cycles, without lighting; performing denaturation at 94 °C for 150 seconds, and perfroming incubation at a constant temperature of 40°C for 300 seconds; and performing melting curve analysis at 40-85°C, with a heating rate of 0.05°C/s and with lighting.

### Data analysis:

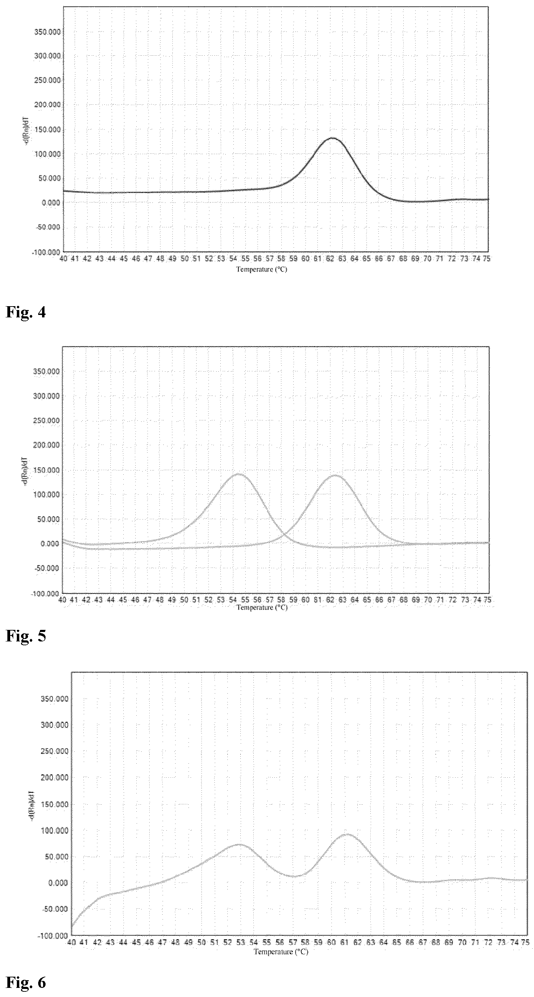

The detection results were subjected to data analysis by using an analysis software of the SLAN real-time fluorescence quantitative PCR instrument from Shanghai Hongshi Medical Technology Co., Ltd. The results were interpreted via Tm values of the melting curves. Fig. 4 is a melting curve of a singleplex PCR respiratory syncytial virus positive sample with the reverse primers (R) with different signal detection regions. Fig. 4 shows that a melting peak with a Tm value of 62.25°C is formed in a PCR reaction well with addition of a RSV positive template in a CY5 channel.

### Example 2: Detection for respiratory syncytial virus (RSV) and influenza A virus (IAV) by using a probe primer system

**Table 3. Probe primer**

| Name of primer probe | Number | Nucleotide sequence (5'-3') | Label |
|---|---|---|---|
| P2 (Probe) | SEQ ID NO:4 | CGCAAGTGAGGCGACTACGTGA GCGATGGACGGAGGCAGGA | T16-VIC T27-BHQ2 3' Spacer C3 |
| F3 (Forward primer) | SEQ ID NO:5 | GCCTCACTTGCGAAAAAGCAACC AACAACATTCATTG | / |
| R3 (Reverse primer) | SEQ ID NO:6 | TTTTTCTACGTGAGCGATGGAAA GCAATTCAGCATCACAGAC | / |
| F4 (Forward primer) | SEQ ID NO:7 | GCCTCACTTGCGAAAATAATCAG GCACGAGAACAG | / |
| R4 (Reverse primer) | SEQ ID NO:8 | TTTTTGACTACGTGAGCGATGGA CGGAGGAAAAGACTAGCAACCT CCATGGC | / |

Both of the forward primer F3 (SEQ ID NO: 5) and the reverse primer R3 (SEQ ID NO: 6) are specific primers designed for a target sequence of respiratory syncytial virus. The F3 has a total length of 37 bp, wherein a 1^{st} to 22^{nd} bases at 3' end thereof serve as a target sequence binding region, and a 1^{st} to 12^{th} bases at 5' end thereof serve as a probe anchoring region (A), which is reverse complementary with a sequence of a 1^{st} to 12^{th} bases (i.e. a primer anchoring region (A')) at 5' end of the probe P2 (SEQ ID NO: 4). The R3 has a total length of 42 bp, wherein a 1^{st} to 19^{th} bases at 3' end thereof serve as a target sequence binding region, a 1^{st} to 5^{th} bases at 5' end thereof serve as an extension blocking region, and a 6^{th} to 20^{th} bases at 5' end thereof serve as a primer signal detection region (h), which is identical to a sequence of a 13^{rd} to 27^{th} bases at 3' end of the probe P2 (SEQ ID NO: 4).

Both of the forward primer F4 (SEQ ID NO: 7) and the reverse primer R4 (SEQ ID NO: 8) are specific primers designed for a target sequence of influenza A virus. The F4 has a total length of 35 bp, wherein a 1^{st} to 20^{th} bases at 3' end thereof serve as a target sequence binding region, a 1^{st} to 12^{th} bases at 5' end thereof serve as a probe anchoring region (A), which is reverse complementary with a sequence of a 1^{st} to 12^{th} bases (i.e. a primer anchoring region (A')) at 5' end of the probe P2 (SEQ ID NO: 4). The R4 has a total length of 52 bp, wherein a 1^{st} to 19^{th} bases at 3' end thereof serve as a target sequence binding region, a 1^{st} to 5^{th} bases at 5' end thereof serve as an extension blocking region (M), and a sequence a 6^{th} to 29^{th} bases at 5' end thereof is identical to a sequence of a 6^{th} to 29^{th} bases at 3' end of the probe P2 (SEQ ID NO: 4).

A reaction principle is shown in Fig. 1.

A reaction system for detection for respiratory syncytial virus (RSV) and influenza A virus (IAV) comprises the probe primer composition as shown in Table 3, a template, a DNA polymerase, a reverse transcriptase, and a reaction buffer, and is used for target specific amplification and fluorescence signal detection. Specifically, the reaction system comprises the following components:

**Table 4. Reaction system**

| Reagents and components | | Concentration |
|---|---|---|
| A system for detection for respiratory syncytial virus, and influenza A virus | 2× PCR Reaction Buffer | 1× |
| | DNA Polymerase | 2U |
| | Reverse transcriptase | 5U |
| | Forward Primer (F3) | 500 nM |
| | Reverse primer (R3) | 100 nM |
| | Probe (P2) | 400 nM |
| | Forward primer (F4) | 500 nM |
| | Reverse primer (R4) | 100 nM |
| | Nucleic acid template | 0.1 ng-60 ng |
| | Ultra-pure water | Adding to 20 µL |

The nucleic acid template was devided from an in-vitro transcribed RNA sample. The reaction buffer and a primer probe premixed solution were mixed according to the reaction system described in Table 4, a nucleic acid was extracted from a sample to be detected and was added into the prepared reaction system, and then a PCR amplification and a melting curve analysis were performed. A pathogen type in the sample may be judged based on a characteristic Tm value of the target in a specific detection channel.

The detection was performed by using an SLAN real-time fluorescence quantitative PCR instrument and reagent consumables from Shanghai Hongshi Medical Technology Co., Ltd. The 2xPCR reaction buffer comprises 3 mM MgCl₂, 30 mM Tris-HCl with a pH of 8.3, 0.5 mM dNTP, 70 mM (NH₄)₂SO₄, etc.

In-vitro transcribed RNAs from individual target sequence were used as positive samples, and two target sequence templates of a same fluorescent channel were mixed for detection, to verify a detection ability for mixed virus infection. The ultra-pure water was used as a no template control (NTC).

### Reaction preparation:

After the preparation of the samples was completed, the reaction system was prepared according to the ratios described in Table 4. An experimental principle of this example (i.e. a solution of the present invention) was shown in Fig. 1.

PCR amplification and melting curve analysis:
After a PCR tube was sealed by a cap, the sample was gently and uniformly mixed, and centrifuged for a short time and then left for 5 min at a room temperature. The PCR tube was put in a handheld centrifuge again, centrifuged for a short time, and then transferred to a tray of the fluorescence quantitative PCR instrument. The procedure used was as follows: performing reverse transcription at 55 °C for 15 minutes; performing pre-denaturation at 95°C for 2 minutes; performing denaturation at 94 °C for 10 seconds, and performing annealing and extension at 56 °C for 30 seconds, for a total of 45 cycles, without lighting; performing denaturation at 94 °C for 150 seconds, and perfroming incubation at a constant temperature of 40°C for 300 seconds; and performing melting curve analysis at 40-85°C, with a heating rate of 0.05°C/s and with lighting.

### Data analysis:

The results were interpreted via Tm values of the melting curves. The detection results were subjected to data analysis by using an analysis software of the SLAN real-time fluorescence quantitative PCR instrument from Shanghai Hongshi Medical Technology Co., Ltd.

Figs. 5-6 are melting curves of individual respiratory virus positive samples according to multiple PCR. Fig. 5 shows that dual melting peaks of RSV (52.93°C) and IAV (61.17°C) appear in a PCR reaction well with addition of an IAV positive template and a RSV positive template in a VIC channel, respectively. Fig. 6 shows that dual melting peaks of RSV (52.93°C) and IAV (61.17°C) simultaneously appear in a PCR reaction well with addition of both an IAV positive template and a RSV positive template in a VIC channel. It can be seen that, when the solution of the present invention is used to detect 2 pathogens, a good specificity is achieved, the melting curve has a single pattern, and the Tm values of two positive targets in a same channel may be clearly differentiated.

**Comparative Example:** Detection for respiratory syncytial virus (RSV) by using a primer probe system in a situation where a primer dimer is extremely easily formed:

**Table 5. Probe primer**

| Name of primer probe | Number | Nucleotide sequence (5'-3') | Label |
|---|---|---|---|
| P5 (probe) | SEQ ID NO:21 | CCCTGGGAGCTCATGGAGGTAC TGGTCCTGTCGAC | T16-CY5, T28-BHQ2 3' Spacer C3 |
| P4 (probe) | SEQ ID NO:10 | AGCGACGGTGCCGAGAGGGT | 3'BHQ1 |
| | | | 5'FAM |
| R5 (Reverse primer) | SEQ ID NO:11 | TTATTTCATGGAGGTACTGGTCC TGTCGAAATATTGCAGATACTCC ACCTCATCA | |
| F5 (Forward primer) | SEQ ID NO:12 | GCTCCCAGGGTTTAACAGGCTG CAAGAAGAGGACTAGAAGAT | |
| R2 (Reverse primer) | SEQ ID NO: 13 | AGCGACGGTGCCGAGAGGGTA AATATTGCAGATACTCCACCTCA TCA | / |
| F2 (Forward primer) | SEQ ID NO:14 | AACAGGCTGCAAGAAGAGGAC TAGAAGAT | / |
| F2-1 (Forward primer) | SEQ ID NO:15 | TTTTTTTTTTGATGAGGTGGAGT ATCTGCAATA | / |

In the above probe primer:
the forward primer F5 (SEQ ID NO: 12) is a specific primer designed for a target sequence of respiratory syncytial virus, and the F5 has a total length of 42 bp, wherein a 1^{st} to 29^{th} bases at 3' end thereof serve as a target sequence binding region, and a 1^{st} to 10^{th} bases at 5' end thereof serve as a probe anchoring region (A), which is reverse complementary with a sequence of a 1^{st} to 10^{th} bases (i.e. a primer anchoring region (A')) at 5' end of the probe P3 (SEQ ID NO: 9);
the reverse primer R5 (SEQ ID NO: 11) is a specific primer designed for the target sequence of respiratory syncytial virus, and the R5 has a total length of 55 bp, wherein a 1^{st} to 24^{th} bases at 3' end thereof serve as a target sequence binding region, a 1^{st} to 5^{th} bases at 5' end thereof serves as an entension blocking region (M), and a 6^{th} to 28^{th} bases at 5' end thereof serves as a primer signal detection region (h), which is identical to a sequence of a 3^{rd} to 25^{th} bases at 3' end of the probe P3 (SEQ ID NO: 9);
the forward primer F2 (SEQ ID NO: 14) is a specific primer designed for the target sequence of respiratory syncytial virus, and the F2 has a total length of 29 bp, and is identical to a 1^{st} to 29^{th} base at 3' end of F5;
the reverse primer R2 (SEQ ID NO: 13) is a specific primer designed for the target sequence of respiratory syncytial virus, and the R2 has a total length of 47 bp, wherein a 1^{st} to 24^{th} bases at 3' end thereof serve as a target sequence binding region and is identical to a sequence of a 1^{st} to 24^{th} bases at 3' end of R5, and a sequence of a 1^{st} to 20^{th} bases at 5' end thereof is completely identical to a base sequence of the probe P4 (SEQ ID NO: 10);
the forward primer F2-1 (SEQ ID NO: 15) is a specific dimer primer designed for the target sequence of respiratory syncytial virus, and the F2-1 has a total length of 33 bp, wherein a 1^{st} to 24^{th} bases at 3' end thereof is complementary with a sequence of a 1^{st} to 24^{th} bases at 3' end of the reverse primer R5 (SEQ ID NO: 11);
the probe P5 (SEQ ID NO: 21) has an amino acid sequence consistent with that of the probe P3, except that the labeling group of probe P5 is different from that of the probe P3. The probe P5 is labeled thereon with a CY5 detectable label; and
the probe P4 (SEQ ID NO: 10) is a self-designed sequence that is not complementary with any part of the target sequence, and the P4 has a total length of 20 bp, and is identical to a sequence of a 1^{st} to 20^{th} bases at 5' end of R2.

When a pathogen target is specifically amplified, the forward primer (F5) and the reverse primer (R5) amplify a target nucleic acid to obtain a double-stranded amplification product, wherein one single-stranded pre-amplification product (S1) sequentially comprises the probe anchoring region (A), the target sequence, a reverse complementary sequence (h') of the primer signal detection region, and a reverse complementary sequence (M') of the extension blocking region; and the probe signal detection region (H) of the probe (P5) is complementarily paired with the reverse complementary sequence (h') of the primer signal detection region of the single-stranded pre-amplification product (S1), the primer anchoring region (A') of the probe is paired with the probe anchoring region (A) at 5' end of the single-stranded pre-amplification product (S1) in a reverse complementary manner, and the reverse complementary sequence (M') of the extension blocking region at 3' end of the single-stranded pre-amplification product (S1) is not complementary with any part of the probe (P5). At this time, the probe (P5) is changed from a single-stranded state to a double-stranded state, and a distance between the first detectable label and the second detectable label is changed, thereby generating a signal that may be detected by the instrument; and after the amplification is completed, a melting curve analysis is performed, wherein, during a process of temperature change, the double-stranded product formed by the probe (P5) and the single-stranded product will melt at a certain temperature, at this time, the probe (P5) with the first detectable label and the second detectable label will be changed to the single-stranded state, thereby generating a signal change that may be detected by the instrument

When the pathogen target is specifically amplified, the F2 (SEQ ID NO: 14) and the R2 (SEQ ID NO: 13) amplify the target nucleic acid to obtain a double-stranded pre-amplification product, wherein a 5' end of one single-stranded pre-amplification product is reverse complementary with the probe P4 (SEQ ID NO: 10). At this time, a distance between the first detectable label and the second detectable label at two ends of the probe P4 (SEQ ID NO: 10) is increased due to a change from single strand to double strand, and the efficiency of fluorescence resonance energy transfer is decreased, such that a fluorescence signal change is produced and may be detected by an instrument. After the amplification is completed, a melting curve analysis is performed, and the double-stranded product formed with the probe P4 (SEQ ID NO: 10) will melt at a certain melting temperature, at this time, the probe P4 (SEQ ID NO: 10) with the first detectable label and the second detectable label will be changed to a single-stranded state, such that a fluorescence signal change is produced and may be detected by the instrument.

When there is no pathogen targe in the sample to be detected, a double-stranded primer dimer is obtained by reverse complementary amplification of the forward primer F2-1 (SEQ ID NO: 15) and the reverse primer R5 (SEQ ID NO: 11), and since the forward primer F2-1 (SEQ ID NO: 15) does not contain a probe anchoring region (A) at 5' end thereof, neither of the single strands of the obtained double-stranded primer dimer contains the probe anchoring region (A), then the reverse complementary sequence (h') of the primer signal detection region cannot bind with the probe (P5) without the probe anchoring region (A). After amplification, a melting curve analysis is performed, and no positive signal is generated due to the absence of a double-stranded product formed with the probe P5 (SEQ ID NO: 21).

When there is no pathogen targe in the sample to be detected, a double-stranded primer dimer is obtained by reverse complementary amplification of the forward primer F2-1 (SEQ ID NO: 15) and the reverse primer R5 (SEQ ID NO: 13), wherein one single-stranded product may be reverse complementary with the probe P4 (SEQ ID NO: 10), such that a distance between the first detectable label and the second detectable label at two ends of the probe P4 (SEQ ID NO: 10) is increased due to a change from single strand to double strand, and the efficiency of fluorescence resonance energy transfer is decreased, and thus a fluorescence signal change is produced and may be detected by an instrument, thereby generating a false positive signal. After the amplification is completed, a melting curve analysis is performed, and the double-stranded product formed with the probe P4 (SEQ ID NO: 10) will melt at a certain melting temperature, at this time, the probe P4 (SEQ ID NO: 10) with the first detectable label and the second detectable label will be changed to a single-stranded state, such that a fluorescence signal change is produced and may be detected by the instrument, thereby generating a false positive signal.

A reaction system for detection for respiratory syncytial virus (RSV) in a situation where a primer dimer is extremely easily formed, comprises the primer probe as shown in above Table 5, a template, a DNA polymerase, a reverse transcriptase, and a reaction buffer, and is used for target specific amplification and fluorescence signal detection. The reaction system is involved in the following steps:
In a cycle of PCR amplification reaction, the forward primer (F5) and the reverse primer (R5) may specifically amplify a target nucleic acid sequence. After amplification, a double-stranded amplification product will be obtained, wherein one single-stranded amplification product (S1) comprises the probe anchoring region (A), the target sequence, a reverse complementary sequence (h') of the primer signal detection region, and a reverse complementary sequence (M') of the extension blocking region from 5' end to 3' end; the probe signal detection region (H) of the probe (P5) is complementarily paired with the reverse complementary sequence (h') of the primer signal detection region of the single-stranded amplification product (S1), the primer anchoring region (A') of the probe (P5) is paired with the probe anchoring region (A') at 5' end of the single-stranded amplification product (S1) in a reverse complementary manner, and the reverse complementary sequence (M') of the extension blocking region at 3' end of the single-stranded amplification product (S1) is not complementary with any part of the probe (P5). At this time, the probe (P5) is changed from a single-stranded state to a double-stranded state, and a distance between the first detectable label and the second detectable label is changed, thereby generating a signal that may be detected by the instrument. After the PCR amplification is completed, a melting curve analysis is performed, and the double-stranded product formed by the probe (P5) and the single-stranded amplification product (S1) will melt at a certain temperature, at this time, the probe (P5) with the first detectable label and the second detectable label will be changed to the single-stranded state, thereby generating a signal change that may be detected by the instrument.

In a cycle of PCR amplification reaction, a double-stranded primer dimer is obtained by reverse complementary amplification of the forward primer (F2-1) and the reverse primer (R5), and since the double-stranded primer dimer does not contain the probe anchoring region (A), then the reverse complementary sequence (h') of the primer signal detection region cannot bind with the probe (P5) without the probe anchoring region (A). After amplification, a melting curve analysis is performed, and no positive signal is generated due to the absence of a double-stranded product formed with the probe (P5).

In a cycle of PCR amplification reaction, the forward primer (F2) and the reverse primer (R2) may amplify a target nucleic acid sequence. After amplification, one single-stranded amplification product may be reversely complementary with the probe (P4), such that a distance between the fluoresce group and the quencher group at two ends of the probe (P4) is changed, thereby generating a fluorescence signal change that may be detected by the instrument. After the PCR amplification is completed, a melting curve analysis is performed, and the double-stranded product formed with the probe (P4) will melt at a certain melting temperature, at this time, the probe (P4) with the first detectable label and the second detectable label will be changed to a single-stranded state, such that a fluorescence signal change is produced and may be detected by the instrument.

In a cycle of PCR amplification reaction, a double-stranded primer dimer is obtained by reverse complementary amplification of the forward primer (F2-1) and the reverse primer (R2). After amplification, one single-stranded amplification product may be reverse complementary with the probe (P4), such that a distance between the fluoresce group and the quencher group at two ends of the probe (P4) is changed, thereby generating a fluorescence signal change that may be detected by the instrument, and obtaining a false positive signal. After the PCR amplification is completed, a melting curve analysis is performed, and the double-stranded product formed with the probe (P4) will melt at a certain melting temperature, at this time, the probe (P4) with the first detectable label and the second detectable label will be changed to a single-stranded state, such that a fluorescence signal change is produced and may be detected by the instrument, and a false positive signal is obtained.

The system for detection for respiratory syncytial virus (RSV) in a situation where a primer dimer is extremely easily formed, comprises the following components:

**Table 6. Reaction system in Comarative Example**

| Reagents and components | | Concentration |
|---|---|---|
| Reaction system for singleplex detection for respiratory syncytial virus (P5 probe system) | 2× PCR Reaction Buffer | 1× |
| | DNA Polymerase | 2U |
| | Reverse transcriptase | 5U |
| | Forward primer (F5) | 500 nM |
| | Reverse primer (R5) | 100 nM |
| | Probe (P5) | 400 nM |
| | Nucleic acid template | 0.1 ng-60 ng |
| | Ultra-pure water | Adding to 20 µL |
| Reaction system for singleplex detection for respiratory syncytial virus (P5 probe primer dimer system) | 2× PCR Reaction Buffer | 1× |
| | DNA Polymerase | 2U |
| | Reverse transcriptase | 5U |
| | Forward primer (F2-1) | 100 nM |
| | Reverse primer (R5) | 100 nM |
| | Probe (P5) | 400 nM |
| | Nucleic acid template | 0.1 ng-60 ng |
| | Ultra-pure water | Adding to 20 µL |
| Reaction system for singleplex detection for respiratory syncytial virus (P4 probe system) | 2× PCR Reaction Buffer | 1× |
| | DNA Polymerase | 2U |
| | Reverse transcriptase | 5U |
| | Forward primer (F2) | 500 nM |
| | Reverse primer (R2) | 100 nM |
| | Probe (P4) | 400 nM |
| | Nucleic acid template | 0.1 ng-60 ng |
| | Ultra-pure water | Adding to 20 µL |
| Reaction system for singleplex detection for respiratory syncytial virus (P4 probe primer dimer system) | 2× PCR Reaction Buffer | 1× |
| | DNA Polymerase | 2U |
| | Reverse transcriptase | 5U |
| | Forward primer (F2-1) | 100 nM |
| | Reverse primer (R2) | 100 nM |
| | Probe (P4) | 400 nM |
| | Nucleic acid template | 0.1 ng-60 ng |
| | Ultra-pure water | Adding to 20 µL |

The nucleic acid template was devided from an in-vitro transcribed RNA sample. The reaction buffer and a primer probe premixed solution were mixed according to the 4 reaction systems described in Table 6 respectively, a nucleic acid was extracted from a sample to be detected and was added into each of the prepared reaction systems, and then a PCR amplification and a melting curve analysis were performed. A pathogen type in the sample may be judged based on a characteristic Tm value of each target in a specific detection channel.

The detection was performed by using a SLAN real-time fluorescence quantitative PCR instrument and reagent consumables from Shanghai Hongshi Medical Technology Co., Ltd. The 2xPCR reaction buffer comprises 3 mM MgCl₂, 30 mM Tris-HCl with a pH of 8.3, 0.5 mM dNTP, and 70 mM (NH₄)₂SO₄.

In-vitro transcribed RNAs from target sequences of respiratory syncytial virus were used as positive samples, and the reverse primers (R) with different signal detection region sequences were used to separately detect the target sequences of respiratory syncytial virus respectively, to verify a detection ability for single virus infection. The pure water was used as a no template control (NTC).

### Reaction preparation:

After the preparation of the samples was completed, the reaction systems were prepared according to the ratios described in Table 6.

PCR amplification and melting curve analysis:
After a PCR tube was sealed by a cap, the sample was gently and uniformly mixed, and centrifuged for a short time and then left for 5 min at a room temperature. The PCR tube was put in a handheld centrifuge again, centrifuged for a short time, and then transferred to a tray of the fluorescence quantitative PCR instrument. The procedure used was as follows: performing reverse transcription at 55 °C for 15 minutes; performing pre-denaturation at 95°C for 3 minutes; performing denaturation at 94 °C for 10 seconds, and performing annealing and extension at 56 °C for 30 seconds, for a total of 45 cycles, without lighting; performing denaturation at 94 °C for 150 seconds, and perfroming incubation at a constant temperature of 40°C for 300 seconds; and performing melting curve analysis at 40-85°C, with a heating rate of 0.05°C/s and with lighting.

### Data analysis:

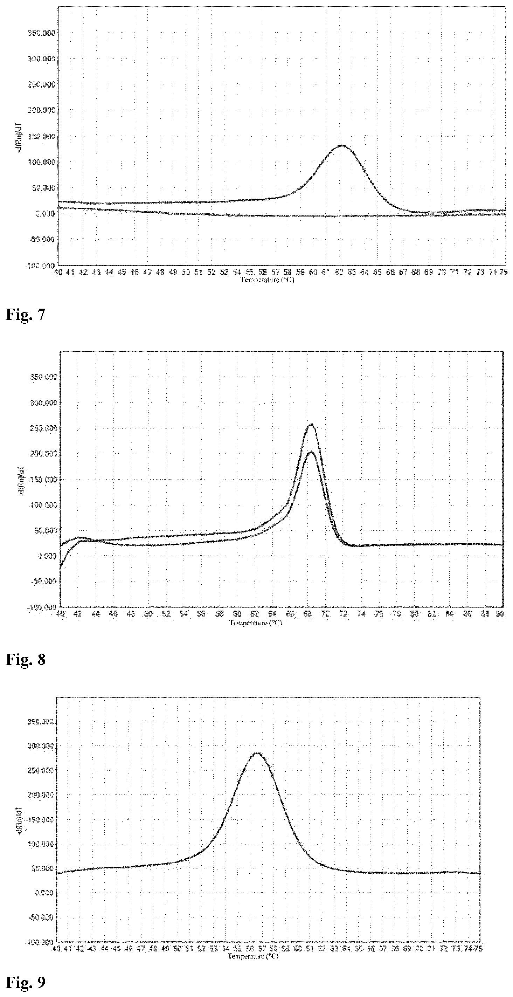

The results were interpreted via Tm values of the melting curves. A data analysis was performed by using an analysis software of the SLAN real-time fluorescence quantitative PCR instrument from Shanghai Hongshi Medical Technology Co., Ltd. Fig. 7 shows melting curves of single PCR respiratory syncytial virus positive samples and a primer dimer that are amplified by using probe P5. Fig. 8 shows melting curves of single PCR respiratory syncytial virus positive samples and a primer dimer that are amplified by using probe P4.

Fig. 7 shows that a melting peak with a Tm value of 62.38°C is formed in a PCR reaction well with addition of a RSV positive template in a CY5 channel when the probe P5 is used, while the primer dimer does not produce a melting peak; and
Fig. 8 shows that a melting peak with a Tm value of 68.48°C and a primer dimer melting peak with a Tm value of 68.52°C are formed in a PCR reaction well with addition of a RSV positive template in an FAM channel when the probe P4 is used.

Thus it can be seen that, when the probe primer composition of the present invention is used, false positives generated by primer dimers may be effectively avoided.

### Example 3: Detection for respiratory syncytial virus (RSV) by using a probe primer system

**Table 7. Probe primer**

| Name of primer probe | Number | Nucleotide sequence (5'-3') | Label |
|---|---|---|---|
| P3 (Probe) | SEQ ID NO:9 | CCCTGGGAGCTCATGGAGGTACT GGTCCTGTCGAC | T16-FAM, T26-BHQ1 3' Spacer C3 |
| R6 (Reverse primer) | SEQ ID NO:16 | TTATTTGGAGGTACTGGTCCTAA ATATTGCAGATACTCCACCTCATC A | / |
| F6 (First forward primer) | SEQ ID NO:17 | GACTGCAGTCAATTTAACAGGCT GCAAGAAGAGGACTAGAAGAT | / |
| F6-1 (Second forward primer) | SEQ ID NO:18 | GCTCCCAGGGAAAGACTGCAGT CAA | / |

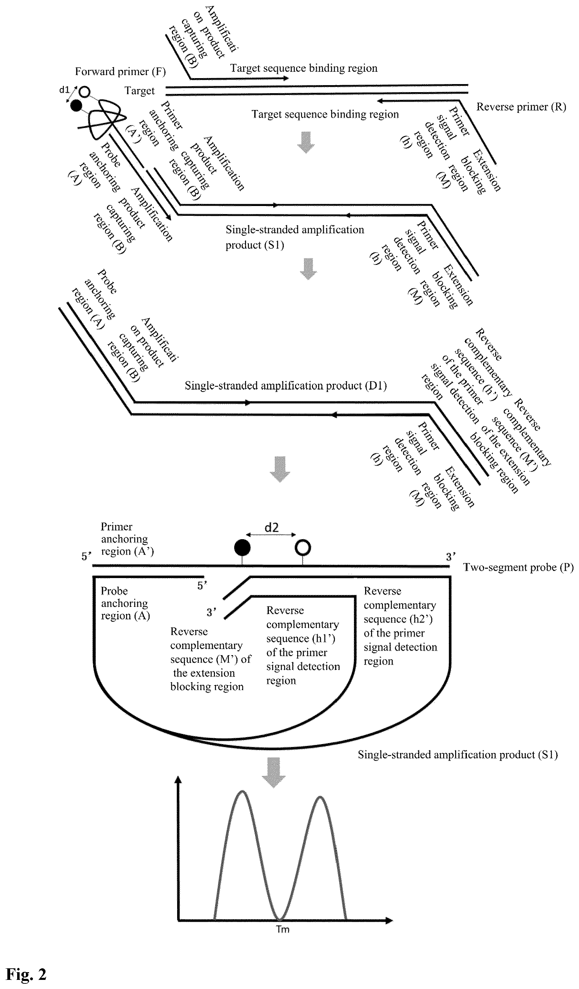

In the above probe primer:
the first forward primer F6 (SEQ ID NO: 17) is a specific primer designed for a target sequence of respiratory syncytial virus, and the F6 has a total length of 44 bp, wherein a 1^{st} to 29^{th} bases at 3' end thereof serve as a target sequence binding region, and a 1^{st} to 12^{th} bases at 5' end thereof serve as an amplification product capturing region (B);
the reverse primer R6 (SEQ ID NO: 16) is a specific primer designed for the target sequence of respiratory syncytial virus, and the R6 has a total length of 48 bp, wherein a 1^{st} to 24^{th} bases at 3' end thereof serve as a target sequence binding region, a 1^{st} to 5^{th} bases at 5' end thereof serve as an extension blocking region (M), and a 6^{th} to 21^{st} bases at 5' end thereof serve as a primer signal detection region (h), which is identical to a sequence of a 7^{th} to 22^{th} bases at 3' end of the probe P3 (SEQ ID NO: 9);
and the second forward primer F6-1 (SEQ ID NO: 18) has a total length of 25 bp, wherein a 1^{st} to 12^{th} bases at 3' end thereof serve as an amplification product capturing region (B), and a 1^{st} to 10^{th} bases at 5' end thereof serve as a probe anchoring region (A), which is reverse complementary with a sequence of a 1^{st} to 10^{th} bases (i.e. a primer anchoring region (A')) at 5' end of the probe P3 (SEQ ID NO: 9). A reaction principle is shown in Fig. 2.

A reaction system for detection for respiratory syncytial virus (RSV) comprises the above probe primer composition decribed in Table 7, a template, a DNA polymerase, a reverse transcriptase, and a reaction buffer, and is used for target specific amplification and fluorescence signal detection. Specially, the reaction system comprises the following components:

**Table 8. Reaction system in this Example**

| Reagents and components | | Concentration |
|---|---|---|
| | 2× PCR Reaction Buffer | 1× |
| System for singleplex detection for respiratory syncytial virus | DNA Polymerase | 2U |
| | Reverse transcriptase | 5U |
| | Probe (P3) | 400 nM |
| | First forward primer (F6) | 100 nM |
| | Reverse primer (R6) | 45 nM |
| | Second forward primer (F6-1) | 200 nM |
| | Nucleic acid template | 0.1 ng-60 ng |
| | Ultra-pure water | Adding to 20 µL |

Among others, according to a specific embodiment of the present invention, the nucleic acid template was devided from an in-vitro transcribed RNA sample. The reaction buffer and a primer probe premixed solution were mixed according to the reaction system described in Table 8, a nucleic acid was extracted from a sample to be detected and was added into the prepared reaction system, and then a PCR amplification and a melting curve analysis were performed. A pathogen type in the sample may be judged based on a characteristic Tm value of the target in a specific detection channel.

The detection was performed by using an SLAN real-time fluorescence quantitative PCR instrument and reagent consumables from Shanghai Hongshi Medical Technology Co., Ltd. The 2xPCR reaction buffer comprises 3 mM MgCl₂, 30 mM Tris-HCl with a pH of 8.3, 0.5 mM dNTP, and 70 mM (NH₄)₂SO₄.

An in-vitro transcribed RNA from the target sequence of respiratory syncytial virus was used as a positive sample, and the reverse primers (R) with different signal detection region sequences were used to separately detect the target sequence of respiratory syncytial virus respectively, to verify a detection ability for single virus infection. The pure water was used as a no template control (NTC).

### Reaction preparation:

After the preparation of the samples was completed, the reaction system was prepared according to the ratios described in Table 8. The reaction principle was shown in Fig. 2

PCR amplification and melting curve analysis:
After a PCR tube was sealed by a cap, the sample was gently and uniformly mixed, and centrifuged for a short time and then left for 5 min at a room temperature. The PCR tube was put in a handheld centrifuge again, centrifuged for a short time, and then transferred to a tray of the fluorescence quantitative PCR instrument. The procedure used was as follows: performing reverse transcription at 55 °C for 15 minutes; performing pre-denaturation at 95°C for 2 minutes; performing denaturation at 94 °C for 10 seconds, and performing annealing and extension at 56 °C for 30 seconds, for a total of 45 cycles, without lighting; performing denaturation at 94 °C for 150 seconds, and perfroming incubation at a constant temperature of 40°C for 300 seconds; and performing melting curve analysis at 40-95°C, with a heating rate of 0.05°C/s and with lighting.

### Data analysis:

The results were interpreted via a Tm value of the melting curve. A data analysis was performed by using an analysis software of the SLAN real-time fluorescence quantitative PCR instrument from Shanghai Hongshi Medical Technology Co., Ltd.

Fig. 9 shows that a melting peak with a Tm value of 56.62°C is formed in a PCR reaction well with addition of a RSV positive template in an EAM channel in Example 3.

### Example 4: Detection for respiratory syncytial virus (RSV) by using a probe primer system

**Table 9. Probe primer composition**

| Name of primer probe | Number | Nucleotide sequence (5'-3') | Label |
|---|---|---|---|
| P3 (Probe) | SEQ ID NO:9 | CCCTGGAGCTCATGGAGGTAC TGGTCCTGTCGAC | T16-FAM, T26-BHQ1 3' Spacer C3 |
| R7 (First reverse primer) | SEQ ID NO:19 | CGCATTACAAGTTAAATATTGCA GATACTCCACCTCATCA | / |
| F6 (First forward primer) | SEQ ID NO:17 | GACTGCAGTCAATTTAACAGGC TGCAAGAAGAGGACTAGAAGAT | / |
| F6-1 (Second forward primer) | SEQ ID NO:18 | GCTCCCAGGGAAAGACTGCAGT CAA | / |
| R7-1 (Second reverse primer) | SEQ ID NO:20 | TTATTTGGAGGTACTGGTCCTAA ACGCATTACAAGTT | / |

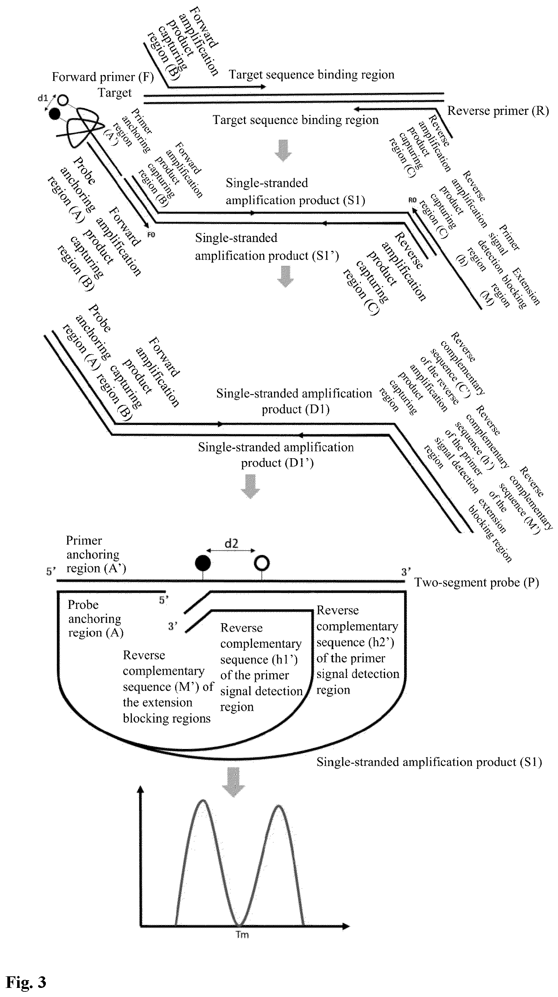

In the above primer probe:
the first forward primer F6 (SEQ ID NO: 17) is a specific primer designed for a target sequence of respiratory syncytial virus, and the F6 has a total length of 44 bp, wherein a 1^{st} to 29^{th} bases at 3' end thereof serve as a target sequence binding region, and a 1^{st} to 12^{th} bases at 5' end thereof serve as an amplification product capturing region (B);
the first reverse primer R7 (SEQ ID NO: 19) is a specific primer designed for the target sequence of respiratory syncytial virus, and the R7 has a total length of 40 bp, wherein a 1^{st} to 24^{th} bases at 3' end thereof serve as a target sequence binding region, and a 1^{st} to 13^{th} bases at 5' end thereof serve as a second amplification product capturing region (C);
the second forward primer F6-1 (SEQ ID NO: 18) has a total length of 25 bp, wherein a 1^{st} to 12^{th} bases at 3' end thereof serve as an amplification product capturing region (B), and a 1^{st} to 10^{th} bases at 5' end thereof serve as a probe anchoring region (A), which is reverse complementary with a sequence of a 1^{st} to 10^{th} bases (i.e. a primer anchoring region (A')) at 5' end of the probe P3 (SEQ ID NO: 9); and
the second reverse primer R7-1 (SEQ ID NO: 20) has a total length of 37 bp, wherein a 1^{st} to 5^{th} bases at 5' end thereof serve as extension blocking region (M), a 6^{th} to 21^{th} bases at 5' end thereof serve as a primer signal detection region (h), and a 1^{st} to 13^{th} bases at 3' end thereof serve as a second amplification product capturing region (C). A reaction principle is shown in Fig. 3.

Specially, a reaction system for detection for respiratory syncytial virus comprises the following components:

**Table 10. Reaction system in this Example**

| Reagents and components | | Concentration |
|---|---|---|
| System for singleplex detection for respiratory syncytial virus | 2× PCR Reaction Buffer | 1× |
| | DNA Polymerase | 2U |
| | Reverse transcriptase | 5U |
| | Probe (P3) | 400 nM |
| | First forward primer (F6) | 100 nM |
| | First reverse primer (R7) | 45 nM |
| | Second forward primer (F6-1) | 250 nM |
| | Second reverse primer (R7-1) | 100 nM |
| | Nucleic acid template | 0.1 ng-60 ng |
| | Ultra-pure water | Adding to 20 µL |

The nucleic acid template was devided from an in-vitro transcribed RNA sample. The reaction buffer and a primer probe premixed solution were mixed according to the reaction system described in Table 10, a nucleic acid was extracted from a sample to be detected and was added into the prepared reaction system, and then a PCR amplification and a melting curve analysis were performed. A pathogen type in the sample may be judged based on a characteristic Tm value of the target in a specific detection channel.

The detection was performed by using an SLAN real-time fluorescence quantitative PCR instrument and reagent consumables from Shanghai Hongshi Medical Technology Co., Ltd. The 2xPCR reaction buffer comprises 3 mM MgCl₂, 30 mM Tris-HCl with a pH of 8.3, 0.5 mM dNTP, and 70 mM (NH₄)₂SO₄.

Sample preparation: an in-vitro transcribed RNA from the target sequence of respiratory syncytial virus was used as a positive sample, and the reverse primers (R) with different signal detection region sequences were used to separately detect the target sequence of respiratory syncytial virus respectively, to verify a detection ability for single virus infection. The pure water was used as a no template control (NTC).

### Reaction preparation:

After the preparation of the samples was completed, the reaction system was prepared according to the ratios described in Table 10. The reaction principle was shown in Fig. 3

PCR amplification and melting curve analysis:
After a PCR tube was sealed by a cap, the sample was gently and uniformly mixed, and centrifuged for a short time and then left for 5 min at a room temperature. The PCR tube was put in a handheld centrifuge again, centrifuged for a short time, and then transferred to a tray of the fluorescence quantitative PCR instrument. The procedure used was as follows: performing reverse transcription at 55 °C for 15 minutes; performing pre-denaturation at 95°C for 2 minutes; performing denaturation at 94 °C for 10 seconds, and performing annealing and extension at 56 °C for 30 seconds, for a total of 45 cycles, without lighting; performing denaturation at 94 °C for 150 seconds, and perfroming incubation at a constant temperature of 40°C for 300 seconds; and performing melting curve analysis at 40-85°C, with a heating rate of 0.05°C/s and with lighting.

### Data analysis:

The results were interpreted via a Tm value of the melting curve. A data analysis was performed by using an analysis software of the SLAN real-time fluorescence quantitative PCR instrument from Shanghai Hongshi Medical Technology Co., Ltd.

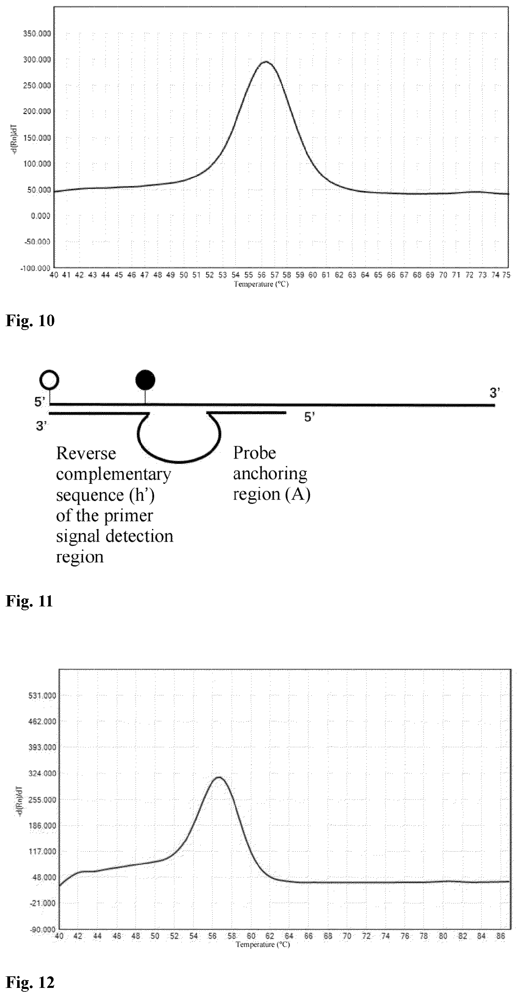

Fig. 10 shows that a melting peak with a Tm value of 56.36°C is formed in a PCR reaction well with addition of a RSV positive template in an FAM channel in Example 4.

### Example 5: Singleplex detection for non-small cell lung cancer EGFR exon 20 H773_V774insH mutation by using a primer probe system

**Table 11. Primer probe**

| Name of primer probe | Number | Nucleotide sequence (5'-3') | Label |
|---|---|---|---|
| P6 | SEQ ID NO:22 | CCACGTCTCAACTATGACACTCCT CCGACCATCCGCTTCCTCCA | C1-FAM, T15-BHQ1 3' Spacer C3 |
| R8 | SEQ ID NO:23 | CCACGTCTCAACTATAAAAGTCTG AACGACCTACACTCC | / |
| F8 | SEQ ID NO:24 | TGGAGGAAGCGGATGTTTCCCAG CAGCTGACACA | / |

In the above probe primer composition:
the forward primer F8 (SEQ ID NO: 24) is a specific primer designed for a target sequence of non-small cell lung cancer EGFR exon 20 H773_V774insH mutation, and the F8 has a total length of 34 bp, wherein a 1^{st} to 16^{th} bases at 3' end thereof serve as a target sequence binding region, and a 1^{st} to 15^{th} bases at 5' end thereof serve as a probe anchoring region (A), which is reverse complementary with a sequence of a 1^{st} to 15^{th} bases (i.e. a primer anchoring region (A')) at 3' end of the probe P6 (SEQ ID NO: 22); and
the reverse primer R8 (SEQ ID NO: 23) is a specific primer designed for the target sequence of non-small cell lung cancer EGFR exon 20 H773_V774insH mutation, and the R8 has a total length of 39 bp, wherein a 1^{st} to 21^{st} bases at 3' end thereof serve as a target sequence binding region, a 1^{st} to 15^{th} bases at 5' end thereof serve as a primer signal detection region (h), which is identical to a sequence of a 1^{st} to 15^{th} bases at 5' end of the probe P6 (SEQ ID NO: 22)

A reaction system comprises the above primer probe, a template, a DNA polymerase, a reverse transcriptase, and a reaction buffer, and is used for target specific amplification and fluorescence signal detection. Specifically, the reaction system is involved in the following steps:
(1) Firstly, in a cycle of PCR amplification reaction, the forward primer (F8) and the reverse primer (R8) may specifically amplify a target nucleic acid sequence. After amplification, a double-stranded amplification product will be obtained, wherein one single-stranded amplification product (S1) comprises the probe anchoring region (A), the target sequence, and a reverse complementary sequence (h') of the primer signal detection region from 5' end to 3' end; and the probe signal detection region (H) of the probe (P6) is complementarily paired with the reverse complementary sequence (h') of the primer signal detection region of the single-stranded amplification product (S1), and the primer anchoring region (A') of the probe (P6) is paired with the probe anchoring region (A) at 5' end of the single-stranded amplification product (S1) in a reverse complementary manner. At this time, a distance between the first detectable label and the second detectable label of the probe (P) is changed, thereby generating a signal that may be detected by the instrument.
(2) After the PCR amplification is completed, a melting curve analysis is performed, and the double-stranded product formed by the probe (P6) and the single-stranded amplification product (S1) will melt at a certain temperature, at this time, the probe (P6) with the first detectable label and the second detectable label will be changed to the single-stranded state, thereby generating a fluorescence signal change that may be detected by the instrument.

The reaction system comprises the following components:

**Table 12. Reaction system in this Example**

| Reagents and components | | Concentration |
|---|---|---|
| System for singleplex detection for non-small cell lung cancer EGFR exon 20 H773_V774i nsH mutation | 2× PCR Reaction Buffer | 1× |
| | DNA Polymerase | 2U |
| | Probe (P6) | 400 nM |
| | Forward primer (F8) | 500 nM |
| | Reverse primer (R8) | 100 nM |
| | Nucleic acid template | 30 copies |
| | Ultra-pure water | Adding to 20 µL |

A non-small cell lung cancer EGFR exon 20 H773_V774insH mutation was used as a positive sample, and the reverse primer (R) with a signal detection region sequence was used to separately detect the target sequence of non-small cell lung cancer EGFR exon 20 H773_V774insH mutation, to verify a detection ability for single mutation. The pure water was used as a no template control (NTC). A reaction principle was shown in Fig. 11.

The reaction buffer and a primer probe premixed solution were mixed according to the reaction system described in Table 12, a nucleic acid was extracted from a sample to be detected by a suitable method and was added into the prepared reaction system, and then a PCR amplification and a melting curve analysis were performed. An EGFR mutation type in the sample may be judged based on a characteristic Tm value of the target in a specific detection channel.

The detection was performed by using an SLAN real-time fluorescence quantitative PCR instrument and reagent consumables from Shanghai Hongshi Medical Technology Co., Ltd. The 2xPCR reaction buffer comprises 3 mM MgCl₂, 30 mM Tris-HCl with a pH of 8.3, 0.5 mM dNTP, 70 mM (NH₄)₂SO₄, etc.

PCR amplification and melting curve analysis:
After a PCR tube was sealed by a cap, the sample was gently and uniformly mixed, and centrifuged for a short time and then left for 5 min at a room temperature. The PCR tube was put in a handheld centrifuge again, centrifuged for a short time, and then transferred to a tray of the fluorescence quantitative PCR instrument. The procedure used was as follows: performing pre-denaturation at 95°C for 3 minutes; performing denaturation at 94°C for 10 seconds, and performing annealing and extension at 56 °C for 30 seconds, for a total of 45 cycles; and performing melting curve analysis at 40-85°C, with a heating rate of 0.05°C/s and with lighting.

### Data analysis:

The detection results were subjected to data analysis by using an analysis software of the SLAN real-time fluorescence quantitative PCR instrument from Shanghai Hongshi Medical Technology Co., Ltd. The results were interpreted via a Tm value of the melting curve. Fig. 12 shows that a melting peak with a Tm value of 56.76°C is formed in a PCR reaction well with addition of a non-small cell lung cancer EGFR exon 20 H773_V774insH mutation positive sample in an FAM channel

### Example 6: Singleplex detection for non-small cell lung cancer EGFR exon 20 H773_V774insH mutation by using a primer probe system

**Table 13. Primer probe**

| Name of primer probe | Number | Nucleotide sequence (5'-3') | Label |
|---|---|---|---|
| P7 | SEQ ID NO:25 | GGCCGTAATCTGCACTGCATCACATCGCC CGCTAGCCCGTCCAATGCC | T20-CY5, T33-BHQ2 3' Spacer C3 |
| R9 | SEQ ID NO:26 | GGCCGTAATCTGCACTAAAAGTCTGAACG ACCTACACTCC | / |
| F9 | SEQ ID NO:27 | TAGCGGGCGATGTGATTTCCCAGCAGCTG ACACA | / |

In the above probe primer composition:
the forward primer F9 (SEQ ID NO: 27) is a specific primer designed for a target sequence of non-small cell lung cancer EGFR exon 20 H773_V774insH mutation, and the F9 has a total length of 34 bp, wherein a 1^{st} to 16^{th} bases at 3' end thereof serve as a target sequence binding region, and a 1^{st} to 15^{th} bases at 5' end thereof serve as a probe anchoring region (A), which is reverse complementary with a sequence of a 20^{th} to 34^{th} bases (i.e. a primer anchoring region (A')) at 5' end of the probe P7 (SEQ ID NO: 25); and
the reverse primer R9 (SEQ ID NO: 26) is a specific primer designed for the target sequence of non-small cell lung cancer EGFR exon 20 H773_V774insH mutation, and the R9 has a total length of 40 bp, wherein a 1^{st} to 21^{st} bases at 3' end thereof serve as a target sequence binding region, a 1^{st} to 16^{th} bases at 5' end thereof serve as a primer signal detection region (h), which is identical to a sequence of a 1^{st} to 16^{th} bases at 3' end of the probe P7 (SEQ ID NO: 25)

A reaction system comprises the above primer probe, a template, a DNA polymerase, a reverse transcriptase, and a reaction buffer, and is used for target specific amplification and fluorescence signal detection. Specifically, the reaction system is involved in the following steps:
(1) Firstly, in a cycle of PCR amplification reaction, the forward primer (F) and the reverse primer (R) may specifically amplify a target nucleic acid sequence. After amplification, a double-stranded amplification product will be obtained, wherein one single-stranded amplification product (S1) comprises the probe anchoring region (A), the target sequence, and a reverse complementary sequence (h') of the primer signal detection region from 5' end to 3' end; and the probe signal detection region (H) of the probe (P) is complementarily paired with the reverse complementary sequence (h') of the primer signal detection region of the single-stranded amplification product (S1), and the primer anchoring region (A') of the probe (P) is paired with the probe anchoring region (A) at 5' end of the single-stranded amplification product (S1) in a reverse complementary manner. At this time, a distance between the first detectable label and the second detectable label of the probe (P) is changed, thereby generating a signal that may be detected by the instrument.
(2) After the PCR amplification is completed, a melting curve analysis is performed, and the double-stranded product formed by the probe (P) and the single-stranded amplification product (S1) will melt at a certain temperature, at this time, the probe (P) with the first detectable label and the second detectable label will be changed to the single-stranded state, thereby generating a fluorescence signal change that may be detected by the instrument.

The reaction system comprises the following components:

**Table 14 Reaction system in this Example**

| Reagents and components | | Concentration |
|---|---|---|
| System for singleplex detection for non-small cell lung cancer EGFR exon 20 H773_V77 4insH mutation | 2× PCR Reaction Buffer | 1× |
| | DNA Polymerase | 2U |
| | Probe (P7) | 400 nM |
| | Forward primer (F9) | 500 nM |
| | Reverse primer (R9) | 100 nM |
| | Nucleic acid template | 30 copies |
| | Ultra-pure water | Adding to 20 µL |

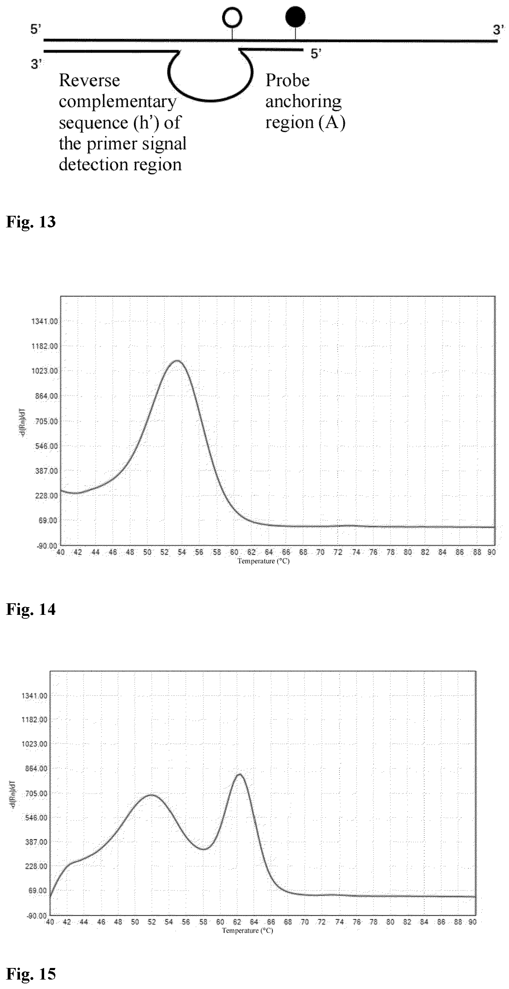

A non-small cell lung cancer EGFR exon 20 H773_V774insH mutation was used as a positive sample, and the reverse primer (R) with a signal detection region sequence was used to separately detect the target sequence of non-small cell lung cancer EGFR exon 20 H773_V774insH mutation, to verify a detection ability for single mutation. The pure water was used as a no template control (NTC). A reaction principle was shown in Fig. 13.

The reaction buffer and a primer probe premixed solution were mixed according to the reaction system described in Table 14, a nucleic acid was extracted from a sample to be detected by a suitable method and was added into the prepared reaction system, and then a PCR amplification and a melting curve analysis were performed. An EGFR mutation type in the sample may be judged based on a characteristic Tm value of the target in a specific detection channel.

The detection was performed by using an SLAN real-time fluorescence quantitative PCR instrument and reagent consumables from Shanghai Hongshi Medical Technology Co., Ltd. The 2X PCR reaction buffer comprises 3 mM MgCl₂, 30 mM Tris-HCl with a pH of 8.3, 0.5 mM dNTP, 70 mM (NH₄)₂SO₄, etc.

PCR amplification and melting curve analysis:
After a PCR tube was sealed by a cap, the sample was gently and uniformly mixed, and centrifuged for a short time and then left for 5 min at a room temperature. The PCR tube was put in a handheld centrifuge again, centrifuged for a short time, and then transferred to a tray of the fluorescence quantitative PCR instrument. The procedure used was as follows: performing pre-denaturation at 95°C for 3 minutes; performing denaturation at 94°C for 10 seconds, and performing annealing and extension at 56 °C for 30 seconds, for a total of 45 cycles; and performing melting curve analysis at 40-85°C, with a heating rate of 0.05°C/s and with lighting.

### Data analysis:

The detection results were subjected to data analysis by using an analysis software of the SLAN real-time fluorescence quantitative PCR instrument from Shanghai Hongshi Medical Technology Co., Ltd. The results were interpreted via a Tm value of the melting curve. Fig. 14 shows that a melting peak with a Tm value of 53.67°C is formed by reverse complementation of the probe anchoring region (A) of the forward primer (F9) and the primer anchoring region (A') of the probe (P7) in a PCR reaction well as negative control well (NTC) in a CY5 channel. Fig. 15 shows that a melting peak with a Tm value of 52.43°C and a positive melting peak with a Tm value of 62.41°C are formed by reverse complementation of the probe anchoring region (A) of the forward primer (F9) and the primer anchoring region (A') of the probe (P7) in a PCR reaction well with addition of non small cell lung cancer EGFR exon 20 H773_V774insH mutation positive sample in a CY5 channel.

### Example 7: Multiplex PCR detection for non-small cell lung cancer EGFR exon 20 T790M mutation, exon 21 L858R mutation, and exon 21 L861Q mutation

**Table 15. Primer probe**

| Name of primer probe | Number | Nucleotide sequence (5'-3') | Label |
|---|---|---|---|
| P8 | SEQ ID NO:28 | AGACGCACGGTGACATGTGTTTG ACTCAGTTCTGCTGCTGCCCACC GTCTGGAGTGCGT | T16-FAM, T31-BHQ2 3'Spacer C3 |
| F10 | SEQ ID NO:29 | CACCGTGCGTCTAAATGATGGAC AGCGTGGACAA | / |
| R10 | SEQ ID NO:30 | TTTTTCATGTGTTTGACTCAGTTT TTCGAAGAGCATGAGCTGCA | / |
| F11 | SEQ ID NO:31 | CACCGTGCGTCTAAAGTCAAGAT CACAGATTTTGGGCG | / |
| R11 | SEQ ID NO:32 | CTGCTGCTGCTTTCTGGCTGACCT AAAGCCACC | / |
| F12 | SEQ ID NO:33 | CACCGTGCGTCTAAAAGATTTTG GGCTGGCCAAACA | / |
| R12 | SEQ ID NO:34 | CTGGAGTGCGTTTTGCCTCCTTCT GCATGGTATTCT | / |

In the above probe primer composition:
Both of the forward primer F10 (SEQ ID NO: 29) and the reverse primer R10 (SEQ ID NO: 30) are specific primers designed for a target sequence of non-small cell lung cancer EGFR exon 20 T790M mutation. The F10 has a total length of 34 bp, wherein a 1^{st} to 19^{th} bases at 3' end thereof serve as a target sequence binding region 1, and a 1^{st} to 12^{th} bases at 5' end thereof serve as a probe anchoring region (A1), which is reverse complementary with a sequence of a 1^{st} to 12^{th} bases (i.e. a primer anchoring region (A1')) of the probe P8 (SEQ ID NO: 28). The R10 has a total length of 44 bp, wherein a 1^{st} to 18^{th} bases at 3' end thereof serve as a target sequence binding region 2, a 1^{st} to 5^{th} bases at 5' end thereof serve as an extension blocking region, and a 6^{th} to 23^{rd} bases at 5' end thereof serve as a primer signal detection region (h1), which is identical to a sequence of a 14^{th} to 31^{st} bases at 5' end of the probe P8 (SEQ ID NO: 28).

Both of the forward primer F11 (SEQ ID NO: 31) and the reverse primer R11 (SEQ ID NO: 32) are specific primers designed for a target sequence of non-small cell lung cancer EGFR exon 21 L858R mutation. The F11 has a total length of 38 bp, wherein a 1^{st} to 23^{rd} bases at 3' end thereof serve as a target sequence binding region 3, and a 1^{st} to 12^{th} bases at 5' end thereof serve as a probe anchoring region (A1), which is reverse complementary with a sequence of a 1^{st} to 12^{th} bases (i.e. a primer anchoring region (A1')) at 5' end of the probe P8 (SEQ ID NO: 28). The R11 has a total length of 33 bp, wherein a 1^{st} to 20^{th} bases at 3' end thereof serve as a target sequence binding region 4, a 1^{st} to 10^{th} bases at 5' end thereof serve as a primer signal detection region (h2), which is identical to a sequence of a 19^{th} to 28^{th} bases at 3' end of the probe P8 (SEQ ID NO: 28).

Both of the forward primer F12 (SEQ ID NO: 33) and the reverse primer R12 (SEQ ID NO: 34) are specific primers designed for a target sequence of non-small cell lung cancer EGFR exon 21 L861Q mutation. The F12 has a total length of 36 bp, wherein a 1^{st} to 21^{st} bases at 3' end thereof serve as a target sequence binding region 5, and a 1^{st} to 12^{th} bases at 5' end thereof serve as a probe anchoring region (A1), which is reverse complementary with a sequence of a 1^{st} to 12^{th} bases (i.e. a primer anchoring region (A1')) at 5' end of the probe P8 (SEQ ID NO: 28). The R12 has a total length of 36 bp, wherein a 1^{st} to 22^{nd} bases at 3' end thereof serve as a target sequence binding region 6, a 1^{st} to 10^{th} bases at 5' end thereof serve as a primer signal detection region (h3), which is identical to a sequence of a 1^{st} to 10^{th} bases at 3' end of the probe P8 (SEQ ID NO: 28).

The characteristic Tm values of the above probes are shown in Table 16:

**Table 16:**

| Fluorescent channel | Melting temperature (Tm, °C) | | |
|---|---|---|---|
| | 56≤Tm<62 | 64≤Tm<72 | 72≤Tm<80 |
| FAM | T790M mutation | L858R mutation | L861Q mutation |

A multiplex PCR detection for non small cell lung cancer EGFR exon 20 T790M mutation, exon 21 L858R mutation, and exon 21 L861Q mutation was performed by using the above primer probe.

A reaction system used for detection was shown in Table 17.

**Table 17: Reaction system for multiplex PCR detection for non small cell lung cancer EGFR exon 20 T790M mutation, exon 21 L858R mutation, and exon 21 L861Q mutation**

| | Reagents and components | Concentration |
|---|---|---|
| | 2× PCR Reaction Buffer | 1× |
| | DNA Polymerase | 2U |
| | Probe (P8) | 400 nM |
| | Forward primer (F10) | 500 nM |
| | Reverse primer (R10) | 100 nM |
| | Forward primer (F11) | 500 nM |
| | Reverse primer (R11) | 100 nM |
| | Forward primer (F12) | 500 nM |
| Reverse primer (R12) | | 100 nM |
| Non small cell lung cancer EGFR exon 20 T790M mutation nucleic acid sample or exon 21 L858R mutation nucleic acid sample or exon 21 L861Q mutation nucleic acid sample | | 400 copies |
| Ultra-pure water | | Adding to 20 µL |

| | | |
|---|---|---|
| Note: The 2XPCR reaction buffer comprises 3 mM MgCl₂, 30 mM Tris-HCl buffer with a pH of 8.3, 0.5 mM dNTP, and 70 mM (NH₄)₂SO₄. | | |

Nucleic acid template: a non small cell lung cancer EGFR exon 20 T790M mutation nucleic acid sample or exon 21 L858R mutation nucleic acid sample or exon 21 L861Q mutation nucleic acid sample was used as the nucleic acid template respectively, to verify a detection ability for single gene mutation in a multiplex system. The pure water was used as a no template control (NTC).

### Reaction preparation:

After the preparation of the nucleic acid templates was completed, the reaction system was prepared according to the ratios described in Table 17.

PCR amplification and melting curve analysis:

After a PCR tube was sealed by a cap, the sample was gently and uniformly mixed, and centrifuged for a short time and then left for 5 min at a room temperature. The PCR tube was put in a handheld centrifuge again, centrifuged for a short time, and then transferred to a tray of SLAN real-time fluorescence quantitative PCR instrument from Shanghai Hongshi Medical Technology Co., Ltd. The procedure used was as follows: performing pre-denaturation at 95°C for 3 minutes; performing denaturation at 94 °C for 10 seconds, and performing annealing and extension at 56 °C for 30 seconds, for a total of 45 cycles, without lighting; performing denaturation at 94 °C for 150 seconds, and perfroming incubation at a constant temperature of 40°C for 300 seconds; and performing melting curve analysis at 62-86°C, with a heating rate of 0.05°C/s and with lighting, to obtain melting curves.

### Data analysis:

The results were interpreted via Tm values of the melting curves by using an analysis software of the SLAN real-time fluorescence quantitative PCR instrument from Shanghai Hongshi Medical Technology Co., Ltd.

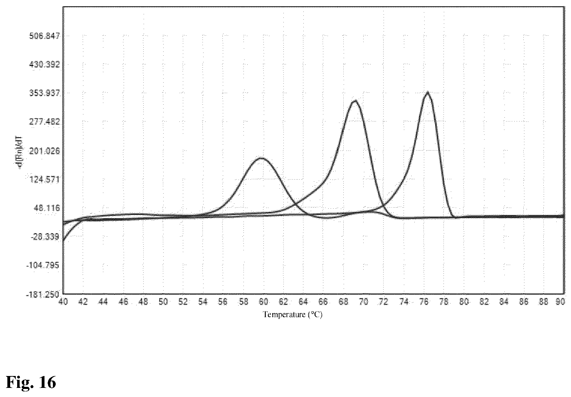

Fig. 16 shows that melting peaks of non-small cell lung cancer EGFR exon 20 T790M mutation (59.81°C), non-small cell lung cancer EGFR exon 21 L858R mutation (69.13°C), and non-small cell lung cancer EGFR exon 21 L861Q mutation (76.31°C) appear in PCR reaction wells with addition of non small cell lung cancer EGFR exon 20 T790M mutation nucleic acid sample, exon 21 L858R mutation nucleic acid sample and exon 21 L861Q mutation nucleic acid sample in an FAM channel, respectively.

## Claims

1. A probe primer composition for PCR detection, comprising a probe and a first primer set, wherein the first primer set comprises a first primer and a second primer that are a forward primer and a reverse primer or a reverse primer and a forward primer, respectively,
the probe comprises a first binding region A' and a second binding region H,
the first primer comprises a sequence region A that is partially or completely complementary with the first binding region A' of the probe, and the second primer comprises a sequence region h that is partially or completely identical to the second binding region H of the probe,
none of the first binding region A', the second binding region H, the sequence region A and the sequence region h are complementary or identical to a target sequence or any fragment thereof, and
the probe has a detectable label thereon.

2. The probe primer composition of claim 1, wherein the first primer further comprises a first target sequence binding region, and the second primer further comprises a second target sequence binding region;
preferably, wherein the first primer further comprises an amplification product capturing region B, and the probe primer composition further comprises a second primer set, the second primer set comprises a third primer and the second primer that are a forward primer and a reverse primer or a reverse primer and a forward primer, respectively, the third primer comprises a third target sequence binding region and an amplification product capturing region B, and the second primer further comprises a second target sequence binding region;
preferably, wherein the second primer further comprises an amplification product capturing region C, and the probe primer composition further comprises a second primer set, the second primer set comprises the first primer and a fourth primer that are a forward primer and a reverse primer or a reverse primer and a forward primer, respectively, wherein the fourth primer comprises a fourth target sequence binding region and an amplification product capturing region C, and the first primer further comprises a first target sequence binding region.

3. The probe primer composition of claim 1, wherein the first primer further comprises an amplification product capturing region B, the second primer further comprises an amplification product capturing region C, and the probe primer composition further comprises a second primer set, the second primer set comprises a third primer and a fourth primer that are a forward primer and a reverse primer or a reverse primer and a forward primer, respectively, the third primer set comprises a third target sequence binding region and an amplification product capturing region B, and the fourth primer comprises a fourth target sequence binding region and an amplification product capturing region C;
preferably, wherein a single-stranded amplification product of the first primer and the second primer comprises the sequence region A and a sequence h' that is partially or completely complementary with the second binding region H of the probe;
preferably, the first binding region A' is located at a 5' end or 3' end of the probe, and preferably, a blocking region is provided at the 3' end of the probe.

4. The probe primer composition of claim 1, wherein the detectable label comprises a first detectable label and a second detectable label, the first detectable label is a fluorescence reporter, and the second detectable label is a quencher or other labeling groups that can produce a signal change with the first detectable label through fluorescence resonance energy transfer,
preferably, both of the first detectable label and the second detectable label are located on the first binding region A' or both of the first detectable label and the second detectable label are located on the second binding region H; or
preferably, an spacer region between the first detectable label and the second detectable label is at least partially located on the first binding region A', or the spacer region between the first detectable label and the second detectable label is at least partially located on the second binding region H.

5. The probe primer composition of claim 2, wherein the first primer sequentially comprises the sequence region A and the first target sequence binding region from 5' end to 3' end;
the second primer sequentially comprises the sequence region h and the second target sequence binding region from 5' end to 3' end,
preferably, the second primer further comprises an extension blocking region, which is a sequence that is not identical to or complementary with a base sequence of any part of the probe, and
more preferably, the second primer sequentially comprises the extension blocking region, the sequence region h, and the second target sequence binding region from 5' end to 3' end; preferably, wherein the probe primer composition for n target sequences comprises n first primer sets and m probes, wherein 1 ≤ n ≤ 20, 1 ≤ m ≤ n;
preferably, the probe primer composition for n target sequences comprises n first primer sets and 1 or several probes, wherein 1 ≤ n ≤ 20;
preferably, single-stranded amplification products obtained by amplifying different target sequences with all of the first primer sets are different from each other in an annealing temperature with the probe,
preferably, single-stranded amplification products obtained by amplifying different target sequences with all of the first primer sets are different from each other by a ΔTm of 6-10°C in an annealing temperature with the probe, and
preferably, the target sequence binding regions included in all of the first primers are different from each other, and the target sequence binding regions included in all of the second primers are different from each other.

6. The probe primer composition of claim 2, wherein the third primer sequentially comprises the amplification product capturing region B and the third target sequence binding region from 5' end to 3' end; the first primer sequentially comprises the sequence region A and the amplification product capturing region B from 5' end to 3' end; and the second primer sequentially comprises the sequence region h and the second target sequence binding region from 5' end to 3' end;
the amplification product capturing region B doses not bind with the target sequence or any fragment thereof in a paired manner;
preferably, the second primer further comprises an extension blocking region, which is a sequence that is not identical to or complementary with a base sequence of any part of the probe; and
more preferably, the second primer sequentially comprises the extension blocking region, the sequence region h, and the second target sequence binding region from 5' end to 3' end.

7. The probe primer composition of claim 2, wherein the probe primer composition for n target sequences comprises n second primer sets, m probes, and o first primers, wherein 1 ≤ n ≤ 20, 1 ≤ m ≤ n, and 1 ≤ o ≤ n,
preferably, the probe primer composition for n target sequences comprises n second primer sets, 1 or several probes, and 1 or several first primers, wherein 1 ≤ n ≤ 20,
preferably, single-stranded amplification products obtained by amplifying with all of the first primer sets are different from each other in an annealing temperature with the probe,
preferably, single-stranded amplification products obtained by amplifying with all of the first primer sets are different from each other by a ΔTm of 6-10°C in an annealing temperature with the probe, and
preferably, the target sequence binding regions included in all of the third primers are different from each other, and the target sequence binding regions included in all of the second primers are different from each other.

8. The probe primer composition of claim 2, wherein the first primer sequentially comprises the sequence region A and the first target sequence binding region from 5' end to 3' end; the second primer sequentially comprises the sequence region h and the amplification product capturing region C from 5' end to 3' end; and the fourth primer sequentially comprises the amplification product capturing region C and the fourth target sequence binding region from 5' end to 3' end; the amplification product capturing region C doses not bind with the target sequence or any fragment thereof in a paired manner;
preferably, the second primer further comprises an extension blocking region, which is a sequence that is not identical to or complementary with a base sequence of any part of the probe; and
more preferably, the second primer sequentially comprises the extension blocking region, the sequence region h, and the amplification product capturing region C from 5' end to 3' end; preferably, wherein the probe primer composition for n target sequences comprises n second primer sets, m probes, and o second primers, wherein 1 ≤ n ≤ 20, 1 ≤ m ≤ n, and 1 ≤ o ≤ n, preferably, the probe primer composition for n target sequences comprises n second primer sets, 1 or several probes, and 1 or several second primers, wherein 1 ≤ n ≤ 20,
preferably, single-stranded amplification products obtained by amplifying with all of the first primer sets are different from each other in an annealing temperature with the probe,
preferably, single-stranded amplification products obtained by amplifying with all of the first primer sets are different from each other by a ΔTm of 6-10°C in an annealing temperature with the probe, and
preferably, the target sequence binding regions included in all of the first primers are different from each other, and the target sequence binding regions included in all of the fourth primers are different from each other.

9. The probe primer composition of claim 3, wherein the first primer sequentially comprises the sequence region A and the amplification product capturing region B from 5' end to 3' end; the second primer sequentially comprises the sequence region h and the amplification product capturing region C from 5' end to 3' end; the third primer sequentially comprises the amplification product capturing region B and the third target sequence binding region from 5' end to 3' end; and the fourth primer sequentially comprises the amplification product capturing region C and the fourth target sequence binding region from 5' end to 3' end;
neither the amplification product capturing region B nor the amplification product capturing region C is paired with the target sequence or any fragment thereof;
preferably, the second primer further comprises an extension blocking region, which is a base sequence that is not identical to or complementary with the probe or any fragmet thereof; and more preferably, the second primer sequentially comprises the extension blocking region, the sequence region h, and the amplification product capturing region C from 5' end to 3' end;
preferably, wherein the probe primer composition for n target sequences comprises n second primer sets, m probes, and p first primer sets, wherein 1 ≤ n ≤ 20, 1 ≤ m ≤ n, and 1 ≤ P ≤ n, preferably, the probe primer composition for n target sequences comprises n second primer sets, 1 or several probes, and 1 or several first primer sets, wherein 1 ≤ n ≤ 20,
preferably, single-stranded amplification products obtained by amplifying with all of the first primer sets are different from each other in an annealing temperature with the probe,
preferably, single-stranded amplification products obtained by amplifying with all of the first primer sets are different from each other by a ΔTm of 6-10°C in an annealing temperature with the probe, and
preferably, the target sequence binding regions included in all of the third primers are different from each other, and the target sequence binding regions included in all of the fourth primers are different from each other.

10. A kit for PCR detection, comprising the probe primer composition of any one of claims 1-9.

11. A method for PCR detection, comprising steps of performing PCR by using the probe primer set of any one of claims 1-9;
preferably, comprising the following steps:
using the first primer set to specifically bind with a target sequence and extend to produce a double-stranded pre-amplification product, wherein one single-stranded amplification product of the double-stranded pre-amplification product comprises the sequence region A, the target sequence, and a complementary sequence h' of the sequence region h; and
forming a signal change, by specifical binding of the sequence region A and the complementary sequence h' of the single-stranded amplification product with the probe, that can be detected by an instrument, and determining whether the target sequence is present based on the signal change;
preferably, the single-stranded amplification product further comprises a complementary sequence of the extension blocking region at 3' end thereof, which is not complementary with any part of the probe.

12. The method of claim 11, the method comprises the following steps:
using the third primer and the second primer of the second primer set to specifically bind with a target sequence and extend to produce a first double-stranded pre-amplification product, wherein one single-stranded amplification product of the first double-stranded pre-amplification product comprises a complementary sequence B' of the amplification product capturing region B, the target sequence, and the sequence region h;
using the first primer set and taking the first double-stranded pre-amplification product as a template, to extend to obtain a second double-stranded amplification product, wherein one single-stranded amplification product of the second double-stranded amplification product comprises the sequence region A, the amplification product capturing region B, the target sequence, and a complementary sequence h' of the sequence region h;
forming a signal change, by specifical binding of the sequence region A and the complementary sequence h' of the single-stranded amplification product with the probe, that can be detected by an instrument, and determining whether the target sequence is present based on the signal change;
preferably, the one single-stranded amplification product of the first double-stranded pre-amplification product further comprises an extension blocking region, and/or the one single-stranded amplification product of the second double-stranded amplification product further comprises a complementary sequence of the extension blocking region, and the complementary sequence of the extension blocking region is not complementary with any part of the probe.

13. The method of claim 11, comprising the following steps:
using the first primer and the fourth primer of the second primer set to specifically bind with a target sequence and extend to produce a first double-stranded pre-amplification product, wherein one single-stranded amplification product of the first double-stranded pre-amplification product comprises a complementary sequence A' of the sequence region A, the target sequence, and the amplification product capture region C;
using the first primer set and taking the first double-stranded pre-amplification product as a template to extend to obtain a second double-stranded amplification product, wherein one single-stranded amplification product of the second double-stranded amplification product comprises the sequence region A, the target sequence, a complementary sequence C' of the amplification product capturing region C, and a complementary sequence h' of the sequence region h, and
forming a signal change, by specifical binding of the sequence region A and the complementary sequence h' of the single-stranded amplification product with the probe, that can be detected by an instrument, and determining whether the target sequence is present based on the signal change;
preferably, the one single-stranded amplification product of the second double-stranded amplification product further comprises a complementary sequence of the extension blocking region, and the complementary sequence of the extension blocking region is not complementary with any part of the probe.

14. The method of claim 11, comprising the following steps:
using the third primer and the fourth primer of the second primer set to specifically bind with a target sequence and extend to produce a first double-stranded pre-amplification product, wherein one single-stranded amplification product of the first double-stranded pre-amplification product comprises a complementary sequence B' of the amplification product capturing region B, the target sequence, and the amplification product capture region C;
using the first primer set and taking the first double-stranded pre-amplification product as a template to extend to obtain a second double-stranded amplification product, wherein one single-stranded amplification product of the second double-stranded amplification product comprises the sequence region A, the amplification product capturing region B, the target sequence, a complementary sequence C' of the amplification product capturing region C, and a complementary sequence h' of the sequence region h, and
forming a signal change, by specifical binding of the sequence region A and the complementary sequence h' of the single-stranded amplification product with the probe, that can be detected by an instrument, and determining whether the target sequence is present based on the signal change; wherein,
preferably, the one single-stranded amplification product of the second double-stranded amplification product further comprises a complementary sequence of the extension blocking region, and the complementary sequence of the extension blocking region is not complementary with any part of the probe;
preferably, wherein the signal change refers to a fluorescence signal change or a change in melting temperature curve; and preferably, the signal change refers to the change in melting temperature curve.

15. Use of the probe primer composition of any one of claims 1-9 in PCR detection;
preferably, in a single-tube super-multiplex PCR detection.
preferably, in pathogenic microorganism detection, non-invasive birth defect screening, single nucleotide polymorphism analysis, methylation analysis, gene mutation analysis, or gene typing.

## Patentansprüche

1. Zusammensetzung von Primern und Sonden zum PCR-Nachweis, umfassend eine Sonde und ein erstes Primerset, wobei das erste Primerset einen ersten Primer und einen zweiten Primer umfasst, die jeweils ein Vorwärtsprimer und ein Rückwärtsprimer oder ein Rückwärtsprimer und ein Vorwärtsprimer sind,
die Sonde einen ersten Bindungsbereich A' und einen zweiten Bindungsbereich H umfasst,
der erste Primer einen Sequenzbereich A umfasst, der teilweise oder vollständig komplementär zu dem ersten Bindungsbereich A' der Sonde ist, und der zweite Primer einen Sequenzbereich h umfasst, der teilweise oder vollständig identisch mit dem zweiten Bindungsbereich H der Sonde ist,
keiner des ersten Bindungsbereichs A', des zweiten Bindungsbereichs H, des Sequenzbereichs A und des Sequenzbereichs h, komplementär oder identisch mit einer Zielsequenz oder einem Fragment davon ist, und
die Sonde eine nachweisbare Markierung aufweist.

2. Zusammensetzung von Primern und Sonden nach Anspruch 1, wobei der erste Primer ferner einen ersten Zielsequenz-Bindungsbereich umfasst und der zweite Primer ferner einen zweiten Zielsequenz-Bindungsbereich umfasst;
vorzugsweise, wobei der erste Primer ferner einen Amplifikationsprodukt-Erfassungsbereich B umfasst und die Zusammensetzung von Primern und Sonden ferner einen zweiten Primersatz umfasst, der zweite Primersatz einen dritten Primer und den zweiten Primer umfasst, die ein Vorwärtsprimer und ein Rückwärtsprimer bzw. ein Rückwärtsprimer und ein Vorwärtsprimer sind, der dritte Primer einen dritten Zielsequenz-Bindungsbereich und einen Amplifikationsprodukt-Erfassungsbereich B umfasst und der zweite Primer ferner einen zweiten Zielsequenz-Bindungsbereich umfasst;
vorzugsweise, wobei der zweite Primer ferner einen Amplifikationsprodukt-Erfassungsbereich C umfasst und die Zusammensetzung von Primern und Sonden ferner einen zweiten Primersatz umfasst, der zweite Primersatz den ersten Primer und einen vierten Primer umfasst, die ein Vorwärtsprimer und ein Rückwärtsprimer bzw. ein Rückwärtsprimer und ein Vorwärtsprimer sind, wobei der vierte Primer einen vierten Zielsequenz-Bindungsbereich und einen Amplifikationsprodukt-Erfassungsbereich C umfasst und der erste Primer ferner einen ersten Zielsequenz-Bindungsbereich umfasst.

3. Zusammensetzung von Primern und Sonden nach Anspruch 1, wobei der erste Primer ferner einen Amplifikationsprodukt-Erfassungsbereich B umfasst, der zweite Primer ferner einen Amplifikationsprodukt-Erfassungsbereich C umfasst und die Zusammensetzung von Primern und Sonden ferner einen zweiten Primersatz umfasst, der zweite Primersatz einen dritten Primer und einen vierten Primer umfasst, die ein Vorwärtsprimer und ein Rückwärtsprimer bzw. ein Rückwärtsprimer und ein Vorwärtsprimer sind, der dritte Primer einen dritten Zielsequenz-Bindungsbereich und einen Amplifikationsprodukt-Erfassungsbereich B umfasst und der vierte Primer einen vierten Zielsequenz-Bindungsbereich und einen Amplifikationsprodukt-Erfassungsbereich C umfasst;
vorzugsweise, wobei ein einzelsträngiges Amplifikationsprodukt des ersten Primers und des zweiten Primers den Sequenzbereich A und eine Sequenz h' umfasst, die teilweise oder vollständig komplementär zum zweiten Bindungsbereich H der Sonde ist;
vorzugsweise befindet sich der erste Bindungsbereich A' an einem 5'-Ende oder 3'-Ende der Sonde, und vorzugsweise ist ein Blockierungsbereich am 3'-Ende der Sonde bereitgestellt.

4. Zusammensetzung von Primern und Sonden nach Anspruch 1, wobei die nachweisbare Markierung eine erste nachweisbare Markierung und eine zweite nachweisbare Markierung umfasst, die erste nachweisbare Markierung ein Fluoreszenzreporter ist und die zweite nachweisbare Markierung ein Quencher ist oder zu anderen Markierungsgruppen gehört, die eine Signaländerung mit der ersten nachweisbaren Markierung durch Fluoreszenzresonanzenergietransfer erzeugen können,
vorzugsweise befinden sich sowohl die erste nachweisbare Markierung als auch die zweite nachweisbare Markierung an dem ersten Bindungsbereich A' oder befinden sich sowohl die erste nachweisbare Markierung als auch die zweite nachweisbare Markierung an dem zweiten Bindungsbereich H; oder
vorzugsweise befindet sich ein Abstandsbereich zwischen der ersten nachweisbaren Markierung und der zweiten nachweisbaren Markierung mindestens teilweise im ersten Bindungsbereich A', oder der Abstandsbereich zwischen der ersten nachweisbaren Markierung und der zweiten nachweisbaren Markierung befindet sich mindestens teilweise im zweiten Bindungsbereich H.

5. Zusammensetzung von Primern und Sonden nach Anspruch 2, wobei der erste Primer sequentiell den Sequenzbereich A und den ersten Zielsequenz-Bindungsbereich vom 5'-Ende bis zum 3'-Ende umfasst;
der zweite Primer sequentiell den Sequenzbereich h und den zweiten Zielsequenz-Bindungsbereich vom 5'-Ende bis zum 3'-Ende umfasst,
vorzugsweise der zweite Primer ferner eine verlängerungsblockierende Region umfasst, die eine Sequenz ist, die nicht identisch oder komplementär zu einer Basensequenz eines Teils der Sonde ist, und
mehr bevorzugt der zweite Primer sequentiell die verlängerungsblockierende Region, den Sequenzbereich h und den zweiten Zielsequenz-Bindungsbereich vom 5'-Ende bis zum 3'-Ende umfasst; vorzugsweise wobei die Zusammensetzung von Primern und Sonden für n Zielsequenzen n erste Primersätze und m Sonden umfasst, wobei 1 ≤ n ≤ 20, 1 ≤ m ≤ n ist;
vorzugsweise die Zusammensetzung von Primern und Sonden für n Zielsequenzen n erste Primersätze und 1 oder mehrere Sonden umfasst, wobei 1 ≤ n ≤ 20 ist;
vorzugsweise einzelsträngige Amplifikationsprodukte, die durch Amplifikation verschiedener Zielsequenzen mit allen ersten Primersätzen erhalten werden, sich hinsichtlich ihrer Anlagerungstemperatur an die Sonde voneinander unterscheiden, vorzugsweise einzelsträngige Amplifikationsprodukte, die durch Amplifikation verschiedener Zielsequenzen mit allen ersten Primersätzen erhalten werden, sich hinsichtlich ihrer Anlagerungstemperatur an die Sonde um ein ΔTm von 6-10 °C voneinander unterscheiden, und
vorzugsweise die Zielsequenz-Bindungsbereiche, die in allen der ersten Primer enthalten sind, sich voneinander unterscheiden, und die Zielsequenz-Bindungsbereiche, die in allen der zweiten Primer enthalten sind, sich voneinander unterscheiden.

6. Zusammensetzung von Primern und Sonden nach Anspruch 2, wobei der dritte Primer sequentiell den Amplifikationsprodukt-Erfassungsbereich B und den dritten Zielsequenz-Bindungsbereich vom 5'-Ende zum 3'-Ende umfasst; der erste Primer sequentiell den Sequenzbereich A und den Amplifikationsprodukt-Erfassungsbereich B vom 5'-Ende zum 3'-Ende umfasst; und der zweite Primer sequentiell den Sequenzbereich h und den zweiten Zielsequenz-Bindungsbereich vom 5'-Ende zum 3'-Ende umfasst;
der Amplifikationsprodukt-Erfassungsbereich B nicht paarweise an die Zielsequenz oder ein Fragment davon bindet;
vorzugsweise der zweite Primer ferner eine verlängerungsblockierende Region, die eine Sequenz ist, die nicht identisch mit oder komplementär zu einer Basensequenz eines beliebigen Teils der Sonde ist, umfasst; und
mehr bevorzugt der zweite Primer sequentiell die verlängerungsblockierende Region, den Sequenzbereich h und den zweiten Zielsequenz-Bindungsbereich vom 5'-Ende zum 3'-Ende umfasst.

7. Zusammensetzung von Primern und Sonden nach Anspruch 2, wobei die Zusammensetzung von Primern und Sonden für n Zielsequenzen n zweite Primersätze, m Sonden und o erste Primer umfasst, wobei 1 ≤ n ≤ 20, 1 ≤ m n und 1 ≤ o ≤ n ist,
vorzugsweise die Zusammensetzung von Primern und Sonden für n Zielsequenzen n zweite Primersätze, 1 oder mehrere Sonden und 1 oder mehrere erste Primer umfasst, wobei 1 ≤ n ≤ 20 ist,
vorzugsweise einzelsträngige Amplifikationsprodukte, die durch Amplifikation mit allen ersten Primersätzen erhalten werden, sich hinsichtlich ihrer Anlagerungstemperatur an die Sonde voneinander unterscheiden, vorzugsweise einzelsträngige Amplifikationsprodukte, die durch Amplifikation mit allen ersten Primersätzen erhalten werden, sich hinsichtlich ihrer Anlagerungstemperatur an die Sonde um ein ΔTm von 6-10 °C voneinander unterscheiden, und
vorzugsweise sich die Zielsequenz-Bindungsbereiche, die in allen dritten Primern enthalten sind, voneinander unterscheiden, und die Zielsequenz-Bindungsbereiche, die in allen zweiten Primern enthalten sind, sich voneinander unterscheiden.

8. Zusammensetzung von Primern und Sonden nach Anspruch 2, wobei der erste Primer sequentiell den Sequenzbereich A und den ersten Zielsequenz-Bindungsbereich vom 5'-Ende bis zum 3'-Ende umfasst; der zweite Primer sequentiell den Sequenzbereich h und den Amplifikationsprodukt-Erfassungsbereich C vom 5'-Ende bis zum 3'-Ende umfasst; und der vierte Primer sequentiell den Amplifikationsprodukt-Erfassungsbereich C und den vierten Zielsequenz-Bindungsbereich vom 5'-Ende bis zum 3'-Ende umfasst;
der Amplifikationsprodukt-Erfassungsbereich C sich nicht paarweise an die Zielsequenz oder ein Fragment davon bindet;
vorzugsweise der zweite Primer ferner eine verlängerungsblockierende Region, die eine Sequenz ist, die nicht identisch mit oder komplementär zu einer Basensequenz eines beliebigen Teils der Sonde ist, umfasst; und
mehr bevorzugt der zweite Primer sequentiell die verlängerungsblockierende Region, den Sequenzbereich h und den Amplifikationsprodukt-Erfassungsbereich C vom 5'-Ende bis zum 3'-Ende umfasst; bevorzugt die Zusammensetzung von Primern und Sonden für n Zielsequenzen n zweite Primersätze, m Sonden und o zweite Primer umfasst, wobei 1 ≤ n ≤ 20, 1 ≤ m ≤ n und 1 ≤ o ≤ n ist,
vorzugsweise die Zusammensetzung von Primern und Sonden für n Zielsequenzen n zweite Primersätze, 1 oder mehrere Sonden und 1 oder mehrere zweite Primer umfasst, wobei 1 ≤ n ≤ 20 ist,
vorzugsweise sich einzelsträngige Amplifikationsprodukte, die durch Amplifizieren mit allen der ersten Primersätze erhalten werden, hinsichtlich ihrer Anlagerungstemperatur an die Sonde voneinander unterscheiden,
vorzugsweise einzelsträngige Amplifikationsprodukte, die durch Amplifikation mit allen ersten Primersätzen erhalten werden, sich hinsichtlich ihrer Anlagerungstemperatur an die Sonde um ein ΔTm von 6-10 °C voneinander unterscheiden, und
vorzugsweise sich die Zielsequenz-Bindungsbereiche, die in allen der ersten Primer enthalten sind, voneinander unterscheiden, und die Zielsequenz-Bindungsbereiche, die in allen der vierten Primer enthalten sind, sich voneinander unterscheiden.

9. Zusammensetzung von Primern und Sonden nach Anspruch 3, wobei der erste Primer sequentiell den Sequenzbereich A und den Amplifikationsprodukt-Erfassungsbereich B vom 5'-Ende bis zum 3'-Ende umfasst; der zweite Primer sequentiell den Sequenzbereich h und den Amplifikationsprodukt-Erfassungsbereich C vom 5'-Ende bis zum 3'-Ende umfasst; der dritte Primer sequentiell den Amplifikationsprodukt-Erfassungsbereich B und den dritten Zielsequenz-Bindungsbereich vom 5'-Ende bis zum 3'-Ende umfasst; und der vierte Primer sequentiell den Amplifikationsprodukt-Erfassungsbereich C und den vierten Zielsequenz-Bindungsbereich vom 5'-Ende bis zum 3'-Ende umfasst;
weder der Amplifikationsprodukt-Erfassungsbereich B noch der Amplifikationsprodukt-Erfassungsbereich C mit der Zielsequenz oder einem Fragment davon gepaart ist;
vorzugsweise der zweite Primer ferner eine verlängerungsblockierende Region umfasst, die eine Basensequenz ist, die nicht identisch mit oder komplementär zu der Sonde oder einem Fragment davon ist; und
mehr bevorzugt der zweite Primer sequentiell die verlängerungsblockierende Region, den Sequenzbereich h und den Amplifikationsprodukt-Erfassungsbereich C vom 5'-Ende zum 3'-Ende umfasst;
vorzugsweise, wobei die Zusammensetzung von Primern und Sonden für n Zielsequenzen n zweite Primersätze, m Sonden und p erste Primersätze umfasst, wobei 1 ≤ n ≤ 20, 1 ≤ m ≤ n und 1 ≤ P ≤ n ist,
vorzugsweise die Zusammensetzung von Primern und Sonden für n Zielsequenzen n zweite Primersätze, 1 oder mehrere Sonden und 1 oder mehrere erste Primersätze umfasst, wobei 1 ≤ n ≤ 20 ist,
vorzugsweise sich einzelsträngige Amplifikationsprodukte, die durch Amplifizieren mit allen der ersten Primersätze erhalten werden, hinsichtlich ihrer Anlagerungstemperatur an die Sonde voneinander unterscheiden,
vorzugsweise einzelsträngige Amplifikationsprodukte, die durch Amplifikation mit allen ersten Primersätzen erhalten werden, sich hinsichtlich ihrer Anlagerungstemperatur an die Sonde um ein ΔTm von 6-10 °C voneinander unterscheiden, und
vorzugsweise sich die Zielsequenz-Bindungsbereiche, die in allen dritten Primern enthalten sind, voneinander unterscheiden, und die Zielsequenz-Bindungsbereiche, die in allen vierten Primern enthalten sind, sich voneinander unterscheiden.

10. Ein Kit zum PCR-Nachweis, umfassend die Zusammensetzung von Primern und Sonden nach einem der Ansprüche 1 bis 9.

11. Verfahren zum PCR-Nachweis, umfassend die Schritte des Durchführens einer PCR unter Verwendung des Sondenprimersatzes nach einem der Ansprüche 1 bis 9;
vorzugsweise, die folgenden Schritte umfassend:
Verwenden des ersten Primersatzes, um spezifisch an eine Zielsequenz zu binden und sich zu verlängern, um ein doppelsträngiges Präamplifikationsprodukt zu erzeugen, wobei ein einzelsträngiges Amplifikationsprodukt des doppelsträngigen Präamplifikationsprodukts den Sequenzbereich A, die Zielsequenz und eine komplementäre Sequenz h' des Sequenzbereichs h umfasst; und
Bilden einer Signaländerung durch spezifisches Binden des Sequenzbereichs A und der komplementären Sequenz h' des einzelsträngigen Amplifikationsprodukts mit der Sonde, die durch ein Instrument erfasst werden kann, und Bestimmen, ob die Zielsequenz basierend auf der Signaländerung vorhanden ist;
vorzugsweise umfasst das einzelsträngige Amplifikationsprodukt ferner eine komplementäre Sequenz der verlängerungsblockierenden Region an ihrem 3'-Ende, die zu keinem Teil der Sonde komplementär ist.

12. Verfahren nach Anspruch 11, umfassend die folgenden Schritte:
Verwenden des dritten Primers und des zweiten Primers des zweiten Primersatzes, um spezifisch an eine Zielsequenz zu binden und sich zu verlängern, um ein erstes doppelsträngiges Präamplifikationsprodukt zu erzeugen, wobei ein einzelsträngiges Amplifikationsprodukt des ersten doppelsträngigen Präamplifikationsprodukts eine komplementäre Sequenz B' des Amplifikationsprodukt-Erfassungsbereichs B, der Zielsequenz und des Sequenzbereichs h umfasst;
Verwenden des ersten Primersatzes und Verwenden des ersten doppelsträngigen Präamplifikationsprodukts als Vorlage, um sich zu verlängern, um ein zweites doppelsträngiges Amplifikationsprodukt zu erhalten, wobei ein einzelsträngiges Amplifikationsprodukt des zweiten doppelsträngigen Amplifikationsprodukts den Sequenzbereich A, den Amplifikationsprodukt-Erfassungsbereich B, die Zielsequenz und eine komplementäre Sequenz h' des Sequenzbereichs h umfasst;
Bilden einer Signaländerung durch spezifisches Binden des Sequenzbereichs A und der komplementären Sequenz h' des einzelsträngigen Amplifikationsprodukts mit der Sonde, die durch ein Instrument erfasst werden kann, und Bestimmen, ob die Zielsequenz basierend auf der Signaländerung vorhanden ist;
vorzugsweise, wobei das eine einzelsträngige Amplifikationsprodukt des ersten doppelsträngigen Präamplifikationsprodukts ferner eine verlängerungsblockierende Region umfasst und/oder das eine einzelsträngige Amplifikationsprodukt des zweiten doppelsträngigen Amplifikationsprodukts ferner eine komplementäre Sequenz der verlängerungsblockierenden Region umfasst und die komplementäre Sequenz der verlängerungsblockierenden Region zu keinem Teil der Sonde komplementär ist.

13. Verfahren nach Anspruch 11, umfassend die folgenden Schritte:
Verwenden des ersten Primers und des vierten Primers des zweiten Primersatzes, um spezifisch an eine Zielsequenz zu binden und sich zu verlängern, um ein erstes doppelsträngiges Präamplifikationsprodukt zu erzeugen, wobei ein einzelsträngiges Amplifikationsprodukt des ersten doppelsträngigen Präamplifikationsprodukts eine komplementäre Sequenz A' des Sequenzbereichs A, der Zielsequenz und des Amplifikationsprodukt-Erfassungsbereichs C umfasst;
Verwenden des ersten Primersatzes und Verwenden des ersten doppelsträngigen Präamplifikationsprodukts als Vorlage zum Verlängern, um ein zweites doppelsträngiges Amplifikationsprodukt zu erhalten, wobei ein einzelsträngiges Amplifikationsprodukt des zweiten doppelsträngigen Amplifikationsprodukts den Sequenzbereich A, die Zielsequenz, eine komplementäre Sequenz C' des Amplifikationsprodukt-Erfassungsbereichs C und eine komplementäre Sequenz h' des Sequenzbereichs h umfasst, und
Bilden einer Signaländerung durch spezifisches Binden des Sequenzbereichs A und der komplementären Sequenz h' des einzelsträngigen Amplifikationsprodukts mit der Sonde, die durch ein Instrument erfasst werden kann, und Bestimmen, ob die Zielsequenz basierend auf der Signaländerung vorhanden ist;
Vorzugsweise, wobei das eine einzelsträngige Amplifikationsprodukt des zweiten doppelsträngigen Amplifikationsprodukts ferner eine komplementäre Sequenz der verlängerungsblockierenden Region umfasst und die komplementäre Sequenz der verlängerungsblockierenden Region zu keinem Teil der Sonde komplementär ist.

14. Verfahren nach Anspruch 11, umfassend die folgenden Schritte:
Verwenden des dritten Primers und des vierten Primers des zweiten Primersatzes, um spezifisch an eine Zielsequenz zu binden und sich zu verlängern, um ein erstes doppelsträngiges Präamplifikationsprodukt zu erzeugen, wobei ein einzelsträngiges Amplifikationsprodukt des ersten doppelsträngigen Präamplifikationsprodukts eine komplementäre Sequenz B' des Amplifikationsprodukt-Erfassungsbereichs B, der Zielsequenz und des Amplifikationsprodukt-Erfassungsbereichs C umfasst;
Verwenden des ersten Primersatzes und Verwenden des ersten doppelsträngigen Präamplifikationsprodukts als Vorlage zum Verlängern, um ein zweites doppelsträngiges Amplifikationsprodukt zu erhalten, wobei ein einzelsträngiges Amplifikationsprodukt des zweiten doppelsträngigen Amplifikationsprodukts den Sequenzbereich A, den Amplifikationsprodukt-Erfassungsbereich B, die Zielsequenz, eine komplementäre Sequenz C' des Amplifikationsprodukt-Erfassungsbereichs C und eine komplementäre Sequenz h' des Sequenzbereichs h umfasst, und
Bilden einer Signaländerung durch spezifisches Binden des Sequenzbereichs A und der komplementären Sequenz h' des einzelsträngigen Amplifikationsprodukts mit der Sonde, die durch ein Instrument erfasst werden kann, und Bestimmen, ob die Zielsequenz vorhanden ist, basierend auf der Signaländerung; wobei,
vorzugsweise, wobei das eine einzelsträngige Amplifikationsprodukt des zweiten doppelsträngigen Amplifikationsprodukts ferner eine komplementäre Sequenz der verlängerungsblockierenden Region umfasst und die komplementäre Sequenz der verlängerungsblockierenden Region zu keinem Teil der Sonde komplementär ist;
vorzugsweise, wobei sich die Signaländerung auf eine Fluoreszenzsignaländerung oder eine Änderung der Schmelztemperaturkurve bezieht; und vorzugsweise sich die Signaländerung auf die Änderung der Schmelztemperaturkurve bezieht.

15. Verwendung der Zusammensetzung von Primern und Sonden nach einem der Ansprüche 1 bis 9 beim PCR-Nachweis;
vorzugsweise bei einer Einzelröhren-Supermultiplex-PCR-Detektion.
vorzugsweise beim Nachweis pathogener Mikroorganismen, nicht-invasivem Geburtsfehlerscreening, Einzelnukleotidpolymorphismusanalyse, Methylierungsanalyse, Genmutationsanalyse oder Gentypisierung.

## Revendications

1. Composition d'amorce de sonde pour la détection par PCR, comprenant une sonde et un premier ensemble d'amorces, dans laquelle le premier ensemble d'amorces comprend une première amorce et une deuxième amorce qui sont respectivement une amorce directe et une amorce inverse ou une amorce inverse et une amorce directe,
la sonde comprend une première région de liaison A' et une deuxième région de liaison H,
la première amorce comprend une région de séquence A qui est partiellement ou complètement complémentaire de la première région de liaison A' de la sonde, et la deuxième amorce comprend une région de séquence h qui est partiellement ou complètement identique à la deuxième région de liaison H de la sonde,
aucune région parmi la première région de liaison A', la deuxième région de liaison H, la région de séquence A et la région de séquence h n'est complémentaire de ou identique à une séquence cible ou un fragment de celle-ci, et
la sonde porte un marqueur détectable.

2. Composition d'amorce de sonde selon la revendication 1, dans laquelle la première amorce comprend en outre une première région de liaison de séquence cible, et la deuxième amorce comprend en outre une deuxième région de liaison de séquence cible ;
de préférence, dans laquelle la première amorce comprend en outre une région de capture de produit d'amplification B, et la composition d'amorce de sonde comprend en outre un deuxième ensemble d'amorces, le deuxième ensemble d'amorces comprend une troisième amorce et la deuxième amorce qui sont respectivement une amorce directe et une amorce inverse ou une amorce inverse et une amorce directe, la troisième amorce comprend une troisième région de liaison de séquence cible et une région de capture de produit d'amplification B, et la deuxième amorce comprend en outre une deuxième région de liaison de séquence cible ;
de préférence, dans laquelle la deuxième amorce comprend en outre une région de capture de produit d'amplification C, et la composition d'amorce de sonde comprend en outre un deuxième ensemble d'amorces, le deuxième ensemble d'amorces comprend la première amorce et une quatrième amorce qui sont respectivement une amorce directe et une amorce inverse ou une amorce inverse et une amorce directe, dans laquelle la quatrième amorce comprend une quatrième région de liaison de séquence cible et une région de capture de produit d'amplification C, et la première amorce comprend en outre une première région de liaison de séquence cible.

3. Composition d'amorce de sonde selon la revendication 1, dans laquelle la première amorce comprend en outre une région de capture de produit d'amplification B, la deuxième amorce comprend en outre une région de capture de produit d'amplification C, et la composition d'amorce de sonde comprend en outre un deuxième ensemble d'amorces, le deuxième ensemble d'amorces comprend une troisième amorce et une quatrième amorce qui sont respectivement une amorce directe et une amorce inverse ou une amorce inverse et une amorce directe, le troisième ensemble d'amorces comprend une troisième région de liaison de séquence cible et une région de capture de produit d'amplification B, et la quatrième amorce comprend une quatrième région de liaison de séquence cible et une région de capture de produit d'amplification C ;
de préférence, dans laquelle un produit d'amplification simple brin de la première amorce et la deuxième amorce comprend la région de séquence A et une séquence h' qui est partiellement ou complètement complémentaire de la deuxième région de liaison H de la sonde ;
de préférence, la première région de liaison A' est située à une extrémité 5' ou une extrémité 3' de la sonde, et de préférence, une région de blocage est prévue à l'extrémité 3' de la sonde.

4. Composition d'amorce de sonde selon la revendication 1, dans laquelle le marqueur détectable comprend un premier marqueur détectable et un deuxième marqueur détectable, le premier marqueur détectable est un rapporteur de fluorescence, et le deuxième marqueur détectable est un extincteur ou d'autres groupes de marquage qui peuvent produire un changement de signal avec le premier marqueur détectable par transfert d'énergie de résonance de fluorescence,
de préférence, le premier marqueur détectable et le deuxième marqueur détectable sont tous deux situés sur la première région de liaison A' ou le premier marqueur détectable et le deuxième marqueur détectable sont tous deux situés sur la deuxième région de liaison H ; ou
de préférence, une région d'espacement entre le premier marqueur détectable et le deuxième marqueur détectable est au moins partiellement située sur la première région de liaison A', ou la région d'espacement entre le premier marqueur détectable et le deuxième marqueur détectable est au moins partiellement située sur la deuxième région de liaison H.

5. Composition d'amorce de sonde selon la revendication 2, dans laquelle la première amorce comprend séquentiellement la région de séquence A et la première région de liaison de séquence cible de l'extrémité 5' à l'extrémité 3' ;
la deuxième amorce comprend séquentiellement la région de séquence h et la deuxième région de liaison de séquence cible de l'extrémité 5' à l'extrémité 3',
de préférence, la deuxième amorce comprend en outre une région de blocage d'extension, qui est une séquence qui n'est pas complémentaire de ou identique à une séquence de base d'une partie quelconque de la sonde, et
plus préférablement, la deuxième amorce comprend séquentiellement la région de blocage d'extension, la région de séquence h et la deuxième région de liaison de séquence cible de l'extrémité 5' à l'extrémité 3' ; de préférence, dans laquelle la composition d'amorce de sonde pour n séquences cibles comprend n premiers ensembles d'amorces et m sondes, où 1 ≤ n ≤ 20, 1 ≤ m ≤ n ;
de préférence, la composition d'amorce de sonde pour n séquences cibles comprend n premiers ensembles d'amorces et 1 ou plusieurs sondes, où 1 ≤ n ≤ 20 ;
de préférence, les produits d'amplification simple brin obtenus par amplification de différentes séquences cibles avec tous les premiers ensembles d'amorces sont différents les uns des autres à une température de recuit avec la sonde, de préférence, les produits d'amplification simple brin obtenus par amplification de différentes séquences cibles avec tous les premiers ensembles d'amorces sont différents les uns des autres d'une valeur ΔTm de 6-10 °C à une température de recuit avec la sonde, et
de préférence, les régions de liaison de séquence cible incluses dans toutes les premières amorces sont différentes les unes des autres, et les régions de liaison de séquence cible incluses dans toutes les deuxièmes amorces sont différentes les unes des autres.

6. Composition d'amorce de sonde selon la revendication 2, dans laquelle la troisième amorce comprend séquentiellement la région de capture de produit d'amplification B et la troisième région de liaison de séquence cible de l'extrémité 5' à l'extrémité 3' ; la première amorce comprend séquentiellement la région de séquence A et la région de capture de produit d'amplification B de l'extrémité 5' à l'extrémité 3' ; et la deuxième amorce comprend séquentiellement la région de séquence h et la deuxième région de liaison de séquence cible de l'extrémité 5' à l'extrémité 3' ;
la région de capture de produit d'amplification B ne se lie pas à la séquence cible ou à un fragment de celle-ci de manière appariée ;
de préférence, la deuxième amorce comprend en outre une région de blocage d'extension, qui est une séquence qui n'est pas complémentaire de ou identique à une séquence de base d'une partie quelconque de la sonde ; et
plus préférablement, la deuxième amorce comprend séquentiellement la région de blocage d'extension, la région de séquence h et la deuxième région de liaison de séquence cible de l'extrémité 5' à l'extrémité 3'.

7. Composition d'amorce de sonde selon la revendication 2, dans laquelle la composition d'amorce de sonde pour n séquences cibles comprend n deuxièmes ensembles d'amorces, m sondes et o premières amorces, où 1 ≤ n ≤ 20, 1 ≤ m < n et 1 ≤ o ≤ n,
de préférence, la composition d'amorce de sonde pour n séquences cibles comprend n deuxièmes ensembles d'amorces, 1 ou plusieurs sondes, et 1 ou plusieurs premières amorces, où 1 ≤ n ≤ 20,
de préférence, les produits d'amplification simple brin obtenus par amplification avec tous les premiers ensembles d'amorces sont différents les uns des autres à une température de recuit avec la sonde, de préférence, les produits d'amplification simple brin obtenus par amplification avec tous les premiers ensembles d'amorces sont différents les uns des autres d'une valeur ΔTm de 6-10 °C à une température de recuit avec la sonde, et
de préférence, les régions de liaison de séquence cible incluses dans toutes les troisièmes amorces sont différentes les unes des autres, et les régions de liaison de séquence cible incluses dans toutes les deuxièmes amorces sont différentes les unes des autres.

8. Composition d'amorce de sonde selon la revendication 2, dans laquelle la première amorce comprend séquentiellement la région de séquence A et la première région de liaison de séquence cible de l'extrémité 5' à l'extrémité 3' ; la deuxième amorce comprend séquentiellement la région de séquence h et la région de capture de produit d'amplification C de l'extrémité 5' à l'extrémité 3' ; et la quatrième amorce comprend séquentiellement la région de capture de produit d'amplification C et la quatrième région de liaison de séquence cible de l'extrémité 5' à l'extrémité 3' ;
la région de capture de produit d'amplification C ne se lie pas à la séquence cible ou à un fragment de celle-ci de manière appariée ;
de préférence, la deuxième amorce comprend en outre une région de blocage d'extension, qui est une séquence qui n'est pas complémentaire de ou identique à une séquence de base d'une partie quelconque de la sonde ; et
plus préférablement, la deuxième amorce comprend séquentiellement la région de blocage d'extension, la région de séquence h et la région de capture de produit d'amplification C de l'extrémité 5' à l'extrémité 3' ; de préférence, dans laquelle la composition d'amorce de sonde pour n séquences cibles comprend n deuxièmes ensembles d'amorces, m sondes et o deuxièmes amorces, où 1 ≤ n ≤ 20, 1 ≤ m ≤ n et 1 ≤ o ≤ n,
de préférence, la composition d'amorce de sonde pour n séquences cibles comprend n deuxièmes ensembles d'amorces, 1 ou plusieurs sondes, et 1 ou plusieurs deuxièmes amorces, où 1 ≤ n ≤ 20,
de préférence, les produits d'amplification simple brin obtenus par amplification avec tous les premiers ensembles d'amorces sont différents les uns des autres à une température de recuit avec la sonde,
de préférence, les produits d'amplification simple brin obtenus par amplification avec tous les premiers ensembles d'amorces sont différents les uns des autres d'une valeur ΔTm de 6-10 °C à une température de recuit avec la sonde, et
de préférence, les régions de liaison de séquence cible incluses dans toutes les premières amorces sont différentes les unes des autres, et les régions de liaison de séquence cible incluses dans toutes les quatrièmes amorces sont différentes les unes des autres.

9. Composition d'amorce de sonde selon la revendication 3, dans laquelle la première amorce comprend séquentiellement la région de séquence A et la région de capture de produit d'amplification B de l'extrémité 5' à l'extrémité 3' ; la deuxième amorce comprend séquentiellement la région de séquence h et la région de capture de produit d'amplification C de l'extrémité 5' à l'extrémité 3' ; la troisième amorce comprend séquentiellement la région de capture de produit d'amplification B et la troisième région de liaison de séquence cible de l'extrémité 5' à l'extrémité 3' ; et la quatrième amorce comprend séquentiellement la région de capture de produit d'amplification C et la quatrième région de liaison de séquence cible de l'extrémité 5' à l'extrémité 3' ;
ni la région de capture de produit d'amplification B ni la région de capture de produit d'amplification C ne sont appariées avec la séquence cible ou un fragment de celle-ci ;
de préférence, la deuxième amorce comprend en outre une région de blocage d'extension, qui est une séquence de base qui n'est pas complémentaire de ou identique à la sonde ou un fragment quelconque de celle-ci ; et
plus préférablement, la deuxième amorce comprend séquentiellement la région de blocage d'extension, la région de séquence h et la région de capture de produit d'amplification C de l'extrémité 5' à l'extrémité 3' ;
de préférence, dans laquelle la composition d'amorce de sonde pour n séquences cibles comprend n deuxièmes ensembles d'amorces, m sondes et p premiers ensembles d'amorces, où 1 ≤ n ≤ 20, 1 ≤ m ≤ n et 1 ≤ P ≤ n,
de préférence, la composition d'amorce de sonde pour n séquences cibles comprend n deuxièmes ensembles d'amorces, 1 ou plusieurs sondes, et 1 ou plusieurs premiers ensembles d'amorces, où 1 ≤ n ≤ 20,
de préférence, les produits d'amplification simple brin obtenus par amplification avec tous les premiers ensembles d'amorces sont différents les uns des autres à une température de recuit avec la sonde,
de préférence, les produits d'amplification simple brin obtenus par amplification avec tous les premiers ensembles d'amorces sont différents les uns des autres d'une valeur ΔTm de 6-10 °C à une température de recuit avec la sonde, et
de préférence, les régions de liaison de séquence cible incluses dans toutes les troisièmes amorces sont différentes les unes des autres, et les régions de liaison de séquence cible incluses dans toutes les quatrièmes amorces sont différentes les unes des autres.

10. Kit pour la détection par PCR, comprenant la composition d'amorce de sonde selon l'une quelconque des revendications 1 à 9.

11. Procédé pour la détection par PCR, comprenant les étapes consistant à effectuer une PCR en utilisant l'ensemble d'amorces de sonde de l'une quelconque des revendications 1 à 9 ;
de préférence, comprenant les étapes suivantes consistant à :
utiliser le premier ensemble d'amorces pour se lier spécifiquement à une séquence cible et s'étendre afin de produire un produit de préamplification double brin, dans lequel un produit d'amplification simple brin du produit de préamplification double brin comprend la région de séquence A, la séquence cible et une séquence complémentaire h' de la région de séquence h ; et
former un changement de signal, par liaison spécifique de la région de séquence A et de la séquence complémentaire h' du produit d'amplification simple brin avec la sonde, qui peut être détecté par un instrument, et déterminer si la séquence cible est présente sur la base du changement de signal ;
de préférence, le produit d'amplification simple brin comprend en outre une séquence complémentaire de la région de blocage d'extension à son extrémité 3', qui n'est complémentaire d'aucune partie de la sonde.

12. Procédé selon la revendication 11, comprenant les étapes suivantes consistant à :
utiliser la troisième amorce et la deuxième amorce du deuxième ensemble d'amorces pour se lier spécifiquement à une séquence cible et s'étendre afin de produire un premier produit de préamplification double brin, dans lequel un produit d'amplification simple brin du premier produit de préamplification double brin comprend une séquence complémentaire B' de la région de capture de produit d'amplification B, la séquence cible et la région de séquence h ;
utiliser le premier ensemble d'amorces et prendre le premier produit de préamplification double brin comme modèle, pour s'étendre afin d'obtenir un deuxième produit d'amplification double brin, dans lequel un produit d'amplification simple brin du deuxième produit d'amplification double brin comprend la région de séquence A, la région de capture de produit d'amplification B, la séquence cible et une séquence complémentaire h' de la région de séquence h ;
former un changement de signal, par liaison spécifique de la région de séquence A et de la séquence complémentaire h' du produit d'amplification simple brin avec la sonde, qui peut être détecté par un instrument, et déterminer si la séquence cible est présente sur la base du changement de signal ;
de préférence, le produit d'amplification simple brin du premier produit de préamplification double brin comprend en outre une région de blocage d'extension et/ou le produit d'amplification simple brin du deuxième produit d'amplification double brin comprend en outre une séquence complémentaire de la région de blocage d'extension, et la séquence complémentaire de la région de blocage d'extension n'est complémentaire d'aucune partie de la sonde.

13. Procédé selon la revendication 11, comprenant les étapes suivantes consistant à :
utiliser la première amorce et la quatrième amorce du deuxième ensemble d'amorces pour se lier spécifiquement à une séquence cible et s'étendre afin de produire un premier produit de préamplification double brin, dans lequel un produit d'amplification simple brin du premier produit de préamplification double brin comprend une séquence complémentaire A' de la région de séquence A, la séquence cible et la région de capture de produit d'amplification C ;
utiliser le premier ensemble d'amorces et prendre le premier produit de préamplification double brin comme modèle pour s'étendre afin d'obtenir un deuxième produit d'amplification double brin, dans lequel un produit d'amplification simple brin du deuxième produit d'amplification double brin comprend la région de séquence A, la séquence cible, une séquence complémentaire C' de la région de capture de produit d'amplification C et une séquence complémentaire h' de la région de séquence h, et
former un changement de signal, par liaison spécifique de la région de séquence A et de la séquence complémentaire h' du produit d'amplification simple brin avec la sonde, qui peut être détecté par un instrument, et déterminer si la séquence cible est présente sur la base du changement de signal ;
de préférence, le produit d'amplification simple brin du deuxième produit d'amplification double brin comprend en outre une séquence complémentaire de la région de blocage d'extension, et la séquence complémentaire de la région de blocage d'extension n'est complémentaire d'aucune partie de la sonde.

14. Procédé selon la revendication 11, comprenant les étapes suivantes consistant à :
utiliser la troisième amorce et la quatrième amorce du deuxième ensemble d'amorces pour se lier spécifiquement à une séquence cible et s'étendre afin de produire un premier produit de préamplification double brin, dans lequel un produit d'amplification simple brin du premier produit de préamplification double brin comprend une séquence complémentaire B' de la région de capture de produit d'amplification B, la séquence cible et la région de capture de produit d'amplification C ;
utiliser le premier ensemble d'amorces et prendre le premier produit de préamplification double brin comme modèle pour s'étendre afin d'obtenir un deuxième produit d'amplification double brin, dans lequel un produit d'amplification simple brin du deuxième produit d'amplification double brin comprend la région de séquence A, la région de capture de produit d'amplification B, la séquence cible, une séquence complémentaire C' de la région de capture de produit d'amplification C et une séquence complémentaire h' de la région de séquence h, et
former un changement de signal, par liaison spécifique de la région de séquence A et de la séquence complémentaire h' du produit d'amplification simple brin avec la sonde, qui peut être détecté par un instrument, et déterminer si la séquence cible est présente sur la base du changement de signal ; dans lequel,
de préférence, le produit d'amplification simple brin du deuxième produit d'amplification double brin comprend en outre une séquence complémentaire de la région de blocage d'extension, et la séquence complémentaire de la région de blocage d'extension n'est complémentaire d'aucune partie de la sonde ;
de préférence, dans lequel le changement de signal fait référence à un changement de signal de fluorescence ou à un changement de courbe de température de fusion ; et de préférence, le changement de signal fait référence au changement de courbe de température de fusion.

15. Utilisation de la composition d'amorce de sonde selon l'une quelconque des revendications 1 à 9 dans la détection par PCR ;
de préférence, dans une détection par PCR super-multiplex à tube unique,
de préférence, dans la détection de micro-organismes pathogènes, le dépistage non invasif des anomalies congénitales, l'analyse du polymorphisme mononucléotidique, l'analyse de méthylation, l'analyse de mutation génique ou le typage génique.
